# EUROPEAN PATENT APPLICATION

(11) **EP 4 711 369 A1**
(43) Date of publication of application: **18.03.2026**
(21) Application number: 24814600.3
(22) Date of filing: 31.05.2024
(51) Int. Cl.: C07D 487/14, A61K 31/517, A61P 35/00

(54) **1H-[1,2,3]TRIAZOLO[4,5-H]QUINAZOLINE COMPOUNDS AS PROTEIN KINASE INHIBITORS**

(30) Priority: 01.06.2023 CN 202310664526
(71) Applicant: Chengdu Cynogen Bio-Pharmaceutical Technology Co., Ltd., Chengdu, Sichuan 610213 (CN)
(72) Inventor: CHENG, Hang, Chengdu, Sichuan 610095 (CN); XIONG, Weiyan, Chengdu, Sichuan 610095 (CN); SHI, Pengyi, Chengdu, Sichuan 610095 (CN); NIU, Wei, Chengdu, Sichuan 610095 (CN); YU, Bin, Chengdu, Sichuan 610095 (CN); JIANG, Yu, Chengdu, Sichuan 610095 (CN)
(74) Representative: Maiwald GmbH
(86) International application number: PCT/CN2024/096701
(87) International publication number: WO 2024/245406

(57) **Abstract**

Provided are 1H-[1,2,3]triazolo[4,5-H]quinazoline compounds of general formula (I), and the compounds can be used for treating cell proliferation disorders. The compounds are effective inhibitors of cyclin-dependent kinases (CDKs).

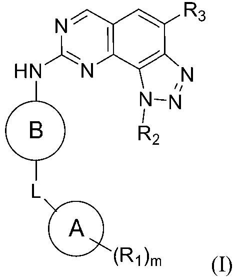

## Description

The present disclosure claims the priority of Chinese patent application 202310664526.9 filed on June 1, 2023, which is incorporated herein by reference in its entirety.

### TECHNICAL FIELD

The present disclosure provides a class of 1H-[1,2,3]triazolo[4,5-h]quinazoline compounds as inhibitors of cyclin-dependent kinase (CDK), which have a broad-spectrum, strong inhibitory activity against CDK. The compounds of the present disclosure are effective in treating diseases such as cancer, inflammation, etc.

### BACKGROUND

Cyclin-dependent kinase (CDK) and cyclin are important factors in cell cycle regulation. CDK can combine with cyclin to form a heterodimer, in which CDK is the catalytic subunit and cyclin is the regulatory subunit. Various cyclin-CDK complexes thus formed phosphorylate different substrates, and promote and transform different phases of the cell cycle.

In the past decade, CDK inhibitors have become a hot topic in the development of new anti-tumor drugs, and more than 20 CDK inhibitors have entered the clinical stage. Although the preclinical pharmacodynamic results of CDK inhibitors are remarkable, the results of most clinical trials are not satisfactory. Problems include lack of efficacy in solid tumors and greater toxicity. Some CDK inhibitor drugs lack selectivity for CDK subtypes, resulting in greater toxicity.

CDK4 and CDK6 are two closely related kinases that bind to Cyclin D during the tumor cell cycle to promote cell cycle progress from G1 phase to S phase and are required for cell cycle progression. It has been shown that in human tumors (such as breast cancer and myeloma), activation of CDK4 and CDK6 leads to cell cycle changes. Inhibition of CDK4 and CDK6 prevents the inactivation of the tumor suppressor protein Rb and interferes with tumor cell cycle progression.

Overexpression of CDK2 is related to abnormal regulation of cell cycle, and cyclin E/CDK2 complex plays an important role in regulating G1/S conversion, histone biosynthesis and centrosome replication. Progressive phosphorylation of Rb by cyclin D/CDK4/6 and cyclin E/CDK2 releases G1 transcription factor E2F and promotes entry of S phase. Activation of cyclin A/CDK2 during early S phase promotes phosphorylation of endogenous substrates, which allows DNA replication and inactivation of E2F to complete S phase. (Asghar et al., The history and future of targeting cyclin-dependent kinases in cancer therapy, Nat. Rev. Drug. Discov. 2015; 14(2): 130-146).

Cyclin-dependent kinase 9 (CDK9) participates in the formation of positive transcription elongation factor (P-TEFb) and plays a key role in the transcription regulation, especially in the regulation of short-lived anti-apoptotic proteins, which are very important for the survival of many tumor cells, so CDK9 has become an important target for cancer treatment. Dinaciclib (MK-7965) and Seliciclib (CYC202), small molecular inhibitors with CDK9 inhibitory activity, have been approved for clinical trials of breast cancer and hematological tumors and combined chemotherapy for advanced solid tumors.

Although many CDK inhibitor compounds have been published, there is still a need for more CDK inhibitors to treat CDK-related diseases.

### SUMMARY

The present disclosure provides a class of 1H-[1,2,3]triazolo[4,5-h]quinazoline compounds as inhibitors of cyclin-dependent kinase, which have a strong inhibitory activity. In addition, compared with the existing drugs, the compounds of the present disclosure can further improve the pharmacokinetic properties, including the significant improvement in the metabolic stability and clearance rate over the existing compounds.

In one aspect, the present disclosure provides a compound of formula (I), or a pharmaceutically acceptable salt, an enantiomer, a diastereomer, a racemate, a solvate, a hydrate, a polymorph, a prodrug, or an isotopic variant thereof, or a mixture thereof: 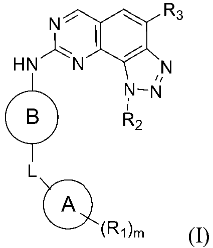 wherein,
L is selected from -C(O)- and -S(O)₂-, alternatively -C(O)-;
ring A is 5- to 10-membered heterocyclyl;
ring B is C₆₋₁₀ aryl;
R₁ is selected from H, D, halogen, -C₀₋₆ alkylene-CN, -C₀₋₆ alkylene-NR₁ₐR_{1b}, -C₀₋₆ alkylene-OR₁ₐ, C₁₋₆ alkyl, C₁₋₆ haloalkyl, C₂₋₆ alkenyl and C₂₋₆ alkynyl;
R₁ₐ is selected from H, D, C₁₋₆ alkyl and C₁₋₆ haloalkyl;
R_{1b} is selected from H, D, C₁₋₆ alkyl and C₁₋₆ haloalkyl;
m is selected from 0, 1, 2, 3, 4 and 5;
R₂ is selected from C₁₋₆ alkyl, C₁₋₆ haloalkyl, C₅₋₁₀ cycloalkyl, 5- to 10-membered heterocyclyl, -C₀₋₆ alkylene-CN, -C₀₋₆ alkylene-NH₂ and -C₁₋₆ alkylene-OH, and the R₂ is optionally substituted with 1, 2, 3, 4 or 5 R₂ₐ;
R₂ₐ is selected from H, D, halogen, ORₐ, CN, NR_{b}R_{c}, C₁₋₆ alkyl and C₁₋₆ haloalkyl;
R₃ is selected from H, D, halogen, C₁₋₆ alkyl and C₁₋₆ haloalkyl;
Rₐ, R_{b} and R_{c} are selected from H, C₁₋₆ alkyl and C₁₋₆ haloalkyl;
each of the above groups may be optionally substituted with one or more deuterium atoms, up to fully deuterated;
with the proviso that, when R₂ is isopropyl and R₃ is H, ring A is not 6-membered heterocyclyl.

In another aspect, the present disclosure provides a pharmaceutical composition, comprising a compound of the present disclosure, and optionally pharmaceutically acceptable excipient(s).

In another aspect, the present disclosure provides a pharmaceutical composition comprising a compound of the present disclosure and pharmaceutically acceptable excipient(s), which further comprises other therapeutic agent(s).

In another aspect, the present disclosure provides a kit comprising a compound of the present disclosure, other therapeutic agent(s) and pharmaceutically acceptable carrier(s), adjuvant(s) or vehicle(s).

In another aspect, the present disclosure provides use of a compound of the present disclosure in the manufacture of a medicament for the treatment and/or prevention of a CDK-mediated disease.

In another aspect, the present disclosure provides a method of treating and/or preventing a CDK-mediated disease in a subject, including administering a compound of the present disclosure or a composition of the present disclosure to the subject.

In another aspect, the present disclosure provides a compound or a composition of the present disclosure, for use in treating and/or preventing a CDK-mediated disease.

In a specific embodiment, the diseases described herein include cell proliferative diseases such as solid tumors such as sarcomas and carcinomas (e.g., fibrosarcoma, myxosarcoma, liposarcoma, chondrosarcoma, osteosarcoma, chordoma, angiosarcoma, endothelial sarcoma, lymphangiosarcoma, lymphangioendothelioma, synovialoma, mesothelioma, ewing sarcoma, leiomyosarcoma, rhabdomyosarcoma, colon cancer, pancreatic cancer, breast cancer, ovarian cancer, prostate cancer, squamous cell carcinoma, basal cell carcinoma, adenocarcinoma, hidradenoma, sebaceous carcinoma, papillary carcinoma, papillary adenocarcinoma, cystadenocarcinoma, medullary carcinoma, bronchial carcinoma, renal cell carcinoma, liver cancer, cholangiocarcinoma, choriocarcinoma, seminoma, embryonal cancer, embryonal carcinosarcoma, cervical cancer, uterine cancer, testicular cancer, lung cancer, small cell lung cancer, bladder cancer, epithelial cancer, glioma, astrocytoma, medulloblastoma, craniopharyngioma, ependymoma, pineal tumor, hemangioblastoma, acoustic neuroma, oligodendroglioma, schwannoma, meningioma, melanoma, neuroblastoma and retinoblastoma).

Other objects and advantages of the present disclosure will be apparent to those skilled in the art from the subsequent specific embodiments, examples and claims.

### Definitions

### Chemical definitions

Definitions of specific functional groups and chemical terms are described in more detail hereafter.

When a range of values is listed, each value and sub-range within the range are intended to be included. For example, "C₁₋₆ alkyl" is intended to include C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅ and C₅₋₆ alkyl.

It should be understood that when described herein any of the moieties defined forth below may be substituted by a variety of substituents, and that the respective definitions are intended to include such substituted moieties within their scope as set out below. Unless otherwise stated, the term "substituted" is to be defined as set out below.

"C₁₋₆ alkyl" refers to a radical of a straight or branched, saturated hydrocarbon group having 1 to 6 carbon atoms. In some embodiments, C₁₋₄ alkyl is alternative. Examples of C₁₋₆ alkyl include methyl (C₁), ethyl (C₂), n-propyl (C₃), iso-propyl (C₃), n-butyl (C₄), tert-butyl (C₄), sec-butyl (C₄), iso-butyl (C₄), n-pentyl (C₅), 3-pentyl (C₅), pentyl (C₅), neopentyl (C₅), 3-methyl-2-butyl (C₅), tert-pentyl (C₅) and n-hexyl (C₆). The term "C₁₋₆ alkyl" also includes heteroalkyl, wherein one or more (e.g., 1, 2, 3 or 4) carbon atoms are subsituted with heteroatoms (e.g., oxygen, sulfur, nitrogen, boron, silicon, phosphorus). Alkyl groups can be optionally substituted with one or more substituents, for example, with 1 to 5 substituents, 1 to 3 substituents or 1 substituent. Conventional abbreviations of alkyl include Me (-CH₃), Et (-CH₂CH₃), iPr (-CH(CH₃)₂), nPr (-CH₂CH₂CH₃), n-Bu (-CH₂CH₂CH₂CH₃) or i-Bu (-CH₂CH(CH₃)₂).

"C₂₋₆ alkenyl" refers to a radical of a straight or branched hydrocarbon group having 2 to 6 carbon atoms and at least one carbon-carbon double bond. In some embodiments, C₂₋₄ alkenyl is alternative. Examples of C₂₋₆ alkenyl include vinyl (C₂), 1-propenyl (C₃), 2-propenyl (C₃), 1-butenyl (C₄), 2-butenyl (C₄), butadienyl (C₄), pentenyl (C₅), pentadienyl (C₅), hexenyl (C₆), etc. The term "C₂₋₆ alkenyl" also includes heteroalkenyl, wherein one or more (e.g., 1, 2, 3 or 4) carbon atoms are replaced by heteroatoms (e.g., oxygen, sulfur, nitrogen, boron, silicon, phosphorus). The alkenyl groups can be optionally substituted with one or more substituents, for example, with 1 to 5 substituents, 1 to 3 substituents or 1 substituent.

"C₂₋₆ alkynyl" refers to a radical of a straight or branched hydrocarbon group having 2 to 6 carbon atoms, at least one carbon-carbon triple bond and optionally one or more carbon-carbon double bonds. In some embodiments, C₂₋₄ alkynyl is alternative. Examples of C₂₋₆ alkynyl include, but are not limited to, ethynyl (C₂), 1-propynyl (C₃), 2-propynyl (C₃), 1-butynyl (C₄), 2-butynyl (C₄), pentynyl (C₅), hexynyl (C₆), etc. The term "C₂₋₆ alkynyl" also includes heteroalkynyl, wherein one or more (e.g., 1, 2, 3 or 4) carbon atoms are replaced by heteroatoms (e.g., oxygen, sulfur, nitrogen, boron, silicon, phosphorus). The alkynyl groups can be substituted with one or more substituents, for example, with 1 to 5 substituents, 1 to 3 substituents or 1 substituent.

"-C₁₋₆ alkylene-, -C₂₋₆ alkenylene- or -C₂₋₆ alkynylene-" refers to a divalent group of the "C₁₋₆ alkyl, C₂₋₆ alkenyl or C₂₋₆ alkynyl" as defined above.

"C₁₋₆ alkylene" refers to a divalent group formed by removing another hydrogen of the C₁₋₆ alkyl, and can be a substituted or unsubstituted alkylene. In some embodiments, C₁₋₄ alkylene is yet alternative. The unsubstituted alkylene groups include, but are not limited to, methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), etc. Examples of substituted alkylene groups, such as those substituted with one or more alkyl (methyl) groups, include, but are not limited to, substituted methylene (-CH(CH₃)-, -C(CH₃)₂-), substituted ethylene (-CH(CH₃)CH₂-, -CH₂CH(CH₃)-, -C(CH₃)₂CH₂-, -CH₂C(CH₃)₂-), substituted propylene (-CH(CH₃)CH₂CH₂-, -CH₂CH(CH₃)CH₂-, -CH₂CH₂CH(CH₃)-, -C(CH₃)₂CH₂CH₂-, -CH₂C(CH₃)₂CH₂-, - CH₂CH₂C(CH₃)₂-), etc.

"C₀₋₆ alkylene" means a chemical bond and "C₁₋₆ alkylene" as defined above.

"C₂₋₆ alkenylene" refers to a C₂₋₆ alkenyl group wherein another hydrogen is removed to provide a divalent radical of alkenylene, and which may be substituted or unsubstituted alkenylene. In some embodiments, C₂₋₄ alkenylene is yet alternative. Exemplary unsubstituted alkenylene groups include, but are not limited to, ethenylene (-CH=CH-) and propenylene (e.g., -CH=CHCH₂-, -CH₂-CH=CH-). Exemplary substituted alkenylene groups, e.g., substituted with one or more alkyl (methyl) groups, include but are not limited to, substituted ethylene (-C(CH₃)=CH-, -CH=C(CH₃)-), substituted propylene (e.g., -C(CH₃)=CHCH₂-, -CH=C(CH₃)CH₂-, -CH=CHCH(CH₃)-, -CH=CHC(CH₃)₂-, -CH(CH₃)-CH=CH-, -C(CH₃)₂-CH=CH-, -CH₂-C(CH₃)=CH-, -CH₂-CH=C(CH₃)-), and the like.

"C₂₋₆ alkynylene" refers to a C₂₋₆ alkynyl group wherein another hydrogen is removed to provide a divalent radical of alkynylene, and which may be substituted or unsubstituted alkynylene. In some embodiments, C₂₋₄ alkynylene is yet alternative. Exemplary alkynylene groups include, but are not limited to, ethynylene (-C≡C-), substituted or unsubstituted propynylene (-C≡CCH₂-), and the like.

"Halo" or "halogen" refers to fluorine (F), chlorine (Cl), bromine (Br) and iodine (I).

"C₁₋₆ haloalkyl" represents the "C₁₋₆ alkyl" described above, which is substituted with one or more halogen groups. Examples include the mono-, di-, poly-halogenated, including perhalogenated, alkyl. A monohalogen substituent may have one iodine, bromine, chlorine or fluorine atom in the group; a dihalogen substituent and a polyhalogen substituent may have two or more identical halogen atoms or a combination of different halogens. Examples of alternative haloalkyl groups include monofluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl. The haloalkyl groups can be substituted at any available point of attachment, for example, with 1 to 5 substituents, 1 to 3 substituents or 1 substituent.

"C₅₋₁₀ cycloalkyl" refers to a radical of non-aromatic cyclic hydrocarbon group having 5 to 10 ring carbon atoms and zero heteroatoms. In some embodiments, C₅₋₆ cycloalkyl is yet alternative, and C₅₋₆ cycloalkyl is still alternative. The cycloalkyl also includes a ring system in which the cycloalkyl described herein is fused with one or more aryl or heteroaryl groups, wherein the point of attachment is on the cycloalkyl ring, and in such case, the number of carbon atoms continues to represent the number of carbon atoms in the cycloalkyl system. Exemplary cycloalkyl groups include, but are not limited to, cyclopentyl (C₅), cyclopentenyl (C₅), cyclohexyl (C₆), cyclohexenyl (C₆), cyclohexadienyl (C₆), cycloheptyl (C₇), cycloheptenyl (C₇), cycloheptadienyl (C₇), cycloheptatrienyl (C₇), etc.

"5- to 10-membered heterocyclyl" refers to a radical of 3- to 10-membered non-aromatic ring system having ring carbon atoms and 1 to 5 ring heteroatoms, wherein each of the heteroatoms is independently selected from nitrogen, oxygen, sulfur, boron, phosphorus and silicon. In the heterocyclyl containing one or more nitrogen atoms, the point of attachment can be a carbon or nitrogen atom as long as the valence permits. In some embodiments, 5- to 8-membered heterocyclyl is alternative, which is a radical of 5- to 8-membered non-aromatic ring system having ring carbon atoms and 1 to 4 ring heteroatoms. 5- to 6-membered heterocyclyl is still alternative, which is a radical of 5- to 6-membered non-aromatic ring system having ring carbon atoms and 1 to 3 ring heteroatoms. The heterocyclyl also includes a ring system wherein the heterocyclyl described above is fused with one or more cycloalkyl groups, wherein the point of attachment is on the cycloalkyl ring, or the heterocyclyl described above is fused with one or more aryl or heteroaryl groups, wherein the point of attachment is on the heterocyclyl ring; and in such cases, the number of ring members continues to represent the number of ring members in the heterocyclyl ring system. Exemplary 5-membered heterocyclyl groups containing one heteroatom include, but are not limited to, tetrahydrofuranyl, dihydrofuranyl, tetrahydrothiophenyl, dihydrothienyl, pyrrolidinyl, dihydropyrrolyl and pyrrolyl-2,5-dione. Exemplary 5-membered heterocyclyl groups containing two heteroatoms include, but are not limited to, dioxolanyl, oxasulfuranyl, disulfuranyl, and oxazolidin-2-one. Exemplary 5-membered heterocyclyl groups containing three heteroatoms include, but are not limited to, triazolinyl, oxadiazolinyl, and thiadiazolinyl. Exemplary 6-membered heterocyclyl groups containing one heteroatom include, but are not limited to, piperidyl, tetrahydropyranyl, dihydropyridyl and thianyl. Exemplary 6-membered heterocyclyl groups containing two heteroatoms include, but are not limited to, piperazinyl, morpholinyl, dithianyl and dioxanyl. Exemplary 6-membered heterocyclyl groups containing three heteroatoms include, but are not limited to, triazinanyl. Exemplary 7-membered heterocycly groups containing one heteroatom include, but are not limited to, azepanyl, oxepanyl and thiepanyl. Exemplary 5-membered heterocyclyl groups fused with a C₆ aryl (also referred as 5,6-bicyclic heterocyclyl herein) include, but are not limited to, indolinyl, isoindolinyl, dihydrobenzofuranyl, dihydrobenzothiophenyl, benzoxazolinonyl, etc. Exemplary 6-membered heterocyclyl groups fused with a C₆ aryl (also referred as 6,6-bicyclic heterocyclyl herein) include, but are not limited to, tetrahydroquinolinyl, tetrahydroisoquinolinyl, etc.

The 5- to 10-membered heterocyclyl also includes spiroheterocyclyl, that is, a group in which two rings (e.g., a heterocycle and a carbocycle) share a carbon atom, wherein at least one of the rings is a heterocyclyl as defined above. More specifically, the spiroheterocyclyl is a spiro ring formed by two 4-membered rings, two 5-membered rings, one 4-membered ring and one 5-membered ring, or one 4-membered ring and one 6-membered ring. Specific spiroheterocyclyl groups include, but are not limited to:

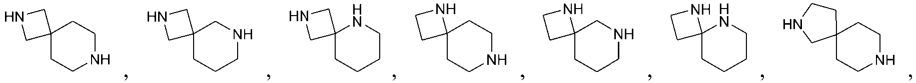

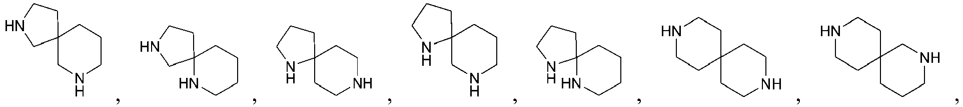

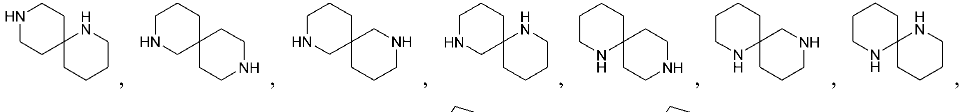

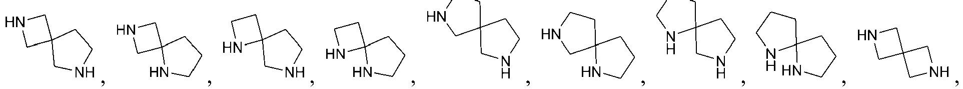

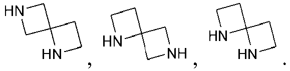

The 5- to 10-membered heterocyclyl also includes the cases where the carbon atoms or heteroatoms on the heterocyclic ring are oxidized or sulfurized, and also includes the cases where the carbon atoms or heteroatoms on the heterocyclic ring are oxidized by one or more oxygen atoms, for example, 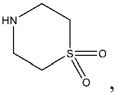 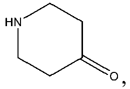 and the like.

"C₆₋₁₀ aryl" refers to a radical of monocyclic or polycyclic (e.g., bicyclic) 4n+2 aromatic ring system having 6-10 ring carbon atoms and zero heteroatoms (e.g., having 6 or 10 shared π electrons in a cyclic array). In some embodiments, the aryl group has six ring carbon atoms ("C₆ aryl"; for example, phenyl). In some embodiments, the aryl group has ten ring carbon atoms ("C₁₀ aryl"; for example, naphthyl, e.g., 1-naphthyl and 2-naphthyl). The aryl group also includes a ring system in which the aryl ring described above is fused with one or more cycloalkyl or heterocyclyl groups, and the point of attachment is on the aryl ring, in which case the number of carbon atoms continues to represent the number of carbon atoms in the aryl ring system.

"5- to 10-membered heteroaryl" refers to a radical of 5- to 10-membered monocyclic or bicyclic 4n+2 aromatic ring system (e.g., having 6 or 10 shared π electrons in a cyclic array) having ring carbon atoms and 1-4 ring heteroatoms, wherein each heteroatom is independently selected from nitrogen, oxygen and sulfur. In the heteroaryl group containing one or more nitrogen atoms, the point of attachment can be a carbon or nitrogen atom as long as the valence permits. Heteroaryl bicyclic systems may include one or more heteroatoms in one or two rings. Heteroaryl also includes ring systems wherein the heteroaryl ring described above is fused with one or more cycloalkyl or heterocyclyl groups, and the point of attachment is on the heteroaryl ring. In such case, the number the carbon atoms continues to represent the number of carbon atoms in the heteroaryl ring system. In some embodiments, 5- to 6-membered heteroaryl groups are yet alternative, which are radicals of 5- to 6-membered monocyclic or bicyclic 4n+2 aromatic ring systems having ring carbon atoms and 1-4 ring heteroatoms. Exemplary 5-membered heteroaryl groups containing one heteroatom include, but are not limited to, pyrrolyl, furyl and thienyl. Exemplary 5-membered heteroaryl groups containing two heteroatoms include, but are not limited to, imidazolyl, pyrazolyl, oxazolyl, isoxazolyl, thiazolyl, and isothiazolyl. Exemplary 5-membered heteroaryl groups containing three heteroatoms include, but are not limited to, triazolyl, oxadiazolyl (such as, 1,2,4-oxadiazoly), and thiadiazolyl. Exemplary 5-membered heteroaryl groups containing four heteroatoms include, but are not limited to, tetrazolyl. Exemplary 6-membered heteroaryl groups containing one heteroatom include, but are not limited to, pyridyl. Exemplary 6-membered heteroaryl groups containing two heteroatoms include, but are not limited to, pyridazinyl, pyrimidinyl, and pyrazinyl. Exemplary 6-membered heteroaryl groups containing three or four heteroatoms include, but are not limited to, triazinyl and tetrazinyl, respectively. Exemplary 7-membered heteroaryl groups containing one heteroatom include, but are not limited to, azepinyl, oxepinyl, and thiepinyl. Exemplary 5,6-bicyclic heteroaryl groups include, but are not limited to, indolyl, isoindolyl, indazolyl, benzotriazolyl, benzothiophenyl, isobenzothiophenyl, benzofuranyl , benzoisofuranyl, benzimidazolyl, benzoxazolyl, benzoisoxazolyl, benzoxadiazolyl, benzothiazolyl, benzoisothiazolyl, benzothiadiazolyl, indolizinyl and purinyl. Exemplary 6,6-bicyclic heteroaryl groups include, but are not limited to, naphthyridinyl, pteridinyl, quinolyl, isoquinolyl, cinnolinyl, quinoxalinyl, phthalazinyl and quinazolinyl.

"Carbonyl", whether used alone or in conjunction with other terms (e.g., aminocarbonyl), is represented by -C(O)-.

"Oxo" represents =O.

"Thioxo" represents =S.

Alkyl, alkenyl, alkynyl, carbocyclyl, heterocyclyl, aryl, and heteroaryl groups, as defined herein, are optionally substituted groups. In general, the term "substituted", whether preceded by the term "optionally" or not, means that at least one hydrogen present on a group (*e.g.,* a carbon or nitrogen atom) is replaced with a permissible substituent, e.g., a substituent which upon substitution results in a stable compound, e.g., a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, any of the substituents described herein that results in the formation of a stable compound. For purposes of this disclosure, heteroatoms such as nitrogen may have hydrogen substituents and/or any suitable substituent as described herein which satisfy the valencies of the heteroatoms and results in the formation of a stable moiety.

Exemplary substituents on carbon atoms include, but are not limited to, halogen, -CN, -NO₂, -N₃, - SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa},-CO₂H, -CHO, -C(OR^{cc})₂, -CO₂R^{aa}, -OC(=O)R^{aa}, -OCO₂R^{aa}, -C(=O)N(R^{bb})₂, -OC(=O)N(R^{bb})₂, -NR^{bb}C(=O)R^{aa}, -NR^{bb}CO₂R^{aa}, -NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa}, - C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, - SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, -C(=S)N(R^{bb})₂, - C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, -OP(=O)(R^{aa})₂, -OP(=O)(OR^{cc})₂, -P(=O)₂N(R^{bb})₂, -OP(=O)₂N(R^{bb})₂, - P(=O)(NR^{bb})₂, -OP(=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂,-OP(R^{cc})₃, -B(R^{aa})₂, -B(OR^{cc})₂, -BR^{aa}(OR^{cc}), alkyl, haloalkyl, alkenyl, alkynyl, carbocyclyl, heterocyclyl, aryl and heteroaryl, wherein each of the alkyl, alkenyl, alkynyl, carbocyclyl, heterocyclyl, aryl and heteroaryl is independently substituted with 0, 1, 2, 3, 4 or 5 R^{dd} groups;
or two geminal hydrogen on a carbon atom are substituted with =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb} or =NOR^{cc} groups;
each of the R^{aa} is independently selected from alkyl, haloalkyl, alkenyl, alkynyl, carbocyclyl, heterocyclyl, aryl and heteroaryl, or two of the R^{aa} groups are combined to form a heterocyclyl or heteroaryl ring, wherein each of the alkyl, alkenyl, alkynyl, carbocyclyl, heterocyclyl, aryl and heteroaryl is independently substituted with 0, 1, 2, 3, 4 or 5 R^{dd} groups;
each of the R^{bb} is independently selected from hydrogen, -OH, -OR^{aa}, -N(R^{cc})₂, -CN, -C(=O)R^{aa}, - C(=O)N(R^{cc})₂, -CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, - SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, carbocyclyl, heterocyclyl, aryl and heteroaryl, or two R^{bb} groups are combined to form a heterocyclyl or a heteroaryl ring, wherein each of the alkyl, alkenyl, alkynyl, carbocyclyl, heterocyclyl, aryl and heteroaryl is independently substituted with 0, 1 , 2, 3, 4 or 5 R^{dd} groups;
each of the R^{cc} is independently selected from hydrogen, alkyl, haloalkyl, alkenyl, alkynyl, carbocyclyl, heterocyclyl, aryl and heteroaryl, or two R^{cc} groups are combined to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 R^{dd} groups;
each of the R^{dd} is independently selected from halogen, -CN, -NO₂, -N₃, -SO₂H, -SO₃H, -OH, -OR^{ee}, -ON(R^{ff})₂, -N(R^{ff})₂, , -N(R^{ff})₃⁺X⁻, -N(OR^{ee})R^{ff}, -SH, -SR^{ee}, -SSR^{ee}, -C(=O)R^{ee}, -CO₂H, -CO₂R^{ee}, -OC(=O)R^{ee}, -OCO₂R^{ee}, -C(=O)N(R^{f})₂, -OC(=O)N(R^{ff})₂, -NR^{ff}C(=O)R^{ee}, -NR^{ff}CO₂R^{ee}, -NR^{ff}C(=O)N(R^{ff})₂, - C(=NR^{ff})OR^{ee}, -OC(=NR^{ff})R^{ee}, -OC(=NR^{ff})OR^{ee}, -C(=NR^{ff})N(R^{ff})₂, -OC(=NR^{ff})N(R^{ff})₂, - NR^{ff}C(=NR^{ff})N(R^{ff})₂, -NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, - OSi(R^{ee})₃, -C(=S)N(R^{ff})₂, -C(=O)SR^{ee}, -C(=S)SR^{ee}, -SC(=S)SR^{ee}, -P(=O)₂R^{ee}, -P(=O)(R^{ee})2, -OP(=O)(R^{ee})₂, - OP(=O)(OR^{ee})₂, alkyl, haloalkyl, alkenyl, alkynyl, carbocyclyl, heterocyclyl, aryl, heteroaryl, wherein each of the alkyl, alkenyl, alkynyl, carbocyclyl, heterocyclyl, aryl and heteroaryl is independently substituted with 0, 1, 2, 3, 4 or 5 R^{gg} groups, or two geminal R^{dd} substituents can be combined to form =O or =S;
each of the R^{ee} is independently selected from alkyl, haloalkyl, alkenyl, alkynyl, carbocyclyl, aryl, heterocyclyl, and heteroaryl, wherein each of the alkyl, alkenyl, alkynyl, carbocyclyl, heterocyclyl, aryl and heteroaryl is independently substituted with 0, 1, 2, 3, 4 or 5 R^{gg} groups;
each of the R^{ff} is independently selected from hydrogen, alkyl, haloalkyl, alkenyl, alkynyl, carbocyclyl, heterocyclyl, aryl and heteroaryl, or two R^{ff} groups are combined to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 R^{gg} groups;
each of the R^{gg} is independently selected from halogen, -CN, -NO₂, -N₃, -SO₂H, -SO₃H, -OH, -OC₁₋₆ alkyl, -ON(C₁₋₆ alkyl)₂, -N(C₁₋₆alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, - OC(=O)NH(C₁₋₆ alkyl), -NHC(=O)(C₁₋₆ alkyl), -N(C₁₋₆ alkyl)C(=O)(C₁₋₆ alkyl), -NHCO₂(C₁₋₆ alkyl), - NHC(=O)N(C₁₋₆ alkyl)₂, -NHC(=O)NH(C₁₋₆ alkyl), -NHC(=O)NH₂, -C(=NH)O(C₁₋₆ alkyl), -OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, - OC(=NH)N(C₁₋₆ alkyl)₂, -OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), -SO₂N(C₁₋₆ alkyl)₂, -SO₂NH(C₁₋₆ alkyl), -SO₂NH₂, -SO₂C₁₋₆ alkyl, -SO₂OC₁₋₆ alkyl, - OSO₂C₁₋₆ alkyl, -SOC₁₋₆ alkyl, -Si(C₁₋₆ alkyl)₃, -OSi(C₁₋₆ alkyl)₃, -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ haloalkyl, C₂-C₆ alkenyl, C₂-C₆ alkynyl, C₃-C₇ carbocyclyl, C₆-C₁₀ aryl, C₃-C₇ heterocyclyl, C₅-C₁₀ heteroaryl; or two geminal R^{gg} substituents may combine to form =O or =S; wherein X⁻ is a counter-ion.

Exemplary substituents on nitrogen atoms include, but are not limited to, hydrogen, -OH, -OR^{aa}, - N(R^{cc})₂, -CN, -C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, - P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, carbocyclyl, heterocyclyl, aryl and heteroaryl, or two R^{cc} groups attached to a nitrogen atom combine to form a heterocyclyl or a heteroaryl ring, wherein each of the alkyl, alkenyl, alkynyl, carbocyclyl, heterocyclyl, aryl and heteroaryl is independently substituted with 0, 1, 2, 3, 4 or 5 R^{dd} groups, and wherein R^{aa}, R^{bb}, R^{cc} and R^{dd} are as described herein.

### Other definitions

As used herein, "cancer" refers to any disease induced or caused by inappropriately high levels of cell division, inappropriately low levels of apoptosis, or both. Examples of cancer include, but are not limited to, leukemias (e.g., acute leukemia, acute lymphoblastic leukemia, acute myeloid leukemia, acute myelogenous leukemia, acute promyelocytic leukemia, acute myelomonocytic leukemia, acute monocytic leukemia, acute erythrocytic leukemia, chronic leukemia, chronic myelogenous leukemia, chronic lymphocytic leukemia), polycythemia vera, lymphoma (Hodgkin disease, non-Hodgkin disease), Waldenstrom macroglobulinemia, heavy chain disease and solid tumors.

The term "treating" as used herein relates to reversing, alleviating or inhibiting the progression or prevention of the disorders or conditions to which the term applies, or of one or more symptoms of such disorders or conditions. The noun "treatment" as used herein relates to the action of treating, which is a verb, and the latter is as just defined.

The term "pharmaceutically acceptable" as used herein refers to the substance, which are suitable for the contact with patients' tissues within a reliable medical judgment, and do not produce inappropriate toxicity, irritation, allergy, etc. They are commensurate with a reasonable benefit/risk ratio, and are effective for their intended use. The term includes, if possible, the zwitterionic form of the compounds of the disclosure.

The term "salt" refers to a relatively non-toxic addition salt of inorganic and organic acids to the compounds of the present disclosure. These salts can be prepared in situ during the final separation and purification of the compounds, or by isolating salts produced by separately reacting the purified compound in the free base form with a suitable organic or inorganic acid.

The pharmaceutically acceptable base addition salts are formed with metals or amines, such as alkali metal and alkaline earth metal hydroxides or organic amines. Examples of the metals used as cations include sodium, potassium, magnesium, calcium, etc. Examples of suitable amines are N,N'-dibenzylethylenediamine, chloroprocaine, choline, diethanolamine, ethylenediamine, N-methylglucamine and procaine.

The salts can be prepared from the inorganic acids, which include sulfates, pyrosulfates, bisulfates, sulfites, bisulfites, nitrates, phosphates, monohydrogen phosphates, dihydrogen phosphates, metaphosphates, pyrophosphates, chlorides, bromides and iodides. Examples of the acids include hydrochloric acid, nitric acid, sulfuric acid, hydrobromic acid, hydroiodic acid, phosphoric acid, etc. The representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthalate, methanesulfonate, glucoheptanate, lactobionate, lauryl sulfonate, isethionate, etc. The salts can also be prepared from the organic acids, which include aliphatic monocarboxylic and dicarboxylic acids, phenyl-substituted alkanoic acids, hydroxyalkanoic acids, alkanedioic acid, aromatic acids, aliphatic and aromatic sulfonic acids, etc. The representative salts include acetate, propionate, octanoate, isobutyrate, oxalate, malonate, succinate, suberate, sebacate, fumarate, maleate, mandelate, benzoate, chlorobenzoate, methyl benzoate, dinitrobenzoate, naphthoate, besylate, tosylate, phenylacetate, citrate, lactate, maleate, tartrate, methanesulfonate, etc. The pharmaceutically acceptable salts can include cations based on alkali metals and alkaline earth metals, such as sodium, lithium, potassium, calcium, magnesium, etc., as well as non-toxic ammonium, quaternary ammonium, and amine cations including, but not limited to, ammonium, tetramethylammonium, tetraethylammonium, methylamine, dimethylamine, trimethylamine, triethylamine, ethylamine, etc. Salts of amino acids are also included, such as arginine salts, gluconates, galacturonates, etc. (for example, see Berge S. M. et al., "Pharmaceutical Salts, " J. Pharm. Sci., 1977; 66: 1-19 for reference).

"Subjects" to which administration is contemplated include, but are not limited to, humans (e.g., males or females of any age group, e.g., paediatric subjects (e.g., infants, children, adolescents) or adult subjects (e.g., young adults, middle-aged adults or older adults)) and/or non-human animals, such as mammals, e.g., primates (e.g., cynomolgus monkeys, rhesus monkeys), cattle, pigs, horses, sheep, goats, rodents, cats and/or dogs. In some embodiments, the subject is a human. In some embodiments, the subject is a non-human animal. The terms "humam", "patient" and "subject" can be used interchangeably herein.

"Disease," "disorder," and "condition" can be used interchangeably herein.

Unless indicated, otherwise the term "treatment" as used herein includes the effect on a subject who is suffering from a particular disease, disorder, or condition, which reduces the severity of the disease, disorder, or condition, or delays or slows the progression of the disease, disorder or condition ("therapeutic treatment"). The term also includes the effect that occurs before the subject begins to suffer from a specific disease, disorder or condition ("prophylactic treatment").

Generally, the "effective amount" of a compound refers to an amount sufficient to elicit a target biological response. As understood by those skilled in the art, the effective amount of the compound of the disclosure can vary depending on the following factors, such as the desired biological endpoint, the pharmacokinetics of the compound, the diseases being treated, the mode of administration, and the age, health status and symptoms of the subjects. The effective amount includes therapeutically effective amount and prophylactically effective amount.

Unless indicated, otherwise the "therapeutically effective amount" of the compound as used herein is an amount sufficient to provide therapeutic benefits in the course of treating a disease, disorder or condition, or to delay or minimize one or more symptoms associated with the disease, disorder or condition. The therapeutically effective amount of a compound refers to the amount of the therapeutic agent that, when used alone or in combination with other therapies, provides a therapeutic benefit in the treatment of a disease, disorder or condition. The term "therapeutically effective amount" can include an amount that improves the overall treatment, reduces or avoids the symptoms or causes of the disease or condition, or enhances the therapeutic effect of other therapeutic agents.

Unless indicated, otherwise the "prophylactically effective amount" of the compound as used herein is an amount sufficient to prevent a disease, disorder or condition, or an amount sufficient to prevent one or more symptoms associated with a disease, disorder or condition, or an amount sufficient to prevent the recurrence of a disease, disorder or condition. The prophylactically effective amount of a compound refers to the amount of a therapeutic agent that, when used alone or in combination with other agents, provides a prophylactic benefit in the prevention of a disease, disorder or condition. The term "prophylactically effective amount" can include an amount that improves the overall prevention, or an amount that enhances the prophylactic effect of other preventive agents.

"Combination" and related terms refer to the simultaneous or sequential administration of the compounds of the present disclosure and other therapeutic agents. For example, the compounds of the present disclosure can be administered simultaneously or sequentially in separate unit dosage with other therapeutic agents, or simultaneously in a single unit dosage with other therapeutic agents.

### BRIEF DESCRIPTION OF DRAWINGS

Fig. 1 shows the results of western blotting for detecting the phosphorylated Rb at Ser780 and Ser807/811 with the treatment of example I-10.
Fig. 2 shows the results of western blotting for detecting the CDK9 downstream molecular pathways and apoptosis molecular pathways with the treatment of example I-10.
Fig. 3 shows the results of the growth inhibition effects of example I-10 and the control on the OVCAR-3 tumor model.
Fig. 4 shows the results of the growth inhibition effects of example I-10 and the control on the MKN1 tumor model.

### DETAILED DESCRIPTION

As used herein, "compounds of the present disclosure" refer to the compounds of formula (I) below, or pharmaceutically acceptable salts, enantiomers, diastereomers, racemates, solvates, hydrates, polymorphs, prodrugs or isotopic variants thereof, or mixtures thereof.

Compounds are generally described herein using standard nomenclature. It should be understood, unless otherwise specified, that compounds with asymmetric center(s) include all optical isomers and mixtures thereof. Furthermore, unless otherwise specified, all isomer compounds and carbon-carbon double bonds included in the present disclosure may be in the form of Z and E. Compounds which exist in different tautomeric forms, one of which is not limited to any particular tautomer, but is intended to cover all tautomeric forms.

In one embodiment, the present disclosure relates to a compound of formula (I), or a pharmaceutically acceptable salt, an enantiomer, a diastereomer, a racemate, a solvate, a hydrate, a polymorph, a prodrug, or an isotopic variant thereof, or a mixture thereof: 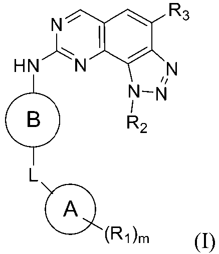 wherein,
L is selected from -C(O)- and -S(O)₂-, alternatively -C(O)-;
ring A is 5- to 10-membered heterocyclyl;
ring B is C₆₋₁₀ aryl;
R₁ is selected from H, D, halogen, -C₀₋₆ alkylene-CN, -C₀₋₆ alkylene-NR₁ₐR_{1b}, -C₀₋₆ alkylene-OR₁ₐ, C₁₋₆ alkyl, C₁₋₆ haloalkyl, C₂₋₆ alkenyl and C₂₋₆ alkynyl;
R₁ₐ is selected from H, D, C₁₋₆ alkyl and C₁₋₆ haloalkyl;
R_{1b} is selected from H, D, C₁₋₆ alkyl and C₁₋₆ haloalkyl;
m is selected from 0, 1, 2, 3, 4 and 5;
R₂ is selected from C₁₋₆ alkyl, C₁₋₆ haloalkyl, C₅₋₁₀ cycloalkyl, 5- to 10-membered heterocyclyl, -C₀₋₆ alkylene-CN, -C₀₋₆ alkylene-NH₂ and -C₁₋₆ alkylene-OH, and the R₂ is optionally substituted with 1, 2, 3, 4 or 5 R₂ₐ;
R₂ₐ is selected from H, D, halogen, ORₐ, CN, NR_{b}R_{c}, C₁₋₆ alkyl and C₁₋₆ haloalkyl;
R₃ is selected from H, D, halogen, C₁₋₆ alkyl and C₁₋₆ haloalkyl;
Rₐ, R_{b} and R_{c} are selected from H, C₁₋₆ alkyl and C₁₋₆ haloalkyl;
each of the above groups may be optionally substituted with one or more deuterium atoms, up to fully deuterated;
with the proviso that, when R₂ is isopropyl and R₃ is H, ring A is not 6-membered heterocyclyl.

### L

In a specific embodiment, L is -C(O)-; in another specific embodiment, L is -S(O)₂-.

### Ring A

In a specific embodiment, ring A is 5- to 10-membered heterocyclyl; in another specific embodiment, ring A is 5- to 8-membered heterocyclyl; in another specific embodiment, ring A is 5-membered heterocyclyl; in another specific embodiment, ring A is 7- to 10-membered heterocyclyl; in another specific embodiment, ring A is 7-membered heterocyclyl.

### m

In a specific embodiment, m is selected from 0, 1, 2, 3, 4 and 5.

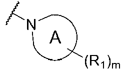

In one embodiment, 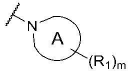 is 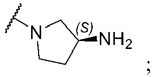 in another specific embodiment, 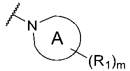 is 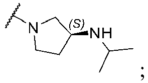 in another specific embodiment, 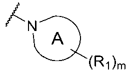 is 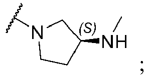 in another specific embodiment, 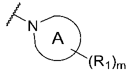 is 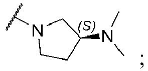 in another specific embodiment, 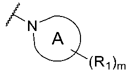 is 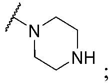 in another specific embodiment, 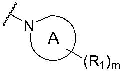 is 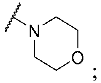 in another specific embodiment, 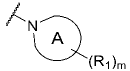 is 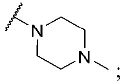 in another specific embodiment, 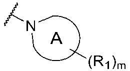 is 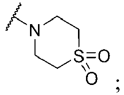 in another specific embodiment, 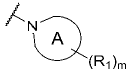 is 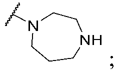 in another specific embodiment, 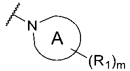 is 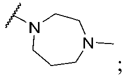 in another specific embodiment, 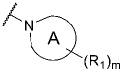 is 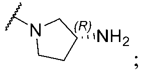 in another specific embodiment, 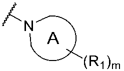 is 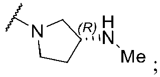 in another specific embodiment, 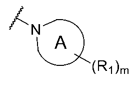 is 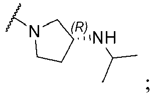 in another specific embodiment, 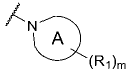 is 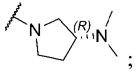 in another specific embodiment, 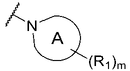 is 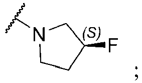 in another specific embodiment, 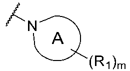 is 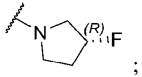 in another specific embodiment, 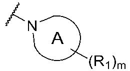 is 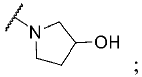 in another specific embodiment, 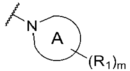 is 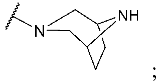 in another specific embodiment, 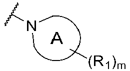 is

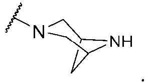

### Ring B

In a specific embodiment, ring B is C₆₋₁₀ aryl.

### R₁

In a specific embodiment, R₁ is H; in another specific embodiment, R₁ is D; in another specific embodiment, R₁ is halogen; in another specific embodiment, R₁ is F; in another specific embodiment, R₁ is - C₀₋₆ alkylene-CN, such as CN; in another specific embodiment, R₁ is -C₀₋₆ alkylene-NR₁ₐR_{1b}; in another specific embodiment, R₁ is NR₁ₐR_{1b}; in another specific embodiment, R₁ is NH₂; in another specific embodiment, R₁ is -C₀₋₆ alkylene-OR₁ₐ; in another specific embodiment, R₁ is -C₁₋₆ alkylene-OH; in another specific embodiment, R₁ is -C₁₋₄ alkylene-OH; in another specific embodiment, R₁ is OH; in another specific embodiment, R₁ is C₁₋₆ alkyl; in another specific embodiment, R₁ is C₁₋₄ alkyl; in another specific embodiment, R₁ is C₁₋₆ haloalkyl; in another specific embodiment, R₁ is C₂₋₆ alkenyl; in another specific embodiment, R₁ is C₂₋₆ alkynyl.

### R₂

In a specific embodiment, R₂ is C₁₋₆ alkyl; in another specific embodiment, R₂ is C₁₋₄ alkyl; in another specific embodiment, R₂ is C₁₋₆ haloalkyl; in another specific embodiment, R₂ is C₅₋₁₀ cycloalkyl; in another specific embodiment, R₂ is C₅₋₈ cycloalkyl; in another specific embodiment, R₂ is 5- to 10-membered heterocyclyl; in another specific embodiment, R₂ is -C₀₋₆ alkylene-CN; in another specific embodiment, R₂ is -C₀₋₆ alkylene-NH₂; in another specific embodiment, R₂ is -C₁₋₆ alkylene-OH; in another specific embodiment, R₂ is -C₁₋₄ alkylene-OH; in another specific embodiment, R₂ is -C₁₋₂ alkylene-OH.

In a more specific embodiment, R₂ is 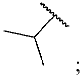 in another more specific embodiment, R₂ is 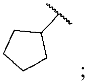 in another more specific embodiment, R₂ is 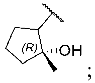 in another more specific embodiment, R₂ is 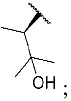 in another more specific embodiment, R₂ is 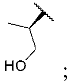 in another more specific embodiment, R₂ is 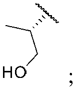 in another more specific embodiment, R₂ is

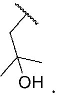

In a specific embodiment, the R₂ is unsubstituted; in another specific embodiment, the R₂ is optionally substituted with n R₂ₐ; in another specific embodiment, the R₂ is optionally substituted with 1, 2, 3, 4 or 5 R₂ₐ.

### R₃

In a specific embodiment, R₃ is H; in another specific embodiment, R₃ is D; in another specific embodiment, R₃ is halogen; in another specific embodiment, R₃ is C₁₋₆ alkyl; in another specific embodiment, R₃ is C₁₋₆ haloalkyl; in another specific embodiment, R₃ is C₁₋₄ haloalkyl, such as CHF₂.

### R₁ₐ

In a specific embodiment, R₁ₐ is H; in another specific embodiment, R₁ₐ is D; in another specific embodiment, R₁ₐ is C₁₋₆ alkyl; in another specific embodiment, R₁ₐ is C₁₋₄ alkyl, such as CH₃ or isopropyl; in another specific embodiment, R₁ₐ is C₁₋₆ haloalkyl.

### R_{1b}

In a specific embodiment, R_{1b} is H; in another specific embodiment, R_{1b} is D; in another specific embodiment, R_{1b} is C₁₋₆ alkyl; in another specific embodiment, R_{1b} is C₁₋₄ alkyl, such as CH₃; in another specific embodiment, R_{1b} is C₁₋₆ haloalkyl.

### R₂ₐ

In a specific embodiment, R₂ₐ is H; in another specific embodiment, R₂ₐ is D; in another specific embodiment, R₂ₐ is halogen; in another specific embodiment, R₂ₐ is ORₐ, such as OH; in another specific embodiment, R₂ₐ is CN; in another specific embodiment, R₂ₐ is NR_{b}R_{c}, such as NH₂; in another specific embodiment, R₂ₐ is C₁₋₆ alkyl; in another specific embodiment, R₂ₐ is C₁₋₄ alkyl, such as CH₃; in another specific embodiment, R₂ₐ is C₁₋₆ haloalkyl;

### R_{2b}

In a specific embodiment, R_{2b} is H; in another specific embodiment, R_{2b} is D; in another specific embodiment, R_{2b} is OH; in another specific embodiment, R_{2b} is CN; in another specific embodiment, R_{2b} is NH₂; in another specific embodiment, R_{2b} is C₁₋₆ alkyl; in another specific embodiment, R_{2b} is C₁₋₄ alkyl, such as CH₃; in another specific embodiment, R_{2b} is C₁₋₆ haloalkyl.

### R_{2c}

In a specific embodiment, R_{2c} is H; in another specific embodiment, R_{2c} is D; in another specific embodiment, R_{2c} is C₁₋₆ alkyl; in another specific embodiment, R_{2c} is C₁₋₄ alkyl, such as CH₃; in another specific embodiment, R_{2c} is C₁₋₆ haloalkyl.

### n

In a specific embodiment, n is selected from 0, 1, 2, 3, 4 and 5.

### Rₐ, R_{b}, and R_{c}

In a specific embodiment, Rₐ is H; in another specific embodiment, Rₐ is C₁₋₆ alkyl, such as C₁₋₄ alkyl; in another specific embodiment, Rₐ is C₁₋₆ haloalkyl.

In a specific embodiment, R_{b} is H; in another specific embodiment, R_{b} is C₁₋₆ alkyl, such as C₁₋₄ alkyl; in another specific embodiment, R_{b} is C₁₋₆ haloalkyl.

In a specific embodiment, R_{c} is H; in another specific embodiment, R_{c} is C₁₋₆ alkyl, such as C₁₋₄ alkyl; in another specific embodiment, R_{c} is C₁₋₆ haloalkyl.
each of the above groups may be optionally substituted with one or more deuterium atoms, up to fully deuterated.

Any technical solution in any one of the above specific embodiments, or any combination thereof, may be combined with any technical solution in other specific embodiments or any combination thereof. For example, any technical solution of L or any combination thereof may be combined with any technical solution of ring A, ring B, R₁-R₃, m and n, etc., or any combination thereof. The present disclosure is intended to include all combination of such technical solutions, which are not exhaustively listed here to save space.

In a specific embodiment, the present disclosure provides a compound of formula (II), or a pharmaceutically acceptable salt, an enantiomer, a diastereomer, a racemate, a solvate, a hydrate, a polymorph, a prodrug, or an isotopic variant thereof, or a mixture thereof, wherein, 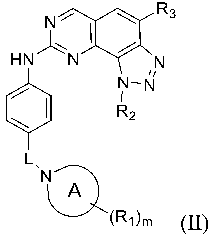 wherein,
L is selected from -C(O)- and -S(O)₂-, alternatively -C(O)-;
ring A is 5- to 10-membered heterocyclyl;
R₁ is selected from H, D, halogen, OH, CN, NR₁ₐR_{1b}, -C₁₋₆ alkylene-OH, C₁₋₆ alkyl and C₁₋₆ haloalkyl;
R₁ₐ is selected from H, D and C₁₋₆ alkyl;
R_{1b} is selected from H, D and C₁₋₆ alkyl;
m is selected from 0, 1, 2, 3, 4 and 5;
R₂ is selected from C₁₋₆ alkyl, C₁₋₆ haloalkyl, C₅₋₁₀ cycloalkyl and -C₁₋₆ alkylene-OH, and the R₂ is optionally substituted with 1, 2, 3, 4 or 5 R₂ₐ;
R₂ₐ is selected from H, D, OH and C₁₋₆ alkyl;
R₃ is selected from H, D, halogen and C₁₋₆ haloalkyl, alternatively H or CHF₂, yet alternatively H;
each of the above groups may be optionally substituted with one or more deuterium atoms, up to fully deuterated.

In a specific embodiment, the present disclosure provides a compound of the above formula (I) or (II), or a pharmaceutically acceptable salt, an enantiomer, a diastereomer, a racemate, a solvate, a hydrate, a polymorph, a prodrug, or an isotopic variant thereof, or a mixture thereof, wherein,
R₂ is selected from C₁₋₄ alkyl, C₅₋₈ cycloalkyl and -C₁₋₄ alkylene-OH, and the R₂ is optionally substituted with 1, 2 or 3 R₂ₐ;
R₂ₐ is selected from H, D, OH and C₁₋₆ alkyl, alternatively H, D, OH or CH₃;
alternatively,
R₂ is selected from 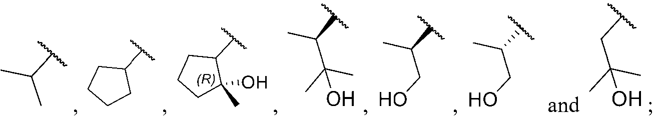
yet alternatively, R₂ is

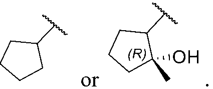

In a specific embodiment, the present disclosure provides a compound of the above formula (I) or (II), or a pharmaceutically acceptable salt, an enantiomer, a diastereomer, a racemate, a solvate, a hydrate, a polymorph, a prodrug, or an isotopic variant thereof, or a mixture thereof, wherein,
ring A is 5- to 8-membered heterocyclyl;
R₁ is selected from H, D, halogen, OH, CN, NR₁ₐR_{1b}, -C₁₋₄ alkylene-OH and C₁₋₄ alkyl;
R₁ₐ is selected from H, D and C₁₋₄ alkyl, alternatively, R₁ₐ is selected from H, CH₃ and isopropyl;
R_{1b} is selected from H, D and C₁₋₄ alkyl, alternatively, R_{1b} is selected from H and CH₃;
m is selected from 0, 1, 2 and 3;
alternatively, 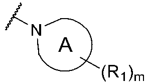 is selected from 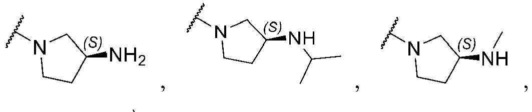 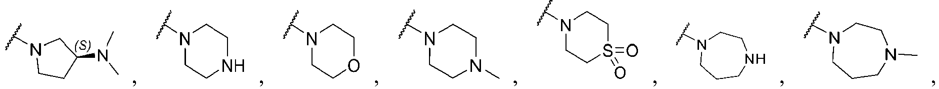 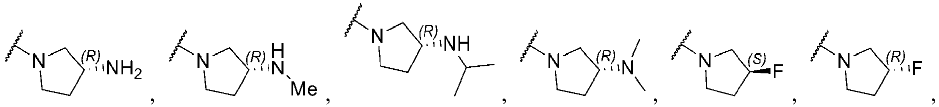 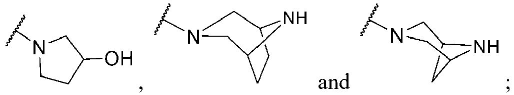
yet alternatively, 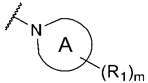 is selected from

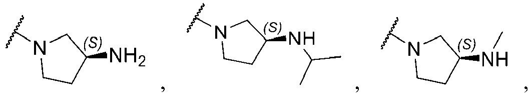

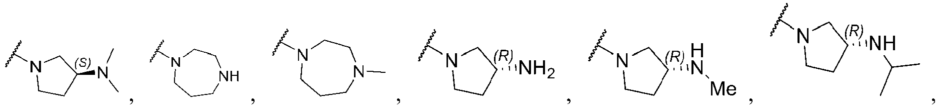

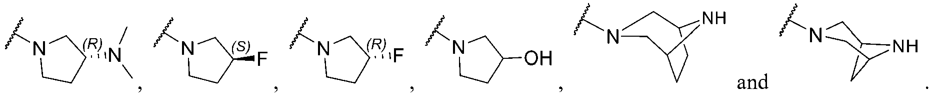

In a specific embodiment, the present disclosure provides a compound of the above formula (I) or (II), or a pharmaceutically acceptable salt, an enantiomer, a diastereomer, a racemate, a solvate, a hydrate, a polymorph, a prodrug, or an isotopic variant thereof, or a mixture thereof, and the compound is a compound of formula (III): 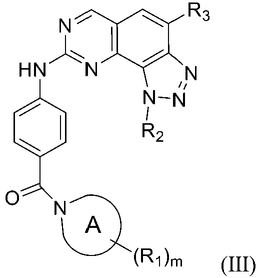 wherein,
ring A is 5- to 8-membered heterocyclyl;
R₁ is selected from H, D, halogen, OH, CN, NR₁ₐR_{1b}, -C₁₋₄ alkylene-OH and C₁₋₄ alkyl;
R₁ₐ is selected from H, D and C₁₋₄ alkyl, alternatively, R₁ₐ is selected from H, CH₃ and isopropyl;
R_{1b} is selected from H, D and C₁₋₄ alkyl, alternatively, R_{1b} is selected from H and CH₃;
m is selected from 0, 1, 2 and 3;
R₂ is selected from C₁₋₄ alkyl, C₅₋₈ cycloalkyl and -C₁₋₄ alkylene-OH, and the R₂ is optionally substituted with 1, 2 or 3 R₂ₐ;
R₂ₐ is selected from H, D, OH and C₁₋₆ alkyl, alternatively H, OH or CH₃;
R₃ is selected from H, D and C₁₋₆ haloalkyl, alternatively H or CHF₂, yet alternatively H;
each of the above groups may be optionally substituted with one or more deuterium atoms, up to fully deuterated.

In a specific embodiment, the present disclosure provides a compound of the above formula (I) or (II), or a pharmaceutically acceptable salt, an enantiomer, a diastereomer, a racemate, a solvate, a hydrate, a polymorph, a prodrug, or an isotopic variant thereof, or a mixture thereof, wherein: 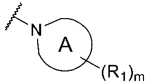 is selected from 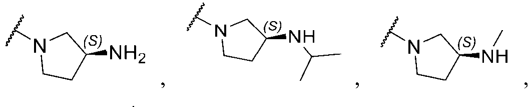 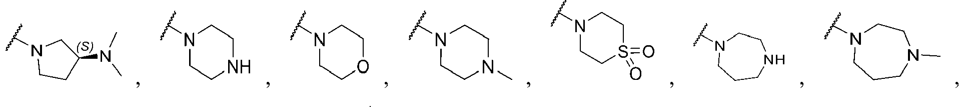 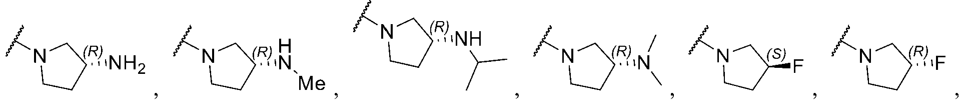 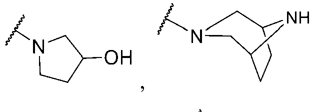 and 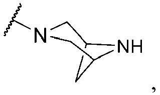 alternatively, 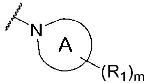 is selected from 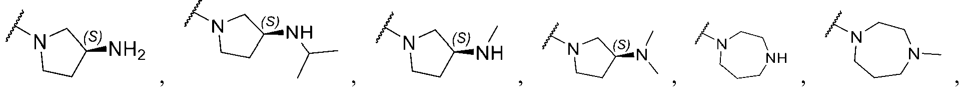 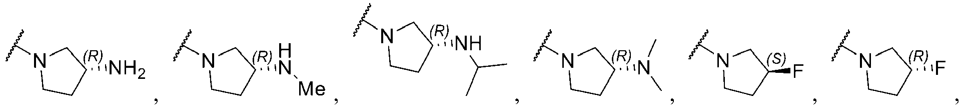 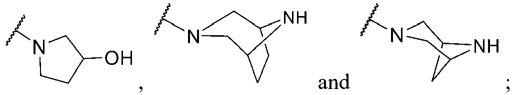
R₂ is selected from isopropyl, cyclopentyl and -C₁₋₂ alkylene-OH, and the R₂ is optionally substituted with 1, 2 or 3 R₂ₐ;
R₂ₐ is selected from H, D, OH and C₁₋₄ alkyl, alternatively selected from H, OH and CH₃;
alternatively, R₂ is selected from 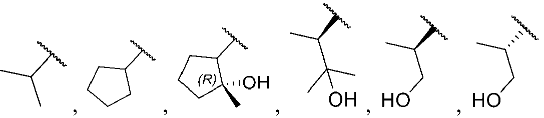 and 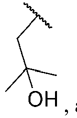 alternatively 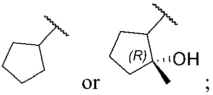
R₃ is selected from H, D and C₁₋₄ haloalkyl, alternatively H or CHF₂, yet alternatively H;
each of the above groups may be optionally substituted with one or more deuterium atoms, up to fully deuterated.

In a specific embodiment, the present disclosure provides a compound of the above formula (I) or (II), or a pharmaceutically acceptable salt, an enantiomer, a diastereomer, a racemate, a solvate, a hydrate, a polymorph, a prodrug, or an isotopic variant thereof, or a mixture thereof, wherein, the compound has the following structure: 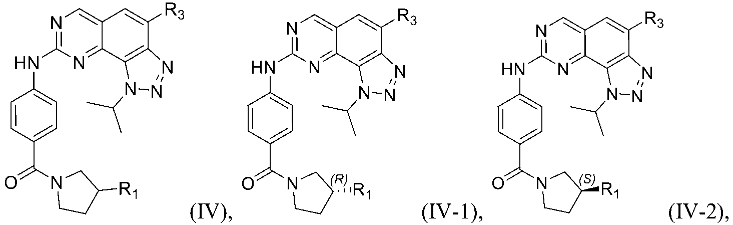 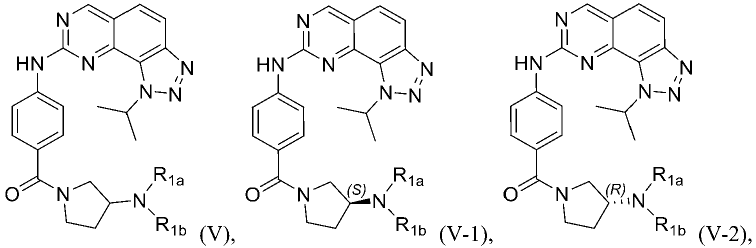 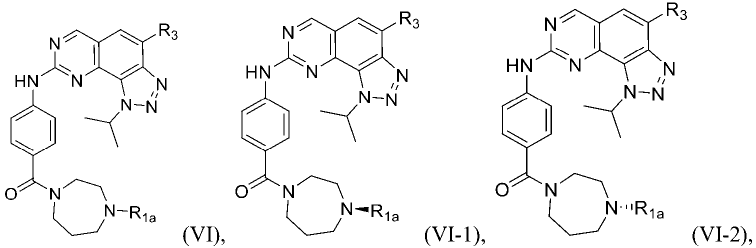 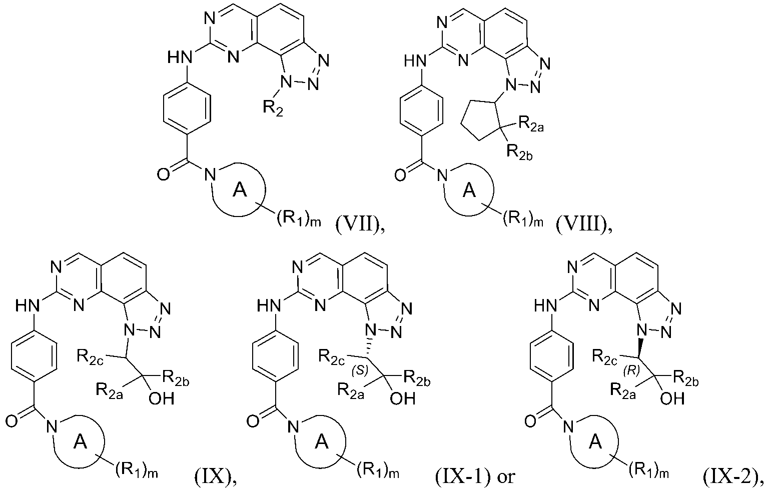 wherein,
R_{2b} is selected from H, D, OH, CN, NH₂, C₁₋₆ alkyl and C₁₋₆ haloalkyl;
R_{2c} is selected from H, D, C₁₋₆ alkyl and C₁₋₆ haloalkyl;
the remaining variables are as defined above.

In a specific embodiment, the present disclosure provides a compound of formula (IV), (IV-1) or (IV-2), or a pharmaceutically acceptable salt, an enantiomer, a diastereomer, a racemate, a solvate, a hydrate, a polymorph, a prodrug, or an isotopic variant thereof, or a mixture thereof, and the compound is: 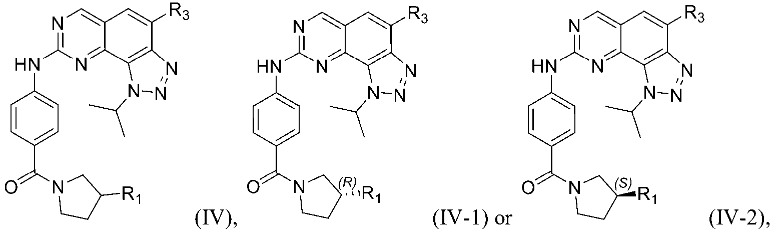 wherein,
R₁ is selected from halogen, OH, CN and NR₁ₐR_{1b};
R₁ₐ is selected from H, D, C₁₋₆ alkyl and C₁₋₆ haloalkyl;
R_{1b} is selected from H, D, C₁₋₆ alkyl and C₁₋₆ haloalkyl;
R₃ is selected from H, D, C₁₋₆ alkyl and C₁₋₆ haloalkyl, alternatively H;
each of the above groups may be optionally substituted with one or more deuterium atoms, up to fully deuterated.

In a specific embodiment, the present disclosure provides a compound of the above formula (IV), (IV-1) or (IV-2), or a pharmaceutically acceptable salt, an enantiomer, a diastereomer, a racemate, a solvate, a hydrate, a polymorph, a prodrug, or an isotopic variant thereof, or a mixture thereof, wherein:
R₁ is selected from halogen, OH, CN and NR₁ₐR_{1b};
R₁ₐ is selected from H, D and C₁₋₄ alkyl;
R_{1b} is selected from H, D and C₁₋₄ alkyl;
R₃ is selected from H, D and C₁₋₄ haloalkyl, alternatively H;
each of the above groups may be optionally substituted with one or more deuterium atoms, up to fully deuterated.

In a specific embodiment, the present disclosure provides a compound of the above formula (IV), (IV-1) or (IV-2), or a pharmaceutically acceptable salt, an enantiomer, a diastereomer, a racemate, a solvate, a hydrate, a polymorph, a prodrug, or an isotopic variant thereof, or a mixture thereof, wherein:
R₁ is selected from F, OH and NR₁ₐR_{1b};
R₁ₐ is selected from H, CH₃ and isopropyl;
R_{1b} is selected from H and CH₃;
R₃ is selected from H and CHF₂, alternatively H.

In a specific embodiment, the present disclosure provides a compound of formula (V), (V-1) or (V-2), or a pharmaceutically acceptable salt, an enantiomer, a diastereomer, a racemate, a solvate, a hydrate, a polymorph, a prodrug, or an isotopic variant thereof, or a mixture thereof: 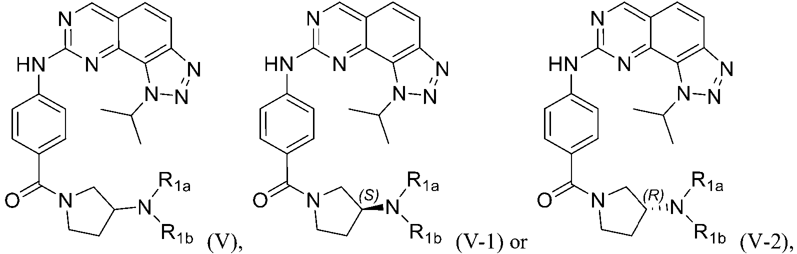 wherein,
R₁ₐ is selected from H, D, C₁₋₆ alkyl and C₁₋₆ haloalkyl;
R_{1b} is selected from H, D, C₁₋₆ alkyl and C₁₋₆ haloalkyl;
alternatively,
R₁ₐ is selected from H, D and C₁₋₄ alkyl;
R_{1b} is selected from H, D and C₁₋₄ alkyl;
yet alternatively,
R₁ₐ is selected from H, CH₃ and isopropyl;
R_{1b} is selected from H and CH₃;
each of the above groups may be optionally substituted with one or more deuterium atoms, up to fully deuterated.

In a specific embodiment, the present disclosure provides a compound of the above formula (V), (V-1) or (V-2), or a pharmaceutically acceptable salt, an enantiomer, a diastereomer, a racemate, a solvate, a hydrate, a polymorph, a prodrug, or an isotopic variant thereof, or a mixture thereof, and the compound is a compound of formula (VI), (VI-1) or (VI-2): 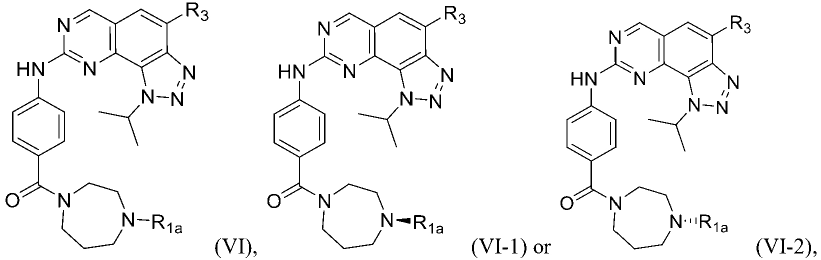 wherein,
R₁ₐ is selected from H, D, C₁₋₆ alkyl and C₁₋₆ haloalkyl;
R₃ is selected from H, D, C₁₋₆ alkyl and C₁₋₆ haloalkyl, alternatively H;
alternatively,
R₁ₐ is selected from H, D and C₁₋₄ alkyl, alternatively H or methyl;
R₃ is selected from H, D and C₁₋₄ haloalkyl, alternatively H or CHF₂, yet alternatively H;
each of the above groups may be optionally substituted with one or more deuterium atoms, up to fully deuterated.

In a specific embodiment, the present disclosure provides a compound of formula (VII), or a pharmaceutically acceptable salt, an enantiomer, a diastereomer, a racemate, a solvate, a hydrate, a polymorph, a prodrug, or an isotopic variant thereof, or a mixture thereof: 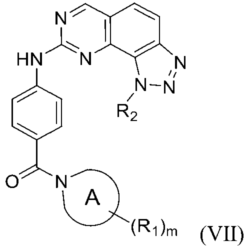 wherein,
ring A is 5- to 10-membered heterocyclyl;
R₁ is selected from H, D, OH, CN, NH₂, C₁₋₆ alkyl, C₁₋₆ haloalkyl and NR₁ₐR_{1b};
R₁ₐ is selected from H, D, C₁₋₆ alkyl and C₁₋₆ haloalkyl;
R_{1b} is selected from H, D, C₁₋₆ alkyl and C₁₋₆ haloalkyl;
m is selected from 0, 1, 2, 3, 4 and 5;
R₂ is C₅₋₁₀ cycloalkyl, alternatively cyclopentyl, and the R₂ is optionally substituted with n R₂ₐ;
R₂ₐ is selected from H, D, OH, CN, NH₂, C₁₋₆ alkyl and C₁₋₆ haloalkyl;
n is selected from 0, 1, 2, 3, 4 and 5;
alternatively,
ring A is selected from 5-membered heterocyclyl and 7- to 10-membered heterocyclyl;
each of the above groups may be optionally substituted with one or more deuterium atoms, up to fully deuterated.

In a specific embodiment, the present disclosure provides a compound of the above formula (VII), or a pharmaceutically acceptable salt, an enantiomer, a diastereomer, a racemate, a solvate, a hydrate, a polymorph, a prodrug, or an isotopic variant thereof, or a mixture thereof, wherein,
ring A is 5- to 8-membered heterocyclyl;
R₁ is selected from H, D, C₁₋₄ alkyl and NR₁ₐR_{1b};
R₁ₐ is selected from H, D and C₁₋₄ alkyl, alternatively H, CH₃ or isopropyl;
R_{1b} is selected from H, D and C₁₋₄ alkyl, alternatively H or CH₃;
m is selected from 0, 1, 2 and 3;
R₂ is C₅₋₈ cycloalkyl, alternatively cyclopentyl, and the R₂ is optionally substituted with n R₂ₐ;
R₂ₐ is selected from H, OH and C₁₋₄ alkyl;
n is selected from 0, 1, 2 and 3;
alternatively,
ring A is selected from 5-membered heterocyclyl and 7-membered heterocyclyl;
each of the above groups may be optionally substituted with one or more deuterium atoms, up to fully deuterated.

In a specific embodiment, the present disclosure provides a compound of the above formula (VII), or a pharmaceutically acceptable salt, an enantiomer, a diastereomer, a racemate, a solvate, a hydrate, a polymorph, a prodrug, or an isotopic variant thereof, or a mixture thereof, wherein, 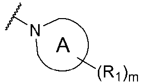 is selected from 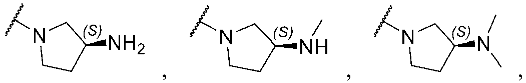 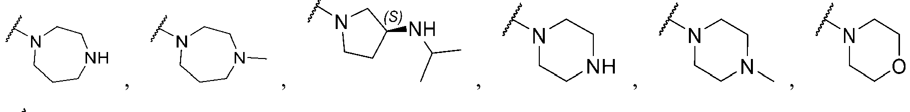 and 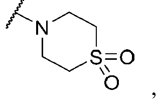 alternatively 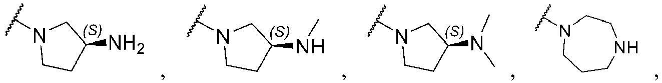 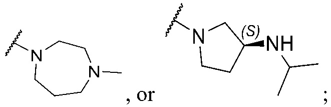
R₂ is cyclopentyl, and the R₂ is optionally substituted with n R₂ₐ;
R₂ₐ is selected from H, OH and CH₃;
n is selected from 0, 1 and 2;
alternatively, R₂ is

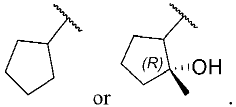

In a specific embodiment, the present disclosure provides a compound of formula (VIII), or a pharmaceutically acceptable salt, an enantiomer, a diastereomer, a racemate, a solvate, a hydrate, a polymorph, a prodrug, or an isotopic variant thereof, or a mixture thereof, and the compound is a compound of formula (VIII): 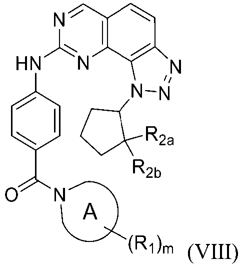 wherein,
R_{2b} is selected from H, D, OH, CN, NH₂, C₁₋₆ alkyl and C₁₋₆ haloalkyl;
alternatively,
R_{2b} is selected from H, D, OH and C₁₋₄ alkyl;
yet alternatively,
R_{2b} is selected from H, D and CH₃;
the remaining variables are as defined above.

In a specific embodiment, the present disclosure provides a compound of formula (IX), (IX-1) or (IX-2), or a pharmaceutically acceptable salt, an enantiomer, a diastereomer, a racemate, a solvate, a hydrate, a polymorph, a prodrug, or an isotopic variant thereof, or a mixture thereof: 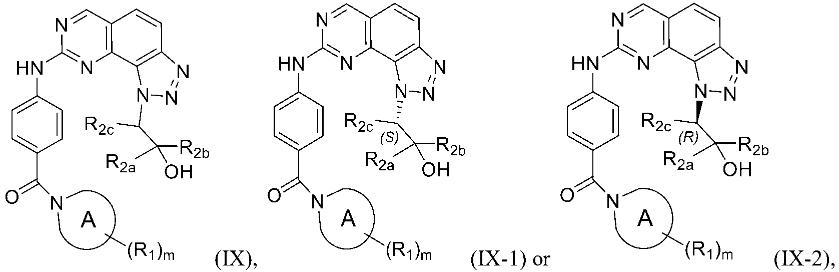 wherein,
ring A is 5- to 10-membered heterocyclyl;
R₁ is selected from H, D, OH, CN, C₁₋₆ alkyl, C₁₋₆ haloalkyl and NR₁ₐR_{1b};
R₁ₐ is selected from H, D, C₁₋₆ alkyl and C₁₋₆ haloalkyl;
R_{1b} is selected from H, D, C₁₋₆ alkyl and C₁₋₆ haloalkyl;
m is selected from 0, 1, 2, 3, 4 and 5;
R₂ₐ is selected from H, D, C₁₋₆ alkyl and C₁₋₆ haloalkyl;
R_{2b} is selected from H, D, C₁₋₆ alkyl and C₁₋₆ haloalkyl;
R_{2c} is selected from H, D, C₁₋₆ alkyl and C₁₋₆ haloalkyl;
alternatively,
ring A is selected from 5-membered heterocyclyl and 7-membered heterocyclyl;
each of the above groups may be optionally substituted with one or more deuterium atoms, up to fully deuterated.

In a specific embodiment, the present disclosure provides a compound of the above formula (IX), (IX-1) or (IX-2), or a pharmaceutically acceptable salt, an enantiomer, a diastereomer, a racemate, a solvate, a hydrate, a polymorph, a prodrug, or an isotopic variant thereof, or a mixture thereof, wherein:
ring A is 5- to 8-membered heterocyclyl;
R₁ is selected from H, D, C₁₋₄ alkyl and NR₁ₐR_{1b};
R₁ₐ is selected from H, D and C₁₋₄ alkyl, alternatively selected from H, CH₃ and isopropyl;
R_{1b} is selected from H, D and C₁₋₄ alkyl, alternatively H or CH₃;
m is selected from 0, 1, 2 and 3;
R₂ₐ is selected from H, D and C₁₋₄ alkyl, alternatively H or CH₃;
R_{2b} is selected from H, D and C₁₋₄ alkyl, alternatively H or CH₃;
R_{2c} is selected from H, D and C₁₋₄ alkyl, alternatively H or CH₃;
alternatively,
ring A is selected from 5-membered heterocyclyl and 7-membered heterocyclyl;
each of the above groups may be optionally substituted with one or more deuterium atoms, up to fully deuterated.

In a specific embodiment, the present disclosure provides a compound of the above formula (IX), (IX-1) or (IX-2), or a pharmaceutically acceptable salt, an enantiomer, a diastereomer, a racemate, a solvate, a hydrate, a polymorph, a prodrug, or an isotopic variant thereof, or a mixture thereof, wherein: 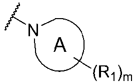 is selected from 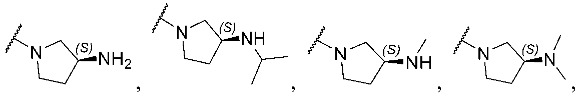 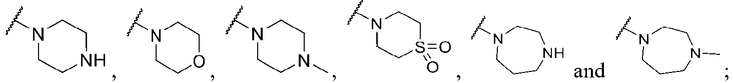
R₂ₐ is selected from H and CH₃;
R_{2b} is selected from H and CH₃;
R_{2c} is selected from H and CH₃;
each of the above groups may be optionally substituted with one or more deuterium atoms, up to fully deuterated.

The compounds of the present disclosure may include one or more asymmetric centers, and thus may exist in a variety of stereoisomeric forms, for example, enantiomers and/or diastereomers. For example, the compounds of the present disclosure may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.

It will be understood by those skilled in the art that the organic compounds can form complexes with solvents in which they are reacted or from which they are precipitated or crystallized. These complexes are known as "solvates." Where the solvent is water, the complex is known as "hydrate." The present disclosure encompasses all solvates of the compounds of the present disclosure.

The term "solvate" refers to forms of a compound or a salt thereof, which are associated with a solvent, usually by a solvolysis reaction. This physical association may include hydrogen bonding. Conventional solvents include water, methanol, ethanol, acetic acid, DMSO, THF, diethyl ether, etc. The compounds described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula R·x H₂O, wherein R is the compound, and x is a number greater than 0. Given compounds can form more than one type of hydrates, including, for example, monohydrates (x is 1), lower hydrates (x is a number greater than 0 and smaller than 1, for example, hemihydrates (R·0.5 H₂O)) and polyhydrates (x is a number greater than 1, for example, dihydrates (R·2 H₂O) and hexahydrates (R·6 H₂O)).

Compounds of the present disclosure may be in an amorphous or a crystalline form (polymorph). Furthermore, the compounds of the present disclosure may exist in one or more crystalline forms. Therefore, the present disclosure includes all amorphous or crystalline forms of the compounds of the present disclosure within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

The present disclosure also comprises compounds that are labeled with isotopes (isotopic variants), which are equivalent to those described in formula (I), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as ²H, ³H, ¹³C, ¹¹C, ¹⁴C, ¹⁵ N, ¹⁸O, ¹⁷O, ³¹P, ³²P, ³⁵S, ¹⁸F and ³⁶Cl, respectively. Compounds of the present disclosure that comprise the above isotopes and/or other isotopes of other atoms, prodrugs thereof and pharmaceutically acceptable salts of said compounds or prodrugs all are within the scope of the present disclosure. Certain isotope-labeled compounds of the present disclosure, such as those incorporating radioactive isotopes (e.g., ³H and ¹⁴C), can be used for the measurement of the distribution of drug and/or substrate in tissue. Tritium, which is ³H and carbon-14, which is ¹⁴C isotope, are yet alternative, because they are easy to prepare and detect. Furthermore, replaced by heavier isotopes, such as deuterium, which is ²H, may provide therapeutic benefits due to the higher metabolic stability, such as prolonging the half-life in vivo or decreasing the dosage requirements, and thus may be alternative in some cases. Isotope-labeled compounds of formula (I) of the present disclosure and prodrugs thereof can be prepared generally by using readily available isotope-labeled reagents to replace non-isotope-labeled reagents in the following schemes and/or the procedures disclosed in the examples and preparation examples.

In addition, prodrugs are also included within the context of the present disclosure. The term "prodrug" as used herein refers to a compound that is converted into an active form that has medical effects in vivo by, for example, hydrolysis in blood. Pharmaceutically acceptable prodrugs are described in T. Higuchi and V. Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19(2) 115-130, each of which is incorporated herein by reference.

The present disclosure also provides a pharmaceutical formulation comprising a therapeutically effective amount of a compound of formula (I), or a therapeutically acceptable salt thereof, and pharmaceutically acceptable carriers, diluents or excipients thereof. All of these forms belong to the present disclosure.

The alternative compounds disclosed herein include but are not limited to the compounds listed below, or a pharmaceutically acceptable salt, an enantiomer, a diastereomer, a racemate, a solvate, a hydrate, a polymorph, a prodrug, or an isotopic variant thereof, or a mixture thereof:

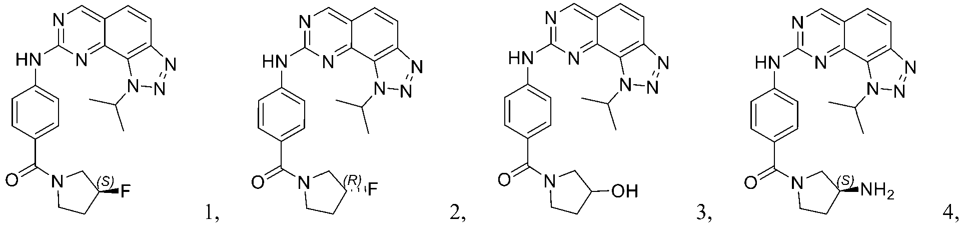

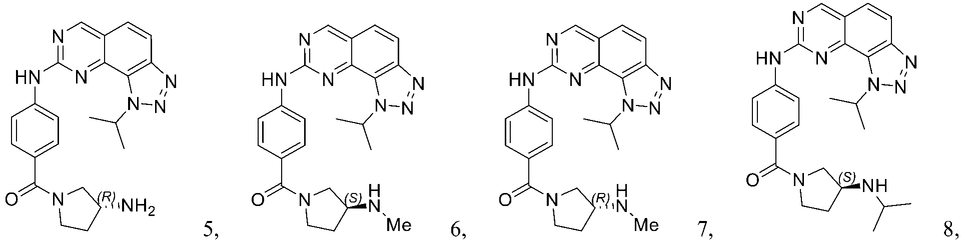

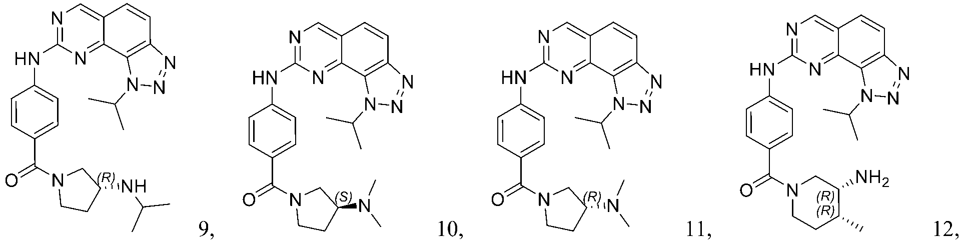

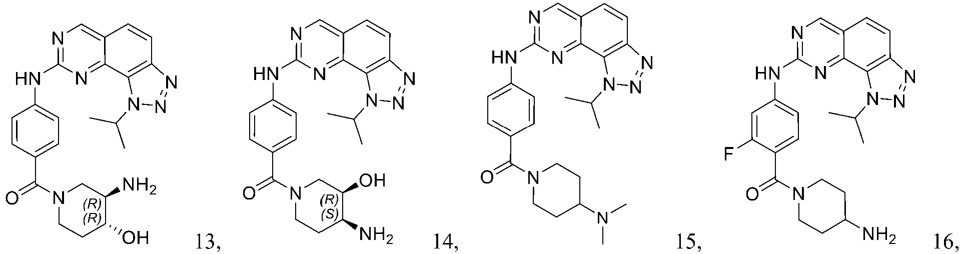

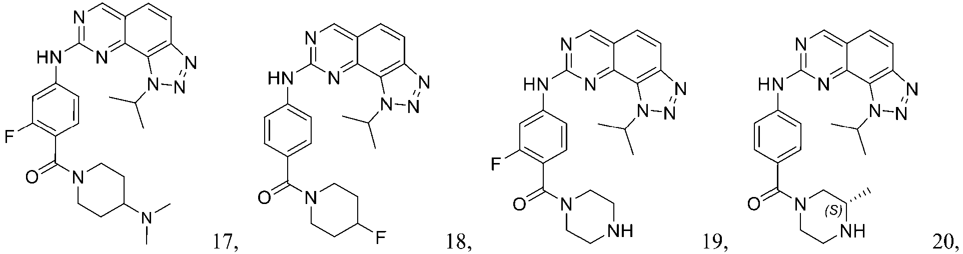

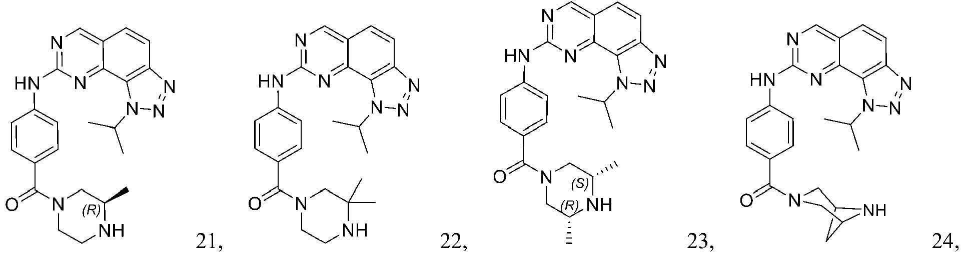

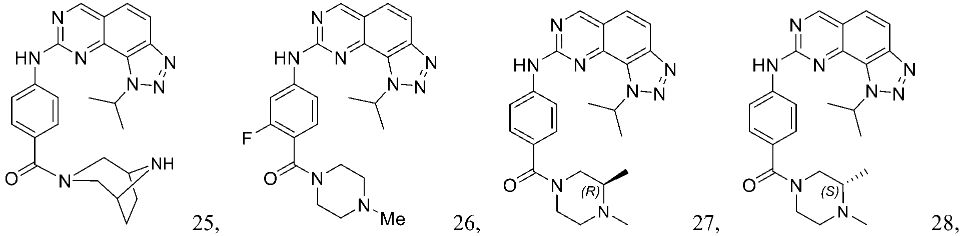

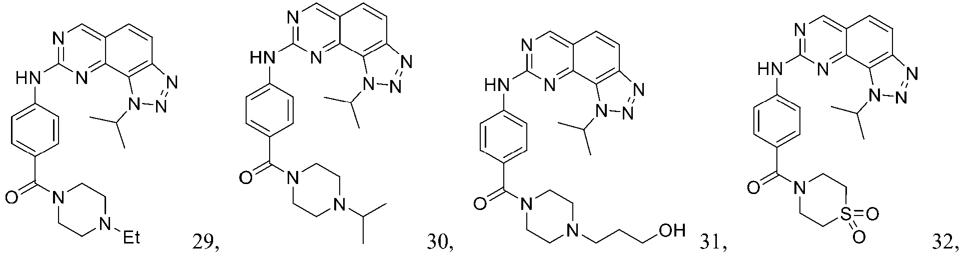

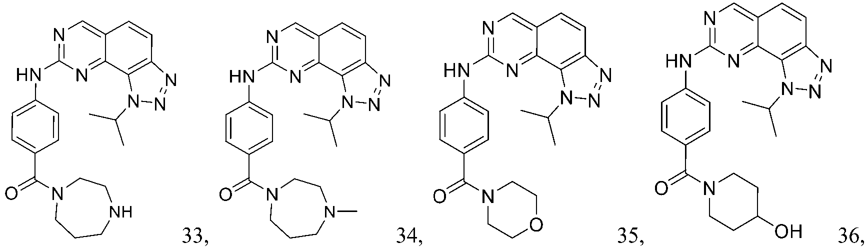

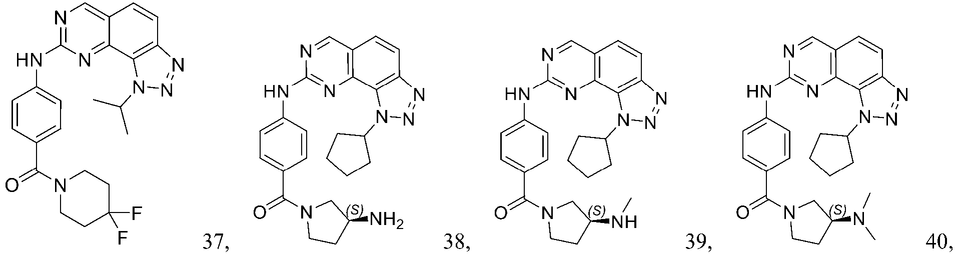

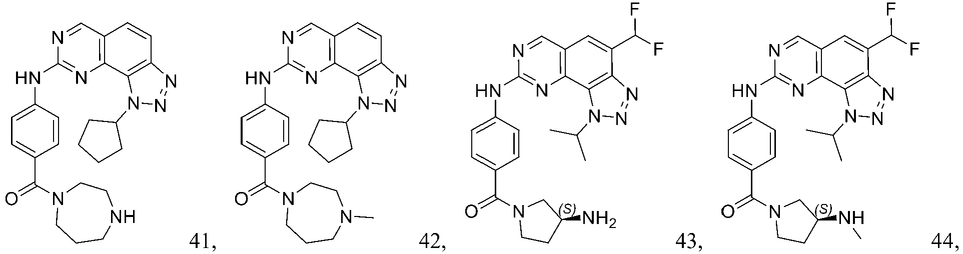

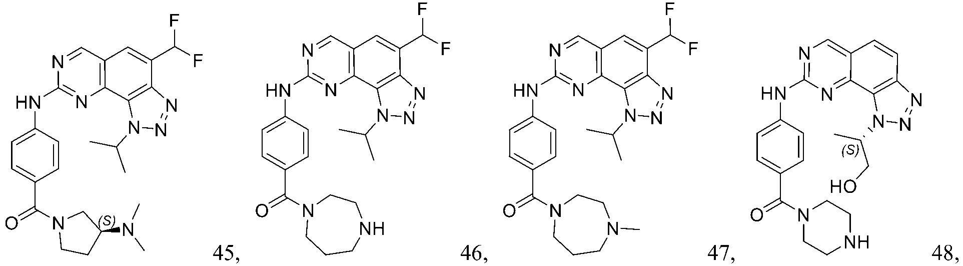

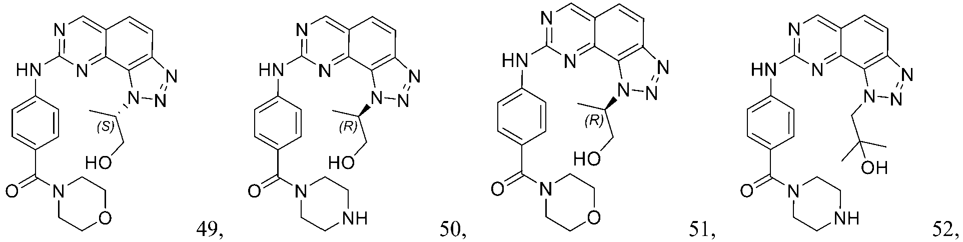

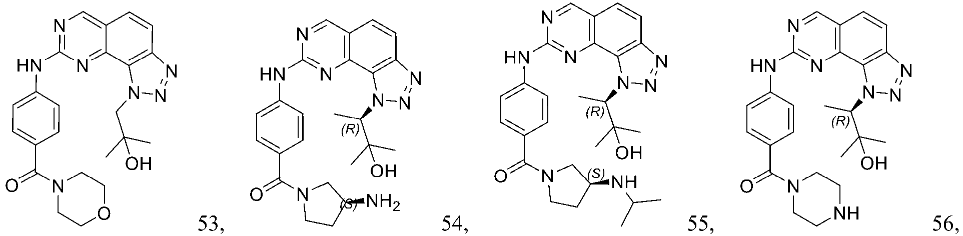

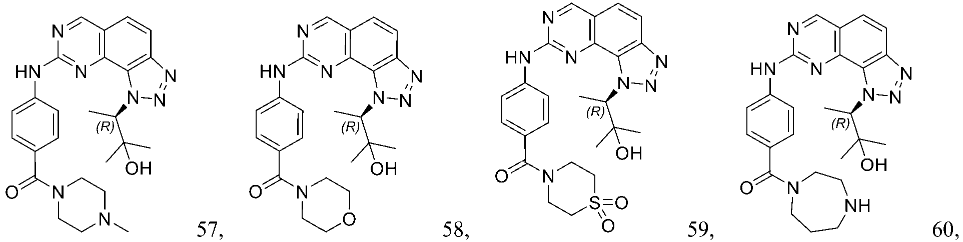

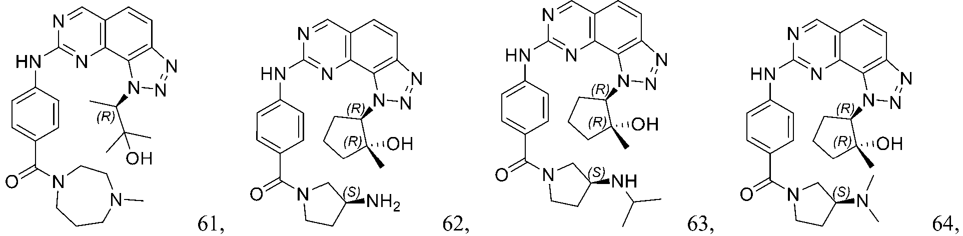

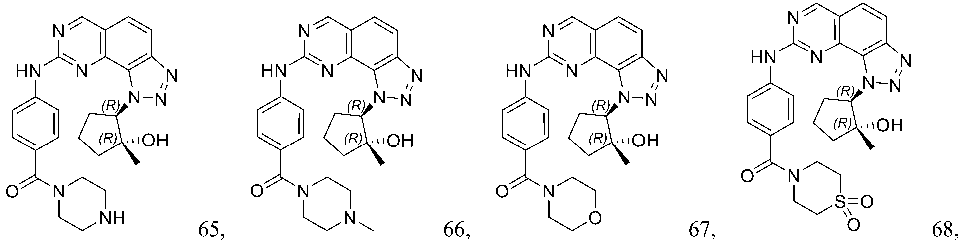

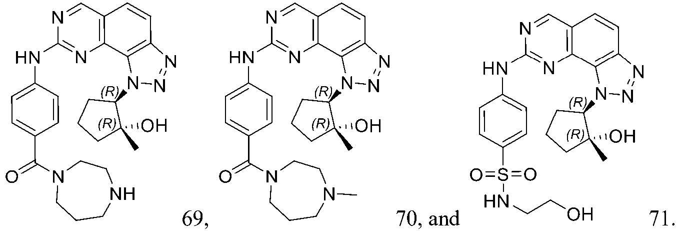

### Pharmaceutical compositions, kits and administration

In another aspect, the present disclosure provides a pharmaceutical composition comprising a compound of the present disclosure (also referred to as the "active ingredient") and pharmaceutically acceptable excipients. In some embodiments, the pharmaceutical composition comprises an effective amount of the compound of the present disclosure. In some embodiments, the pharmaceutical composition comprises a therapeutically effective amount of the compound of the present disclosure. In some embodiments, the pharmaceutical composition comprises a prophylactically effective amount of the compound of the present disclosure.

Pharmaceutically acceptable excipients for use in the present disclosure refer to the non-toxic carriers, adjuvants or vehicles, which do not destroy the pharmacological activity of the compounds formulated together. Pharmaceutically acceptable carriers, adjuvants, or vehicles that can be used in the compositions of the present disclosure include (but are not limited to) ion exchangers, alumina, aluminum stearate, lecithin, serum proteins (such as human serum proteins), buffer substances (such as phosphate), glycine, sorbic acid, potassium sorbate, mixture of partial glycerides of saturated plant fatty acids, water, salts or electrolytes (such as protamine sulfate), disodium hydrogen phosphate, potassium hydrogen phosphate, sodium chloride, zinc salts, silica gel, magnesium trisilicate, polyvinyl pyrrolidone, cellulose-based substance, polyethylene glycol, sodium carboxymethyl cellulose, polyacrylate, wax, polyethylene-polyoxypropylene block polymers, polyethylene glycol and lanolin.

The present disclosure also includes kits (e.g., pharmaceutical packs). The kits provided may include a compound of the present disclosure, other therapeutic agent(s), and a first and a second containers (e.g., vials, ampoules, bottles, syringes, and/or dispersible packages or other suitable containers) containing the compound of the present disclosure and other therapeutic agent(s). In some embodiments, the provided kits can also optionally include a third container containing a pharmaceutically acceptable excipient for diluting or suspending the compound of the present disclosure and/or other therapeutic agent(s). In some embodiments, the compound of the present disclosure provided in the first container and other therapeutic agent(s) provided in the second container are combined to form a unit dosage form.

The pharmaceutical composition provided by the present disclosure can be administered by a variety of routes including, but not limited to, oral administration, parenteral administration, inhalation administration, topical administration, rectal administration, nasal administration, buccal administration, vaginal administration, administration by implant or other means of administration. For example, parenteral administration as used herein includes subcutaneous administration, intradermal administration, intravenous administration, intramuscular administration, intra-articular administration, intra-arterial administration, intrasynovial administration, intra sternal administration, intracerebroventricular administration, intralesional administration, and intracranial injection or infusion techniques.

Generally, the compounds provided herein are administered in an effective amount. The amount of the compound actually administered can be determined by a physician, in the light of the relevant circumstances, including the condition to be treated, the route of administration selected, the actual compound administered, the age, weight and response of the individual patient, the severity of the patient's symptoms, etc.

### Examples

The following examples are provided to provide those skilled in the art with a complete disclosure and description of how to implement, prepare and evaluate the methods and compounds claimed herein, and are intended to be illustrative only without limiting the scope of the present disclosure.

The preparation protocol of a compound disclosed herein is shown, for example, in scheme 1.

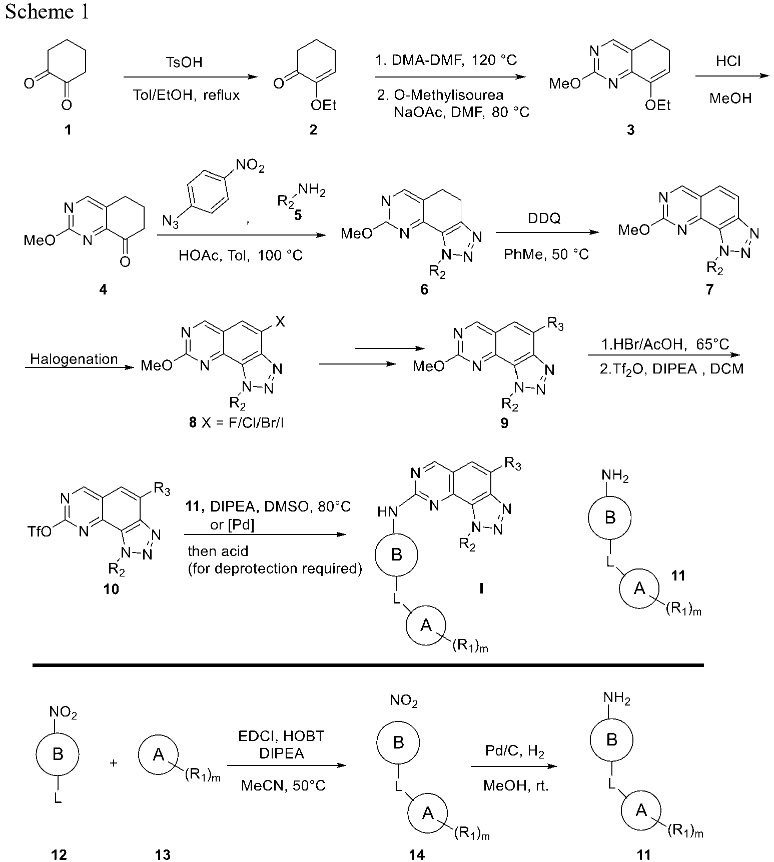

The compound of formula I could be prepared according to the above general scheme. Firstly, 1,2-cyclohexanedione (1) was refluxed in toluene/ethanol under the catalysis of p-toluenesulfonic acid to give an enone intermediate (2). Then, (2) was reacted with N,N-dimethylformamide dimethyl acetal, and the resulting enamine intermediate was further reacted with O-methylisourea to give 8-ethoxy-2-methoxy-5,6-dihydroquinazoline (3). (3) was hydrolyzed under an acidic condition to give 2-methoxy-6,7-dihydroquinazoline-8(5H)-one (4). (4) was reacted with 1-azido-4-nitrobenzene and amine (5) to give 1-substituted 8-methoxy-4,5-dihydro-1H-[1,2,3]triazolo[4,5-H]quinazoline (6). Next, 1-substituted 8-methoxy-1H-[1,2,3]triazolo[4,5-H]quinazoline (7) was obtained by oxidation-aromatization of (6) under the action of 2,3-dichloro-5,6-dicyano-1,4-benzoquinone (DDQ). (7) was subjected to a halogenation reaction to give a halide (**8**), which was then converted into the corresponding deuterated or alkyl or haloalkyl intermediate (**9**) through a functional group transformation. Afterwards, (**9**) was subjected to trifluoromethanesulfonylation to give a trifluoromethanesulfonate (**10**). The compound (**10**) was coupled with amine (**11**), or further deprotected (when the molecule contains a Boc protective group) to give the compound of formula (**I**).

Wherein, amine (**11**) could be prepared according to the above general scheme. Firstly, acid (**12**) and amine (**13**) were subjected to a condensation reaction under the action of EDCI to give a nitro compound (**14**), which was then subjected to a palladium on carbon catalyzed hydrogenation reduction reaction to give an aromatic amine (**11**).

### Example I-1

### (S)-(3-Fluoropyrrolidin-1-yl)(4-((1-isopropyl-1H-[1,2,3]triazolo[4,5-H]quinazolin-8-yl)amino)phenyl)methanone (I-1)

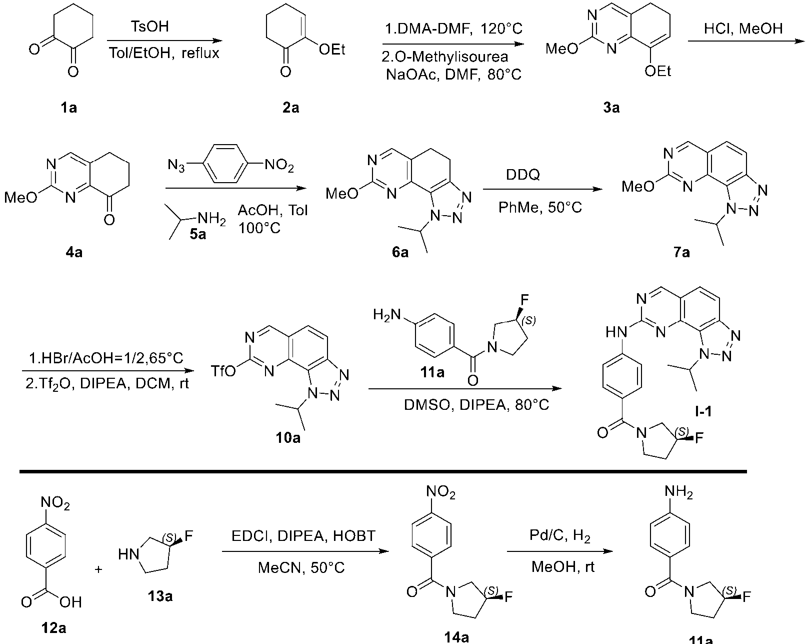

### 1): 2-Ethoxycyclohex-2-en-1-one (2a)

1,2-Cyclohexanedione **1a** (90 g, 803.2 mmol) and p-toluenesulfonic acid (13.8 g, 80.3 mmol) were suspended in toluene/ethanol (2:1, 1000 mL). The mixture was heated to reflux and reacted for another 36 hours. After the reaction solution was cooled to room temperature, the majority of the solvent was removed by distillation under reduced pressure. Then, the reaction solution was neutralized with saturated aqueous solution of sodium bicarbonate (500 mL). The resulting mixture was extracted with dichloromethane (200 mL) three times. The organic layers were combined, dried (over anhydrous sodium sulfate), vacuum filtered, and concentrated. The resulting residue was purified by flash chromatography on a silica gel column (petroleum ether/ethyl acetate = 20:1) to give a light yellow oil, which was the title compound **2a** (91.1 g, 650.6 mmol, 81%). **¹H NMR** (400 MHz, Chloroform-d, *ppm) δ* 5.84 (t, *J* = 4.6 Hz, 1H), 3.74 (q, *J* = 7.0 Hz, 2H), 2.56 - 2.45 (m, 2H), 2.41 (q, *J =* 5.5 Hz, 3H), 1.95 (p, *J =* 6.2 Hz, 2H), 1.36 (t, *J* = 7.0 Hz, 3H).

### 2): 8-Ethoxy-2-methoxy-5,6-dihydroquinazoline (3a)

**2a** (91.1 g, 650.6 mmol) and a solution of N,N-dimethylformamide dimethyl acetal (431 mL, 3253 mol, 5 equiv.) in N,N-dimethylformamide (600 mL) were mixed and stirred at 120 °C for 13 hours. After the reaction solution was cooled to room temperature, the reaction solution was concentrated under reduced pressure to give a brown oil, which was the enamine intermediate. The crude product was used directly in the next step reaction without further purification. **LC-MS** (ESI), C₁₃H₂₀N₃O [M+H]⁺: m/z = 234.2.

The crude product obtained from the previous step, O-methylisourea sulfate (320.5 g, 608.9 mmol), and anhydrous sodium acetate (213.5 g, 2602.1 mmol) were suspended in N,N-dimethylformamide (800 mL). The mixture was heated to 80 °C and reacted for 24 hours. After the reaction solution was cooled to room temperature, the reaction solution was diluted with dichloromethane (500 mL). The resulting mixture was vacuum filtered, and concentrated. The resulting residue was purified by flash chromatography on a silica gel column (petroleum ether/ethyl acetate = 1:1) to give a light yellow solid, which was the title compound **3a** (65.1 g, 315.8 mmol, 53%). **¹H NMR** (400 MHz, Chloroform-d, *ppm*) δ 8.20 (s, 1H), 5.46 (t, *J =* 4.7 Hz, 1H), 3.99 (s, 3H), 3.92 (q, *J* = 7.0 Hz, 2H), 2.70 (t, *J =* 7.9 Hz, 2H), 2.40 (td, *J =* 7.9, 4.8 Hz, 2H), 1.44 (t, *J =* 7.0 Hz, 3H).

### 3): 2-Methoxy-6,7-dihydroquinazolin-8(5H)-one (4a)

Hydrogen chloride (162.6 mL, 4M in dioxane, 650.5 mmol) was slowly added to a solution of **3a** (65.1 g, 315.8 mmol) in methanol (400 mL) in an ice-water bath. The resulting mixture was warmed to room temperature and reacted overnight. Saturated aqueous solution of sodium bicarbonate was added dropwise to the reaction solution in an ice-water bath until the pH reached about 8. The reaction solution was extracted with dichloromethane (200 mL) three times. The organic layers were combined, dried (over anhydrous sodium sulfate), vacuum filtered, and concentrated. The resulting light yellow solid was the title compound **4a** (49.8 g, 280.2 mmol, 93%). **¹H NMR** (400 MHz, Chloroform-d, *ppm)* δ 8.66 (s, 1H), 4.05 (s, 3H), 2.92 (t, *J =* 6.1 Hz, 2H), 2.84 - 2.72 (m, 2H), 2.18 (ddd, *J* = 12.7, 7.2, 5.7 Hz, 2H).

### 4): 1-Isopropyl-8-methoxy-4,5-dihydro-1H-[1,2,3]triazolo[4,5-H]quinazoline (6a)

**4a** (17.9 g, 100.8 mmol) was dissolved in toluene (300 mL). Isopropylamine **5a** (103.0 mL, 302.3 mmol), 1-azido-4-nitrobenzene (33.1 g, 201.6 mmol), and glacial acetic acid (1.72 mL, 30.3 mmol) were added successively at room temperature. The reaction mixture was heated to 100 °C, stirred and reacted overnight. The reaction solution was cooled to room temperature and concentrated under reduced pressure. The resulting residue was purified by flash chromatography on a silica gel column (petroleum ether/ethyl acetate = 3:1) to give a light yellow solid, which was the title compound 6a (13.3 g, 54.1 mmol, 58%). **¹H NMR** (400 MHz, Chloroform-*d*, *ppm) δ* 8.38 (s, 1H), 5.73 (p, *J =* 6.7 Hz, 1H), 4.03 (s, 3H), 3.08 (ddd, *J* = 7.9, 6.6, 1.8 Hz, 2H), 3.05 - 2.92 (m, 2H), 1.70 (d, *J* = 6.7 Hz, 6H).

### 5): 1-Isopropyl-8-methoxy-1H-[1,2,3]triazolo[4,5-H]quinazoline (7a)

2,3-Dichloro-5,6-dicyano-1,4-benzoquinone (36.8 g, 162.4 mmol) was added to a solution of **6a** (13.3 g, 54.1 mmol) in toluene (300 mL). The resulting mixture was heated to 50 °C and reacted for another 24 hours. The resulting mixture was concentrated under reduced pressure, and the resulting residue was purified by flash chromatography on a silica gel column (petroleum ether/ethyl acetate = 20:1 to 6:1) to give a light yellow solid, which was the title compound **7a** (10 g, 41.3 mmol, 76%). **¹H NMR** (400 MHz, Chloroform-*d*, *ppm*) δ 9.32 (s, 1H), 8.00 (d, *J* = 8.8 Hz, 1H), 7.68 (d, *J =* 8.9 Hz, 1H), 6.15 (p, *J =* 6.7 Hz, 1H), 4.21 (s, 3H), 1.87 (d, *J* = 6.8 Hz, 6H).

### 6): 1-Isopropyl-1H-[1,2,3]triazolo[4,5-H]quinazolin-8-yl trifluoromethanesulfonate (10a)

1-Isopropyl-8-methoxy-1H-[1,2,3]triazolo[4,5-H]quinazoline **7a** (10 g, 41 mmol) was dissolved in HBr/AcOH (1:2, 82 mL). The reaction mixture was heated to 65 °C, stirred and reacted for 12 h. The reaction solution was cooled to room temperature and concentrated under reduced pressure. The resulting residue was purified by flash chromatography on a silica gel column (dichloromethane/methanol = 10:1) to give a white solid. **LC-MS** (ESI), C₁₁H₁₂N₅O [M+H]⁺: m/z = 230.2.

The white solid from the previous step was suspended in dichloromethane (205 ml). Diisopropylethylamine (28.5 ml, 164 mmol) was added dropwise. The resulting mixture was stirred to clear. Then, trifluoromethanesulfonic anhydride (13.8 ml, 82 mmol) was added dropwise. The resulting mixture was stirred and reacted for another 1 h. 200 ml of water was added and the resulting mixture was extracted with dichloromethane (200 mL) three times. The organic phases were combined, dried over anhydrous sodium sulfate, vacuum filtered, and concentrated. The resulting residue was purified by flash chromatography on a silica gel column (petroleum ether/ethyl acetate = 4:1) to give a white solid, which was the title compound **10a** (11.84 g, 32.8 mmol, 80%). **¹H NMR** (400 MHz, Chloroform-d, ppm) δ 9.53 (s, 1H), 8.29 (d, *J =* 8.9 Hz, 1H), 7.86 (d, *J =* 8.9 Hz, 1H), 6.05 (p, *J* = 6.7 Hz, 1H), 1.86 (d, *J =* 6.6 Hz, 6H).

### 7): (S)-(3-Fluoropyrrolidin-1-yl)(4-nitrophenyl)methanone (14a)

4-Nitrobenzoic acid **12a** (0.44 g, 2.66 mmol, 1.1 eq), EDCI (0.5 g, 2.66 mmol, 1.1 eq), and HOBT (0.32 g, 0.24 mmol, 0.1 eq) were added to a suspension of (S)-3-fluoro-pyrrolidine **13a** (0.22 g, 2.42 mmol, 1.0 eq) in MeCN (2.5 ml) at room temperature. DIPEA (0.79 ml, 4.84 mmol, 2.0 eq) was added dropwise. After the addition, the resulting mixture was heated to 50 °C and reacted for 4 h. Water (9 ml) was added dropwise at room temperature and the resulting mixture was stirred for 1 h. The mixture was vacuum filtered, and the filter cake was rinsed with water (2 ml). The resulting wet product was vacuum dried under reduced pressure for 2 h to give a yellow solid, which was the title compound **14a** (0.49 g, 2.35 mmol, 95%). **LC-MS** (ESI), C₁₁H₁₁FN₂O₃ [M+H]⁺: m/z = 239.2.

### 8): (S)-(4-Aminophenyl)(3-fluoropyrrolidin-1-yl)methanone (11a)

**14a** (0.49 g, 2.35 mmol, 1.0 eq) was dissolved in MeOH (5 ml) at room temperature. Pd/C (49 mg) was added, and the reaction system was purged with H₂. The resulting mixture was stirred and reacted for 12 h. The filtration funnel was lined with silica gel (0.1 cm) and celite (0.1 cm) from bottom to top. The resulting product was vacuum filtered. The filtrate was concentrated by rotary evaporation under reduced pressure and dried under reduced pressure for 1 h to give a yellow solid, which was the title compound **11a** (0.48 g, 2.29 mmol, 97%). **LC-MS** (ESI), C₁₁H₁₃FN₂O [M+H]⁺: m/z = 209.2.

### 9): (S)-(3-Fluoropyrrolidin-1-yl)(4-((1-isopropyl-1H-[1,2,3]triazolo[4,5-H]quinazolin-8-yl)amino)phenyl)methanone (I-1)

(S)-(4-Aminophenyl)(3-fluoropyrrolidin-1-yl)methanone **11a** (104 mg, 0.5 mmol, 1.2 eq) and **10a** (150 mg, 0.41 mmol, 1.0 eq) were suspended in DMSO (1 ml) at room temperature. The reaction solution was heated to 80 °C, and DIPEA (82 µl, 50.0 mmol, 1.2 eq) was added dropwise to the reaction system. After the addition, the resulting mixture was reacted for 3 h. Then, water (3 ml) was added to the reaction system, and the resulting mixture was stirred for 20 min. The reaction system was cooled to room temperature and then stirred for another 2 h. The resulting mixture was vacuum filtered to give a yellow solid, which was the title compound (I-1) (141 mg, 0.35 mmol, 86%). **¹H NMR** (400 MHz, Chloroform-d, ppm) δ 9.20 (s, 1H), 7.91 (d, *J* = 8.8 Hz, 1H), 7.81 (d, *J* = 8.4 Hz, 2H), 7.69 - 7.54 (m, 4H), 6.22 (p, *J* = 6.7 Hz, 1H), 5.31 (t, *J* = 48.3 Hz, 1H), 4.04 - 3.64 (m, 4H), 2.17 (m, 2H), 1.86 (dd, *J* = 6.8, 3.0 Hz, 6H).

**Table 1 Examples I-2 to I-3**

| **Example No.** | **Side chain structure** | **Example structure** | **Example name** | **LC-MS** (ESI) [M+H]⁺ |
|---|---|---|---|---|
| **I-2** | 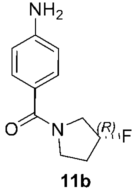 | 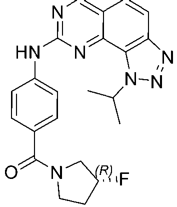 | (R)-(3-Fluoropyrrolidin-1-yl)(4-((1-isopropyl-1H-[1,2,3]triazolo[4,5-H]quinazolin-8-yl)amino)phenyl)methanone | 420.2 |
| **I-3** | 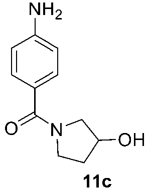 | 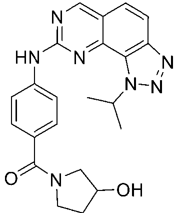 | (3-Hydroxypyrrolidin-1-yl)(4-((1-isopropyl-1H-[1,2,3]triazolo[4,5-H]quinazolin-8-yl)amino)phenyl)methanone | 418.2 |

### Preparation process of example 1-2:

Compound (R)-(4-aminophenyl)(3-fluoropyrrolidin-1-yl)methanone **11b** (1.3 g, 6.3 mmol, 95%) was prepared from **12a** (1 g, 7.3 mmol) and (R)-3-fluoropyrrolidine **13b** (0.59 g, 6.6 mmol) by referring to the synthetic method of **11a. LC-MS** (ESI), C₁₁H₁₄N₂OF [M+H]⁺: m/z =209.2.

Compound **I-2** (167 mg, 279 µmol, 68%) was prepared from **10a** (150 mg, 416 µmol) and **11b** (104 mg, 499 µmol) by referring to the synthetic method of **I-1** in example 1. **¹H NMR** (400 MHz, Chloroform-d, *ppm) δ* 9.20 (s, 1H), 7.91 (d, *J* = 8.8 Hz, 1H), 7.81 (d, *J* = 8.3 Hz, 2H), 7.60 (h, *J* = 12.0, 10.8 Hz, 4H), 6.22 (p, *J* = 6.8 Hz, 1H), 5.37 (d, *J* = 51.2 Hz, 1H), 4.04 - 3.57 (m, 4H), 2.17 (m, 2H),1.86 (dd, *J* = 6.8, 3.0 Hz, 6H).

### Preparation process of example 1-3:

Compound **11c** (1.3 g, 6.1 mmol, 93%) was prepared from **12a** (1 g, 7.3 mmol) and pyrrolidin-3-ol **13c** (0.57 g, 6.6 mmol) by referring to the synthetic method of **11a.** C₁₁H₁₅N₂O₂[M+H]⁺: m/z = 207.1.

Compound **I-3** (123 mg, 295 µmol, 72%) was prepared from **10a** (150 mg, 416 µmol) and (4-aminophenyl)(3-hydroxypyrrolidin-1-yl)methanone **11c** (102 mg, 499 µmol) by referring to the synthetic method of **I-1** in example 1. **¹H NMR** (400 MHz, DMSO-*d*₆, *ppm)* δ 10.38 (s, 1H), 9.46 (s, 1H), 7.90 (dd, *J* = 12.0, 8.6 Hz, 3H), 7.80 (d, *J* = 8.9 Hz, 1H), 7.60 (dd, *J* = 8.5, 4.2 Hz, 2H), 6.18 (p, *J* = 6.8 Hz, 1H), 4.98 (dd, *J =* 30.5, 3.3 Hz, 1H), 4.37 - 4.20 (m, 1H), 3.73 - 3.55 (m, 2H), 3.55 - 3.36 (m, 2H), 2.03 - 1.82 (m, 2H), 1.78 (d, *J= 6.7* Hz, 6H).

### Example I-4

### (S)-(3-Aminopyrrolidin-1-yl)(4-((1-isopropyl-1H-[1,2,3]triazolo[4,5-H]quinazolin-8-yl)amino)phenyl)methanone (I-4)

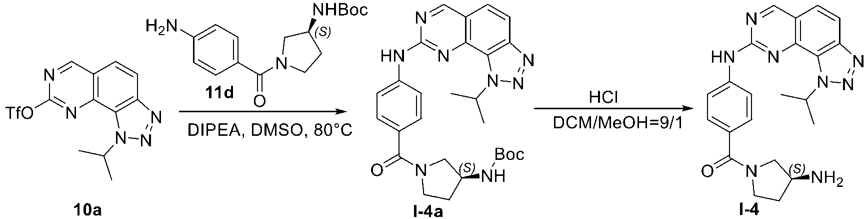

### 1): tert-Butyl (S)-(1-(4-aminobenzoyl)pyrrolidin-3-yl)carbamate (11d)

Compound tert-butyl (S)-(1-(4-aminobenzoyl)pyrrolidin-3-yl)carbamate **11d** (1.9 g, 6.1 mmol, 93%) was prepared from **12a** (1 g, 7.3 mmol) and tert-butyl (S)-pyrrolidin-3-ylcarbamate **13d** (1.3 g, 6.6 mmol) by referring to the synthetic method of **11a.** C₁₆H₂₄N₃O [M+H]⁺: m/z = 306.2.

### 2): tert-Butyl (S)-(1-(4-((1-isopropyl-1H-[1,2,3]triazolo[4,5-H]quinazolin-8-yl)amino)benzoyl)pyrrolidin-3-yl)carbamate (I-4a)

**11d** (152 mg, 0.5 mmol, 1.2 eq) and **10a** (150 mg, 0.41 mmol, 1.0 eq) were suspended in DMSO (1 ml) at room temperature. The reaction solution was heated to 80 °C, and DIPEA (82 µl, 50.0 mmol, 1.2 eq) was added dropwise to the reaction system. After the addition, the resulting mixture was reacted for 3 h. Then, water (3 ml) was added to the reaction system, and the resulting mixture was stirred for 20 min. The reaction system was cooled to room temperature, and then stirred for another 2 h. The resulting mixture was vacuum filtered to give a yellow solid, which was the title compound **I-4a** (180 mg, 0.35 mmol, 86%). **LC-MS** (ESI), C₂₇H₃₃NgO₃ [M+H]⁺: m/z = 517.3.

### 3): (S)-(3-aminopyrrolidin-1-yl)(4-((1-isopropyl-1H-[1,2,3]triazolo[4,5-H]quinazolin-8-yl)amino)phenyl)methanone (I-4)

HCl (4 M in EA, 0.88 ml, 3.5 mmol, 10 eq) was added dropwise to a mixed solution of **I-4a** (180 mg, 0.35 mmol, 1.0 eq) in dichloromethane/methanol (9:1, 3.5 ml) at room temperature. After the addition, the resulting mixture was stirred and reacted for 2 h, and concentrated under reduced pressure. The pH of the aqueous phase was adjusted to greater than 10 with an aqueous solution of NaOH (4 N). Then, the aqueous phase was extracted with DCM (20 ml). The organic phases were combined, washed with saturated brine (20 ml) and H₂O (20 ml) successively, dried over anhydrous sodium sulfate, vacuum filtered, and concentrated to give a yellow solid, which was the title compound **I-4** (140 mg, 0.34 mmol, 96%). **¹H NMR** (400 MHz, DMSO-*d₆, ppm) δ* 10.38 (s, 1H), 9.46 (s, 1H), 8.02 - 7.74 (m, 4H), 7.59 (d, *J* = 8.4 Hz, 2H), 6.18 (q, *J* = 7.2 Hz, 1H), 3.54 (d, *J* = 59.1 Hz, 4H), 3.25 - 3.11 (m, 1H), 2.00 (s, 3H), 1.78 (d, *J* = 6.7 Hz, 6H), 1.64 (s, 1H).

**Table 2 Examples I-5 to I-37**

| **Example No.** | **Side chain structure** | **Example structure** | **Example name** | **LC-MS** (ESI) [M+H]⁺ |
|---|---|---|---|---|
| **I-5** | 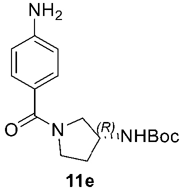 | 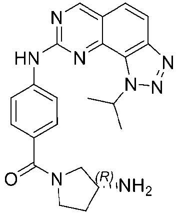 | (R)-(3-Aminopyrrolidin-1-yl)(4-((1-isopropyl-1H-[1,2,3]triazolo[4,5-H]quinazolin-8-yl)amino)phenyl)metha none | 417.2 |
| **I-6** | 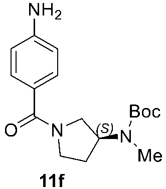 | 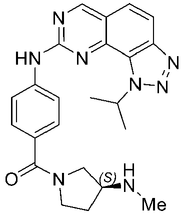 | (S)-(4-((1-Isopropyl-1H-[1,2,3]triazolo[4,5-H]quinazolin-8-yl)amino)phenyl)(3-(methylamino)pyrrolidi n-1-yl)methanone | 431.2 |
| **I-7** | 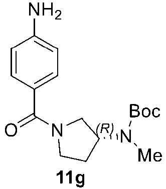 | 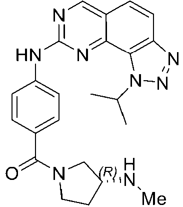 | (R)-(4-((1-Isopropyl-1H-[1,2,3]triazolo[4,5-H]quinazolin-8-yl)amino)phenyl)(3-(methylamino)pyrrolidi n-1-yl)methanone | 431.2 |
| **I-8** | 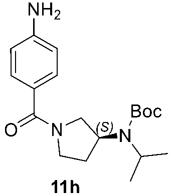 | 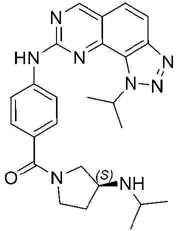 | (S)-(4-((1-Isopropyl-1H-[1,2,3]triazolo[4,5-H]quinazolin-8-yl)amino)phenyl)(3-(isopropylamino)pyrroli din-1-yl)methanone | 459.3 |
| **I-9** | 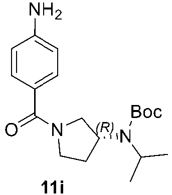 | 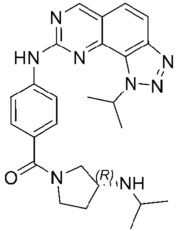 | (R)-(4-((1-Isopropyl-1H-[1,2,3]triazolo[4,5-H]quinazolin-8-yl)amino)phenyl)(3-(isopropylamino)pyrroli din-1-yl)methanone | 459.3 |
| **I-10** | 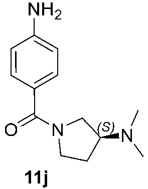 | 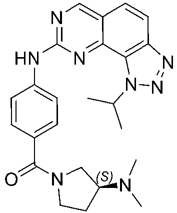 | (S)-(3-(Dimethylamino)pyrroli din-1-yl)(4-((1-isopropyl-1H-[1,2,3]triazolo[4,5-H]quinazolin-8-yl)amino)phenyl)metha none | 445.2 |
| **I-11** | 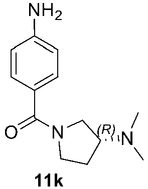 | 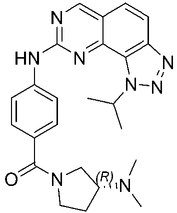 | (R)-(3-(Dimethylamino)pyrroli din-1-yl)(4-((1-isopropyl-1H-[1,2,3]triazolo[4,5-H]quinazolin-8-yl)amino)phenyl)metha none | 445.2 |
| **I-12** | 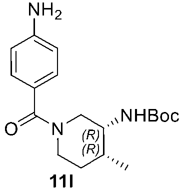 | 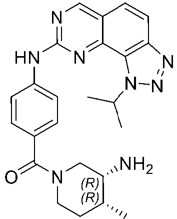 | ((3R,4R)-3-Amino-4-methylpiperidin-1-yl)(4-((1-isopropyl-1H-[1,2,3]triazolo[4,5-H]quinazolin-8-yl)amino)phenyl)metha none | 445.2 |
| **I-13** | 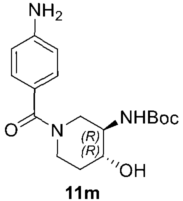 | 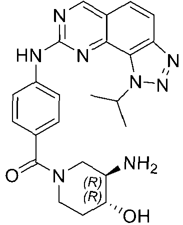 | ((3R,4R)-3-Amino-4-hydroxypiperidin-1-yl)(4-((1-isopropyl-1H-[1,2,3]triazolo[4,5-H]quinazolin-8-yl)amino)phenyl)metha none | 447.2 |
| **I-14** | 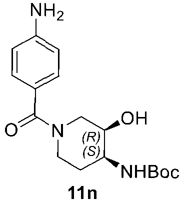 | 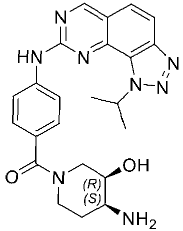 | ((3R,4S)-4-Amino-3-hydroxypiperidin-1-yl)(4-((1-isopropyl-1H-[1,2,3]triazolo[4,5-H]quinazolin-8-yl)amino)phenyl)metha none | 447.2 |
| **I-15** | 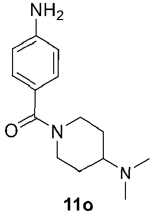 | 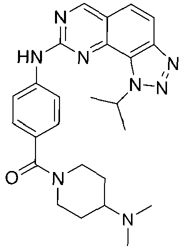 | (4-(Dimethylamino)piperi din-1-yl)(4-((1-isopropyl-1H-[1,2,3]triazolo[4,5-H]quinazolin-8-yl)amino)phenyl)metha none | 459.3 |
| **I-16** | 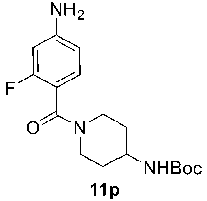 | 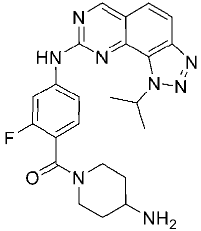 | (4-Aminopiperidin-1-yl)(2-fluoro-4-((1-isopropyl-1H-[1,2,3]triazolo[4,5-H]quinazolin-8-yl)amino)phenyl)metha none | 449.2 |
| **I-17** | 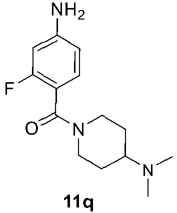 | 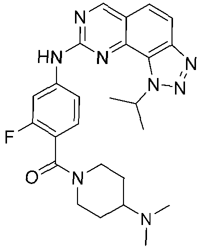 | (4-(Dimethylamino)piperi din-1-yl)(2-fluoro-4-((1-isopropyl-1H-[1,2,3]triazolo[4,5-H]quinazolin-8-yl)amino)phenyl)metha none | 477.2 |
| **I-18** | 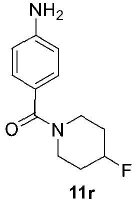 | 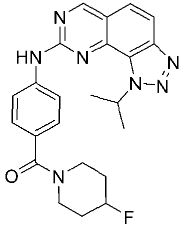 | (4-Fluoropiperidin-1-yl)(4-((1-isopropyl-1H-[1,2,3]triazolo[4,5-H]quinazolin-8-yl)amino)phenyl)metha none | 434.2 |
| **I-19** | 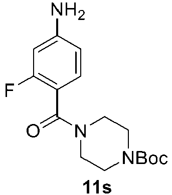 | 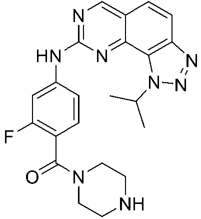 | (2-Fluoro-4-((1-isopropyl-1H-[1,2,3]triazolo[4,5-H]quinazolin-8-yl)amino)phenyl)(piper azin-1-yl)methanone | 435.2 |
| **I-20** | 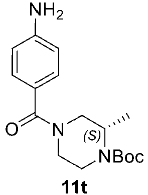 | 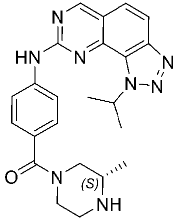 | (S)-(4-((1-Isopropyl-1H-[1,2,3]triazolo[4,5-H]quinazolin-8-yl)amino)phenyl)(3-methylpiperazin-1-yl)methanone | 431.2 |
| **I-21** | 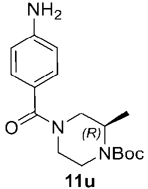 | 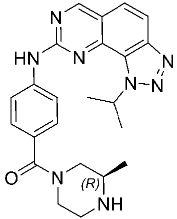 | (R)-(4-((1-Isopropyl-1H-[1,2,3]triazolo[4,5-H]quinazolin-8-yl)amino)phenyl)(3-methylpiperazin-1-yl)methanone | 431.2 |
| **I-22** | 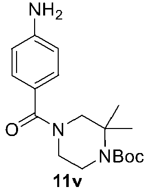 | 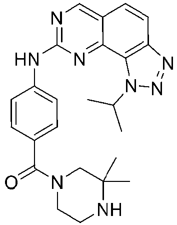 | (3,3-Dimethylpiperazin-1-yl)(4-((1-isopropyl-1H-[1,2,3]triazolo[4,5-H]quinazolin-8-yl)amino)phenyl)metha none | 445.2 |
| **I-23** | 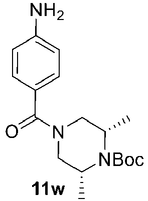 | 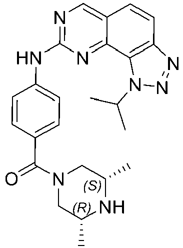 | ((3R,5S)-3,5-dimethylpiperazin-1-yl)(4-((1-isopropyl-1H-[1,2,3]triazolo[4,5-H]quinazolin-8-yl)amino)phenyl)metha none | 445.2 |
| **I-24** | 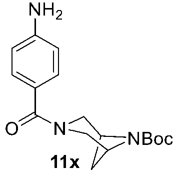 | 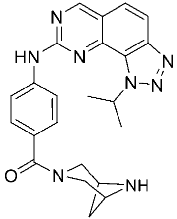 | (3,6-Diazabicyclo[3.1.1]hept an-3-yl)(4-((1-isopropyl-1H-[1,2,3]triazolo[4,5-H]quinazolin-8-yl)amino)phenyl)metha none | 429.2 |
| **I-25** | 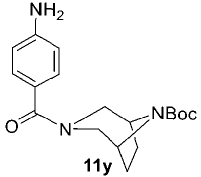 | 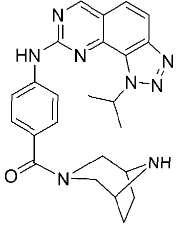 | (3,8-Diazabicyclo[3.2.1]octa n-3-yl)(4-((1-isopropyl-1H-[1,2,3]triazolo[4,5-H]quinazolin-8-yl)amino)phenyl)metha none | 443.2 |
| **I-26** | 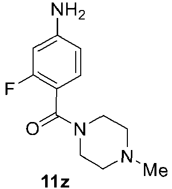 | 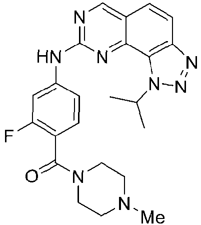 | (2-Fluoro-4-((1-isopropyl-1H-[1,2,3]triazolo[4,5-H]quinazolin-8-yl)amino)phenyl)(4-methylpiperazin-1-yl)methanone | 449.2 |
| **I-27** | 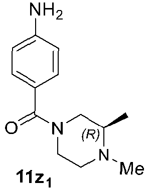 | 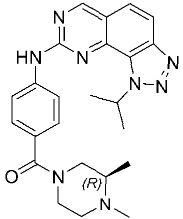 | (R)-(3,4-Dimethylpiperazin-1-yl)(4-((1-isopropyl-1H-[1,2,3]triazolo[4,5-H]quinazolin-8-yl)amino)phenyl)metha none | 445.2 |
| **I-28** | 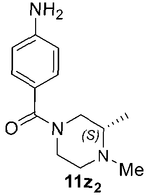 | 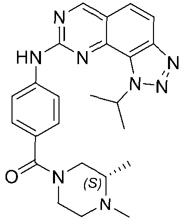 | (S)-(3,4-Dimethylpiperazin-1-yl)(4-((1-isopropyl-1H-[1,2,3]triazolo[4,5-H]quinazolin-8-yl)amino)phenyl)metha none | 445.2 |
| **I-29** | 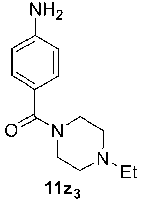 | 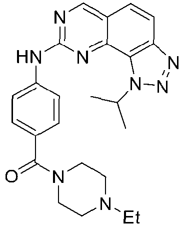 | (4-Ethylpiperazin-1-yl)(4-((1-isopropyl-1H-[1,2,3]triazolo[4,5-H]quinazolin-8-yl)amino)phenyl)metha none | 445.2 |
| **I-30** | 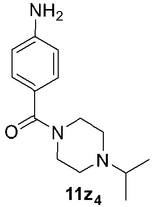 | 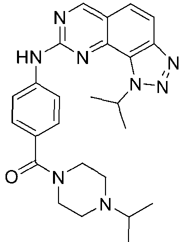 | (4-Isopropylpiperazin-1-yl)(4-((1-isopropyl-1H-[1,2,3]triazolo[4,5-H]quinazolin-8-yl)amino)phenyl)metha none | 459.3 |
| **I-31** | 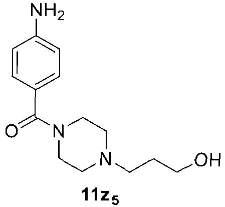 | 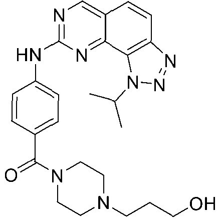 | (4-(3-Hydroxypropyl)piperazi n-1-yl)(4-((1-isopropyl-1H-[1,2,3]triazolo[4,5-H]quinazolin-8-yl)amino)phenyl)metha none | 475.2 |
| **I-32** | 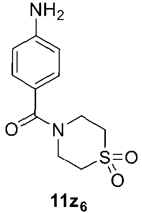 | 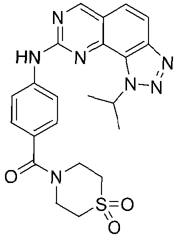 | (1,1-Dioxothiomorpholino)( 4-((1-isopropyl-1H-[1,2,3]triazolo[4,5-H]quinazolin-8-yl)amino)phenyl)metha none | 466.2 |
| **I-33** | 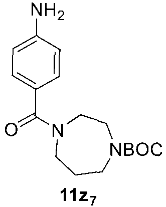 | 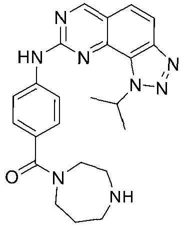 | (1,4-Diazepan-1-yl)(4-((1-isopropyl-1H-[1,2,3]triazolo[4,5-H]quinazolin-8-yl)amino)phenyl)metha none | 431.2 |
| **I-34** | 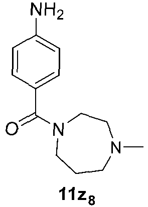 | 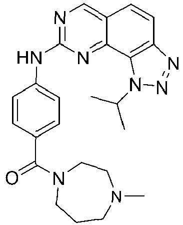 | (4-((1-Isopropyl-1H-[1,2,3]triazolo[4,5-H]quinazolin-8-yl)amino)phenyl)(4-methyl-1,4-diazepan-1-yl)methanone | 445.2 |
| **I-35** | 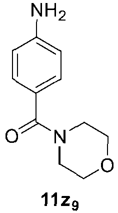 | 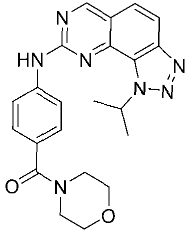 | (4-((1-Isopropyl-1H-[1,2,3]triazolo[4,5-H]quinazolin-8-yl)amino)phenyl)(morp holino)methanone | 418.2 |
| **I-36** | 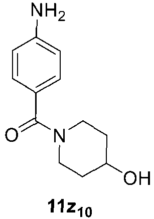 | 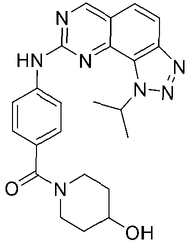 | (4-Hydroxypiperidin-1-yl)(4-((1-isopropyl-1H-[1,2,3]triazolo[4,5-H]quinazolin-8-yl)amino)phenyl)metha none | 432.2 |
| **I-37** | 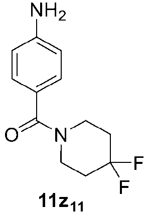 | 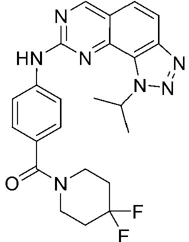 | (4,4-Difluoropiperidin-1-yl)(4-((1-isopropyl-1H-[1,2,3]triazolo[4,5-H]quinazolin-8-yl)amino)phenyl)metha none | 452.2 |

### Preparation process of example 1-5:

Compound tert-butyl (R)-(1-(4-aminobenzoyl)pyrrolidin-3-yl)carbamate **11e** (1.8 g, 6.1 mmol, 92%) was prepared from **12a** (1 g, 7.3 mmol) and tert-butyl (S)-pyrrolidin-3-ylcarbamate **13e** (1.3 g, 6.6 mmol) by referring to the synthetic method of **11a.** C₁₆H₂₄N₃O [M+H]⁺: m/z = 306.2.

Compound **I-5** (106 mg, 254 µmol, two steps 42%) was prepared from **10a** (150 mg, 416 µmol) and **11e** (152 mg, 499 µmol) by referring to the synthetic method of **I-4** in example 1. **¹H NMR** (400 MHz, Chloroform-d, *ppm)* δ 9.19 (s, 1H), 7.90 (d, *J=* 8.8 Hz, 1H), 7.84 - 7.76 (m, 2H), 7.69 - 7.55 (m, 4H), 6.21 (p, *J =* 6.7 Hz, 1H), 3.88 - 3.68 (m, 3H), 3.60 (s, 1H), 3.44 - 3.24 (m, 1H), 2.20 (d, *J=* 11.5 Hz, 1H), 1.85 (d, *J=* 6.7 Hz, 6H), 1.76 (s, 1H).

### Preparation process of example 1-6:

Compound tert-butyl (S)-(1-(4-aminobenzoyl)pyrrolidin-3-yl)(methyl)carbamate **11f** (1.9 g, 6.1 mmol, 93%) was prepared from **12a** (1 g, 7.3 mmol) and tert-buty (S)-methyl(pyrrolidin-3-yl)carbamate **13f** (1.3 g, 6.6 mmol) by referring to the synthetic method of **11a.** C₁₇H₂₆N₃O₂ [M+H]⁺: m/z = 320.2.

Compound **I-6** (116 mg, 270 µmol, two steps 66%) was prepared from **10a** (150 mg, 416 µmol) and **11f** (159 mg, 499 µmol) by referring to the synthetic method of **I-4** in example 1. **¹H NMR** (400 MHz, DMSO-*d*₆, *ppm)* δ 10.38 (s, 1H), 9.45 (s, 1H), 7.90 (dd, *J* = 11.8, 8.7 Hz, 3H), 7.80 (d, *J* = 8.8 Hz, 1H), 7.60 (d, *J* = 8.3 Hz, 2H), 6.18 (p, *J* = 6.8 Hz, 1H), 3.69 - 3.54 (m, 2H), 3.50 (d, *J* = 9.8 Hz, 1H), 3.37 - 3.23 (m, 2H), 3.23 - 3.09 (m, 1H), 2.26 (d, *J* = 42.6 Hz, 3H), 2.03 - 1.93 (m, 1H), 1.78 (d, *J* = 6.7 Hz, 7H).

### Preparation process of example 1-7:

Compound tert-butyl (R)-(1-(4-aminobenzoyl)pyrrolidin-3-yl)(methyl)carbamate **11g** (2.0 g, 6.2 mmol, 94%) was prepared from **12a** (1 g, 7.3 mmol) and tert-butyl (R)-methyl(pyrrolidin-3-yl)carbamate **13g** (1.3 g, 6.6 mmol) by referring to the synthetic method of **11a.** C₁₇H₂₆N₃O₂ [M+H]⁺: m/z = 320.2.

Compound **I-7** (111 mg, 258 µmol, two steps 63%) was prepared from **10a** (150 mg, 416 µmol) and **11g** (159 mg, 499 µmol) by referring to the synthetic method of **I-4** in example 1. **¹H NMR** (400 MHz, Chloroform-d, *ppm)* δ 9.19 (s, 1H), 7.90 (d, *J* = 8.8 Hz, 1H), 7.79 (d, *J* = 8.3 Hz, 2H), 7.68 - 7.56 (m, 4H), 6.35 - 6.07 (m, 1H), 3.93 - 3.65 (m, 3H), 3.61 - 3.48 (m, 1H), 3.41 - 3.25 (m, 1H), 2.45 (d, *J =* 39.9 Hz, 3H), 2.25 - 2.13 (m, 1H), 2.07 *(d, J=* 17.0 Hz, 1H), 1.85 (d, *J* = 6.7 Hz, 6H).

### Preparation process of example 1-8:

Compound tert-butyl (S)-(1-(4-aminobenzoyl)pyrrolidin-3-yl)(isopropyl)carbamate **11h** (2.2 g, 6.3 mmol, 95%) was prepared from **12a** (1 g, 7.3 mmol) and tert-butyl (S)-isopropyl(pyrrolidin-3-yl)carbamate **13h** (1.5 g, 6.6 mmol) by referring to the synthetic method of **11a.** C₁₉H₃₀N₃O₃ [M+H]⁺: m/z = 348.2.

Compound **I-8** (79 mg, 172 µmol, two steps 42%) was prepared from **10a** (150 mg, 416 µmol) and **11h** (152 mg, 499 µmol) by referring to the synthetic method of **I-4** in example 1. **¹H NMR** (400 MHz, Chloroform-d, *ppm)* δ 9.19 (s, 1H), 7.91 (d, *J* = 8.8 Hz, 1H), 7.79 (d, *J* = 8.3 Hz, 2H), 7.67 - 7.53 (m, 4H), 6.22 (p, *J* = 6.6 Hz, 1H), 3.95 (m, 1H), 3.85 - 3.64 (m, 2H), 3.58 - 3.23 (m, 2H), 2.89 (d, *J* = 56.1 Hz, 1H), 2.23 (s, 1H), 2.07 (d, *J* = 25.6 Hz, 1H), 1.86 (d, *J* = 6.7 Hz, 6H), 1.79 (s, 1H), 1.17 - 0.98 (m, 6H).

### Preparation process of example 1-9:

Compound **11i** (2.1 g, 6.1 mmol, 93%) was prepared from **12a** (1 g, 7.3 mmol) and tert-butyl (R)-isopropyl(pyrrolidin-3-yl)carbamate **13i** (1.5 g, 6.6 mmol) by referring to the synthetic method of **11a.** C₁₉H₃₀N₃O₃ [M+H]⁺: m/z = 348.2.

Compound **I-9** (77 mg, 168 µmol, two steps, 41%) was prepared from **10a** (150 mg, 416 µmol) and ester **11i** (152 mg, 499 µmol) by referring to the synthetic method of **I-4** in example 1. **¹H NMR** (400 MHz, Chloroform-d, *ppm)* δ 9.19 (s, 1H), 7.90 (d, *J* = 8.8 Hz, 1H), 7.79 (d, *J* = 8.4 Hz, 2H), 7.67 - 7.54 (m, 4H), 6.21 (p, *J =* 6.8 Hz, 1H), 3.96 (m, 1H),3.85 (m, *J* = 88.8 Hz, 2H), 3.58 (m, 2H), 2.89 (d, *J=* 57.8 Hz, 1H), 2.23 (s, 1H), 2.17 - 1.87 (m, 1H), 1.85 (d, *J=* 6.7 Hz, 6H), 1.80 (d, *J=* 10.6 Hz, 1H), 1.20 - 0.90 (m, 6H).

### Preparation process of example 1-10:

Compound (S)-(4-aminophenyl)(3-(dimethylamino)pyrrolidin-1-yl)methanone **11j** (1.4 g, 6 mmol, 91%) was prepared from **12a** (1 g, 7.3 mmol) and (S)-N,N-dimethylpyrrolidin-3-amine **13j** (0.75 g, 6.6 mmol) by referring to the synthetic method of **11a.** C₁₃H₂₀N₃O [M+H]⁺: m/z = 234.2.

Compound **I-10** (60 mg, 140 µmol, 40 %) was prepared from **10a** (150 mg, 416 µmol) and **11j** (116 mg, 499 µmol) by referring to the synthetic method of **I-1** in example 1. **¹H NMR** (400 MHz, Chloroform-d, *ppm)* δ 9.19 (s, 1H), 7.91 (d, *J* = 8.8 Hz, 1H), 7.80 (t, *J* = 6.0 Hz, 2H), 7.61 (dt, *J* = 12.5, 6.4 Hz, 4H), 6.22 (p, *J* = 6.8 Hz, 1H), 3.91 (dt, *J* = 44.8, 10.5 Hz, 1H), 3.80 - 3.54 (m, 2H), 3.45 (dt, *J* = 20.0, 9.9 Hz, 1H), 2.87 - 2.64 (m, 1H), 2.33 (s, 3H), 2.24 (s, 4H), 1.86 (d, *J =* 6.8 Hz, 7H).

### Preparation process of example I-11:

Compound (R)-(4-aminophenyl)(3-(dimethylamino)pyrrolidin-1-yl)methanone **11k** (1.4 g, 6 mmol, 91%) was prepared from **12a** (1 g, 7.3 mmol) and (S)-N,N-dimethylpyrrolidin-3-amine **13k** (0.75 g, 6.6 mmol) by referring to the synthetic method of **11a.** C₁₃H₂₀N₃O [M+H]⁺: m/z = 234.2.

Compound **I-11** (59 mg, 133 µmol, 32%) was prepared from **10a** (150 mg, 416 µmol) and **11k** (116 mg, 499 µmol) by referring to the synthetic method of **I-1** in example 1. **¹H NMR** (400 MHz, Chloroform-d, *ppm)* δ 9.19 (s, 1H), 7.91 (d, *J =* 8.8 Hz, 1H), 7.80 (d, *J=* 8.2 Hz, 2H), 7.59 (q, *J* = 9.0 Hz, 4H), 6.27 - 6.16 (m, 1H), 4.04 - 3.34 (m, 4H), 2.75 (d, *J =* 40.7 Hz, 1H), 2.29 (d, *J* = 35.3 Hz, 6H), 2.18 - 1.88 (m, 2H).

### Preparation process of example 1-12:

Compound tert-butyl (3R,4R)-1-(4-aminobenzoyl)-4-methylpiperidin-3-yl)carbamate **11l** (2.1 g, 6.3 mmol, 95%) was prepared from **12a** (1 g, 7.3 mmol) and tert-butyl (3R,4R)-4-methylpiperidin-3-yl)carbamate **131** (1.4 g, 6.6 mmol) by referring to the synthetic method of **11a.** C₁₈H₂₈N₃O₃ [M+H]⁺: m/z = 334.2.

Compound **I-12** (136 mg, 308 µmol, two steps 75%) was prepared from **10a** (150 mg, 416 µmol) and **111** (166 mg, 499 µmol) by referring to the synthetic method of **I-4** in example 1. **¹H NMR** (400 MHz, Chloroform-d, *ppm)* δ 9.18 (s, 1H), 7.89 (d, *J* = 8.8 Hz, 1H), 7.78 (d, *J* = 8.5 Hz, 2H), 7.68 - 7.44 (m, 4H), 6.21 (p, *J=* 6.7 Hz, 1H), 3.31 - 3.18 (m, 1H), 3.06 (s, 2H), 1.85 (d, *J =* 6.7 Hz, 8H), 1.01 (m, 3H) ,1.01 (d, *J* = 6.8 Hz, 3H).

### Preparation process of example 1-13:

Compound tert-butyl (3R,4R)-1-(4-aminobenzoyl)-4-hydroxypiperidin-3-yl)carbamate **11m** (2.1 g, 6.1 mmol, 93%) was prepared from **12a** (1 g, 7.3 mmol) and tert-butyl (3R,4R)-4-hydroxypiperidin-3-yl)carbamate **13m** (1.4 g, 6.6 mmol) by referring to the synthetic method of **11a.** C₁₇H₂₆N₃O₄ [M+H]⁺: m/z = 336.2.

Compound **I-13** (102 mg, 300 µmol, two steps 56%) was prepared from **10a** (150 mg, 416 µmol) and **11m** (167 mg, 499 µmol) by referring to the synthetic method of **I-4** in example 1. **¹H NMR** (400 MHz, DMSO-*d*₆, *ppm) δ* 10.37 (s, 1H), 9.46 (s, 1H), 7.90 (dd, *J* = 10.7, 8.6 Hz, 3H), 7.80 (d, *J =* 8.8 Hz, 1H), 7.46 (d, *J* = 8.3 Hz, 2H), 6.18 (p, *J* = 6.7 Hz, 1H), 4.89 (d, *J* = 4.5 Hz, 1H), 3.25 (s, 3H), 1.77 (dd, *J* = 6.8, 2.8 Hz, 9H), 1.35 (q, *J* = 10.5, 9.8 Hz, 1H).

### Preparation process of example 1-14:

Compound **11n** (2.1 g, 6.1 mmol, 93%) was prepared from **12a** (1 g, 7.3 mmol) and tert-butyl (3R,4S)-4-hydroxypiperidin-3-yl)carbamate **13n** (1.4 g, 6.6 mmol) by referring to the synthetic method of **11a.** C₁₇H₂₆N₃O₄ [M+H]⁺: m/z = 336.2.

Compound **I-14** (95 mg, 213 µmol, two steps 52%) was prepared from **10a** (150 mg, 416 µmol) and tert-butyl (3R,4S)-1-(4-aminobenzoyl)-4-hydroxypiperidin-3-yl)carbamate **11n** (167 mg, 499 µmol) by referring to the synthetic method of **I-4** in example 1. **¹H NMR** (400 MHz, DMSO-*d*₆, *ppm)* δ 10.35 (s, 1H), 9.45 (s, 1H), 7.89 (dd, *J* = 8.6, 5.0 Hz, 3H), 7.80 (d, *J* = 8.8 Hz, 1H), 7.48 (s, 2H), 6.28 - 6.08 (m, 1H), 4.76 (s, 1H), 3.59 (d, *J* = 57.6 Hz, 1H), 3.25 (m, 2H), 3.12 (s, 1H), 2.88 (s, 1H), 1.77 (d, *J* = 6.6 Hz, 6H), 1.64 (d, *J* = 24.7 Hz, 2H).

### Preparation process of example 1-15:

Compound (4-aminophenyl)(4-(dimethylamino)piperidin-1-yl)methanone **11o** (1.5 g, 5.9 mmol, 89%) was prepared from **12a** (1 g, 7.3 mmol) and N,N-dimethylpiperidin-4-amine **13o** (0.85 g, 6.6 mmol) by referring to the synthetic method of **11a.** C₁₄H₂₂N₃O [M+H]⁺: m/z = 248.2.

Compound **I-15** (113 mg, 246 µmol, 60%) was prepared from **10a** (150 mg, 416 µmol) and **11o** (159 mg, 499 µmol) by referring to the synthetic method of **I-4a** in example 1. **¹H NMR** (600 MHz, DMSO-*d*₆, *ppm) δ* 10.39 (d, *J* = 3.4 Hz, 1H), 9.46 (d, *J* = 3.9 Hz, 1H), 7.93 (dd, *J* = 8.6, 3.6 Hz, 2H), 7.89 (dd, *J* = 8.8, 4.1 Hz, 1H), 7.81 (dd, *J* = 9.0, 3.7 Hz, 1H), 7.48 (dd, *J* = 8.6, 3.5 Hz, 2H), 6.18 (dq, *J=* 12.7, 6.7 Hz, 1H), 2.62 (s, 5H), 1.99 (s, 2H), 1.77 (dd, *J* = 7.2, 3.8 Hz, 6H), 1.57 (s, 2H).

### Preparation process of example 1-16:

Compound tert-butyl (1-(4-amino-2-fluorobenzoyl)piperidin-4-yl)carbamate **11p** (2.1 g, 6.1 mmol, 93%) was prepared from **12a** (1 g, 7.3 mmol) and tert-butyl piperidin-4-ylcarbamate **13p** (1.3 g, 6.6 mmol) by referring to the synthetic method of **11a.** C₁₇H₂₅FN₃O₃ [M+H]⁺: m/z = 338.2.

Compound **I-16** (132 mg, 295 µmol, two steps 72%) was prepared from **10a** (150 mg, 416 µmol) and **11p** (167 mg, 499 µmol) by referring to the synthetic method of **I-4** in example 1. **¹H NMR** (400 MHz, Chloroform-d, *ppm)* δ 9.21 (d, *J =* 1.2 Hz, 1H), 8.05 (dd, *J =* 12.3, 2.0 Hz, 1H), 7.94 (dd, *J=* 8.9, 1.2 Hz, 1H), 7.68 (s, 1H), 7.61 (dd, *J* = 8.8, 1.2 Hz, 1H), 7.41 (t, *J =* 7.9 Hz, 1H), 7.22 (dd, *J* = 8.3, 2.0 Hz, 1H), 6.22 (p, *J* = 6.7 Hz, 1H), 4.66 (d, *J* = 13.5 Hz, 1H), 3.69 (d, *J=* 13.7 Hz, 1H), 3.14 (s, 1H), 3.06 - 2.91 (m, 2H), 1.97 (d, *J= 13.2* Hz, 1H), 1.88 (d, *J* = 6.8 Hz, 7H), 1.47 - 1.28 (m, 2H).

### Preparation process of example 1-17:

Compound **11q** (1.5 g, 5.8 mmol, 88%) was prepared from **12a** (1 g, 7.3 mmol) and N,N-dimethylpiperidin-4-amine **13q** (0.84 g, 6.6 mmol) by referring to the synthetic method of **11a.** C₁₄H₂₁N₃OF [M+H]⁺: m/z = 266.2.

Compound **I-17** (93 mg, 209 µmol, 51%) was prepared from **10a** (150 mg, 416 µmol) and (4-amino-2-fluorophenyl)(4-(dimethylamino)piperidin-1-yl)methanone **11q** (132 mg, 499 µmol) by referring to the synthetic method of **I-4a** in example 1. **¹H NMR** (400 MHz, DMSO-*d*₆, *ppm)* δ 10.58 (s, 1H), 9.50 (s, 1H), 8.03 (dd, *J =* 12.8, 2.0 Hz, 1H), 7.94 (d, *J=* 8.8 Hz, 1H), 7.83 (d, *J* = 8.8 Hz, 1H), 7.61 (dd, *J* = 8.4, 2.0 Hz, 1H), 7.44 (t, *J* = 8.2 Hz, 1H), 6.19 (p, *J* = 6.7 Hz, 1H), 4.57 (d, *J* = 13.1 Hz, 1H), 3.59 (d, *J* = 13.6 Hz, 1H), 3.17 - 3.07 (m, 1H), 2.82 (t, *J=* 12.5 Hz, 2H), 2.49 (s, 6H), 1.99 (s, 2H), 1.78 (d, *J =* 6.7 Hz, 6H), 1.45 (s, 2H).

### Preparation process of example 1-18:

Compound (4-aminophenyl)(4-fluoropiperidin-1-yl)methanone **11r** (1.4 g, 6.3 mmol, 95%) was prepared from **12a** (1 g, 7.3 mmol) and 4-fluoropiperidine **13r** (0.68 g, 6.6 mmol) by referring to the synthetic method of **11a.** C₁₂H₁₆N₂OF [M+H]⁺: m/z = 223.1.

Compound **I-18** (114 mg, 258 µmol, 63%) was prepared from **10a** (150 mg, 416 µmol) and **11r** (110 mg, 499 µmol) by referring to the synthetic method of **I-4a** in example 1. **¹H NMR** (400 MHz, Chloroform-d, *ppm)* δ 9.19 (s, 1H), 7.91 (d, *J* = 8.8 Hz, 1H), 7.85 - 7.76 (m, 2H), 7.59 (d, *J=* 8.8 Hz, 1H), 7.55 (s, 1H), 7.52 - 7.46 (m, 2H), 6.21 (p, *J =* 6.7 Hz, 1H), 5.03 - 4.82 (m, 1H), 3.68 (s, 4H), 1.93 (s, 4H), 1.85 (d, J = 6.7 Hz, 6H).

### Preparation process of example 1-19:

Compound tert-butyl 4-(4-amino-2-fluorobenzoyl)piperazine-1-carboxylate **11s** (2 g, 6.1 mmol, 92%) was prepared from **12a** (1 g, 7.3 mmol) and tert-butyl piperazine-1-carboxylate **13s** (1.2 g, 6.6 mmol) by referring to the synthetic method of **11a.** C₁₆H₂₃N₃O₃ [M+H]⁺: m/z = 324.2.

Compound **I-19** (112 mg, 258 µmol, two steps 63%) was prepared from **10a** (150 mg, 416 µmol) and **11s** (161 mg, 499 µmol) by referring to the synthetic method of **I-4** in example 1. **¹H NMR** (400 MHz, Chloroform-d, *ppm)* δ 9.21 (s, 1H), 8.05 (dd, J = 12.3, 2.1 Hz, 1H), 7.94 (d, *J =* 8.8 Hz, 1H), 7.77 (s, 1H), 7.61 (d, *J =* 8.9 Hz, 1H), 7.43 (t, *J =* 7.9 Hz, 1H), 7.26 - 7.22 (m, 1H), 6.21 (p, *J* = 6.7 Hz, 1H), 3.83 (t, *J=* 5.1 Hz, 2H), 3.43 (s, 2H), 3.06 - 2.85 (m, 4H), 1.88 (d, *J* = 6.7 Hz, 6H).

### Preparation process of example 1-20:

Compound tert-butyl (S)-4-(4-aminobenzoyl)-2-methylpiperazine-1-carboxylate **11t** (1.9 g, 6 mmol, 91%) was prepared from **12a** (1 g, 7.3 mmol) and tert-butyl (S)-2-methylpiperazine-1-carboxylate **13t** (1.3 g, 6.6 mmol) by referring to the synthetic method of **11a.** C₁₇H₂₆N₃O₃ [M+H]⁺: m/z = 320.2.

Compound **I-20** (114 mg, 267 µmol, two steps 65%) was prepared from **10a** (150 mg, 416 µmol) and **11t** (159 mg, 499 µmol) by referring to the synthetic method of **I-4** in example 1. **¹H NMR** (400 MHz, Chloroform-d, *ppm)* δ 9.19 (s, 1H), 7.91 (d, *J* = 8.9 Hz, 1H), 7.80 (d, *J* = 8.3 Hz, 2H), 7.63 - 7.54 (m, 2H), 7.49 (d, *J* = 8.5 Hz, 2H), 6.31 - 6.07 (m, 1H), 4.50 (d, *J* = 75.7 Hz, 1H), 3.79 (s, 1H), 2.95 (d, *J* = 75.3 Hz, 4H), 1.85 (d, *J =* 6.7 Hz, 7H), 1.14 (d, *J=* 38.2 Hz, 3H).

### Preparation process of example 1-21:

Compound tert-butyl (R)-4-(4-aminobenzoyl)-2-methylpiperazine-1-carboxylate **11u** (1.9 g, 6 mmol, 91%) was prepared from **12a** (1 g, 7.3 mmol) and tert-butyl (R)-2-methylpiperazine-1-carboxylate **13u** (1.3 g, 6.6 mmol) by referring to the synthetic method of **11a.** C₁₇H₂₆N₃O₃ [M+H]⁺: m/z = 320.2.

Compound **I-21** (107 mg, 250 µmol, two steps 61%) was prepared from **10a** (150 mg, 416 µmol) and **11u** (159 mg, 499 µmol) by referring to the synthetic method of **I-4** in example 1. **¹H NMR** (400 MHz, Chloroform-d, *ppm)* δ 9.19 (s, 1H), 7.91 (d, *J* = 8.8 Hz, 1H), 7.80 (d, *J* = 8.3 Hz, 2H), 7.68 - 7.56 (m, 2H), 7.49 (d, *J =* 8.3 Hz, 2H), 6.21 (p, *J* = 6.7 Hz, 1H), 4.59 (s, 1H), 3.87 (d, *J* = 63.1 Hz, 1H), 2.95 (d, *J=* 76.0 Hz, 5H), 1.85 *(d, J=* 6.7 Hz, 6H), 1.09 (s, 3H).

### Preparation process of example 1-22:

Compound tert-butyl 4-(4-aminobenzoyl)-2,2-dimethylpiperazine-1-carboxylate **11v** (2.1 g, 6.2 mmol, 94%) was prepared from **12a** (1 g, 7.3 mmol) and tert-butyl 2,2-dimethylpiperazine-1-carboxylate **13v** (1.4 g, 6.6 mmol) by referring to the synthetic method of **11a.** C₁₈H₂₈N₃O₃ [M+H]⁺: m/z = 334.2.

Compound **I-22** (136 mg, 371 µmol, two steps 75%) was prepared from **10a** (150 mg, 416 µmol) and **11v** (166 mg, 499 µmol) by referring to the synthetic method of **I-4** in example 1. **¹H NMR** (400 MHz, Chloroform-d, *ppm)* δ 9.19 (s, 1H), 7.91 (d, *J* = 8.8 Hz, 1H), 7.86 - 7.73 (m, 2H), 7.67 - 7.53 (m, 2H), 7.48 (d, *J =* 8.2 Hz, 2H), 6.21 (p, *J* = 6.7 Hz, 1H), 3.49 (s, 4H), 2.99 (s, 2H), 1.85 (d, *J =* 6.7 Hz, 6H), 1.17 (d, *J* = 13.5 Hz, 6H).

### Preparation process of example 1-23:

Compound tert-butyl (2R,6S)-4-(4-aminobenzoyl)-2,6-dimethylpiperazine-1-carboxylate **11w** (2.1 g, 6.2 mmol, 94%) was prepared from **12a** (1 g, 7.3 mmol) and tert-butyl (2R,6S)-2,6-dimethylpiperazine-1-carboxylate **13w** (1.4 g, 6.6 mmol) by referring to the synthetic method of **11a.** C₁₈H₂₈N₃O₃ [M+H]⁺: m/z = 334.2.

Compound **I-23** (96 mg, 217 µmol, two steps 53%) was prepared from **10a** (150 mg, 416 µmol) and **11w** (166 mg, 499 µmol) by referring to the synthetic method of **I-4** in example 1. **¹H NMR** (400 MHz, Chloroform-d, *ppm)* δ 9.19 (s, 1H), 7.91 (d, *J* = 8.8 Hz, 1H), 7.85 - 7.74 (m, 2H), 7.59 (d, *J* = 8.6 Hz, 2H), 7.53 - 7.46 (m, 2H), 6.22 (p, *J* = 6.7 Hz, 1H), 4.63 (s, 1H), 3.74 (s, 1H), 2.67 (d, *J=* 180.8 Hz, 4H), 1.85 (d, *J* = 6.7 Hz, 6H), 1.22 - 0.89 (m, 6H).

### Preparation process of example 1-24:

Compound tert-butyl 3-(4-aminobenzoyl)-3,6-diazabicyclo[3.1.1]heptane-6-carboxylate **11x** (1.58 g, 5.8 mmol, 88%) was prepared from **12a** (1 g, 7.3 mmol) and tert-butyl 3,6-diazabicyclo[3.1.1]heptane-6-carboxylate **13x** (1.3 g, 6.6 mmol) by referring to the synthetic method of **11a.** C₁₇H₂₄N₃O₃ [M+H]⁺: m/z = 318.2.

Compound **I-24** (79 mg, 185 µmol, two steps 45%) was prepared from **10a** (150 mg, 416 µmol) and **11x** (158 mg, 499 µmol) by referring to the synthetic method of **I-4** in example 1. **¹H NMR** (400 MHz, Chloroform-d, *ppm)* δ 9.19 (s, 1H), 7.91 (d, *J* = 8.8 Hz, 1H), 7.87 - 7.76 (m, 2H), 7.64 - 7.49 (m, 4H), 6.22 (p, *J =* 6.7 Hz, 1H), 4.14 (d, *J* = 13.6 Hz, 1H), 3.95 - 3.65 (m, 5H), 2.81 - 2.70 (m, 1H), 1.86 (d, *J* = 6.7 Hz, 6H), 1.60 *(d, J=* 9.1 Hz, 2H).

### Preparation process of example 1-25:

Compound **11y** (1.9 g, 5.9 mmol, 89%) was prepared from **12a** (1 g, 7.3 mmol) and tert-butyl 3,8-diazabicyclo[3.2.1]octane-8-carboxylate **13y** (1.4 g, 6.6 mmol) by referring to the synthetic method of **11a.** C₁₈H₂₆N₃O₃ [M+H]⁺: *m*/*z* = 332.2.

Compound **I-25** (79 mg, 176 µmol, two steps 43%) was prepared from **10a** (150 mg, 416 µmol) and tert-butyl 3-(4-aminobenzoyl)-3,8-diazabicyclo[3.2.1]octane-8-carboxylate **11y** (165 mg, 499 µmol) by referring to the synthetic method of **I-4** in example 1. **¹H NMR** (400 MHz, Chloroform-d, *ppm)* δ 9.19 (s, 1H), 7.90 (d, *J* = 8.8 Hz, 1H), 7.84 - 7.75 (m, 2H), 7.59 (d, *J* = 8.9 Hz, 2H), 7.49 - 7.40 (m, 2H), 6.21 (p, *J* = 6.7 Hz, 1H), 4.52 (s, 1H), 3.52 (d, *J=* 64.1 Hz, 4H), 3.06 (s, 1H), 1.85 (d, *J* = 6.7 Hz, 10H).

### Preparation process of example 1-26:

Compound **11z** (1.4 g, 6 mmol, 91%) was prepared from **12a** (1 g, 7.3 mmol) and 1-methylpiperazine **13z** (0.66 g, 6.6 mmol) by referring to the synthetic method of **11a.** C₁₂H₁₇N₃OF [M+H]⁺: m/z = 238.1.

Compound **I-26** (66 mg, 148 µmol, 36%) was prepared from **10a** (150 mg, 416 µmol) and (4-amino-2-fluorophenyl)(4-methylpiperazin-1-yl)methanone **11z** (118 mg, 499 µmol) by referring to the synthetic method of **I-1** in example 1. **¹H NMR** (600 MHz, Chloroform-d, *ppm)* δ 9.22 (s, 1H), 8.06 (dd, *J* = 12.2, 2.1 Hz, 1H), 7.95 (d, *J =* 8.8 Hz, 1H), 7.69 (s, 1H), 7.62 (d, *J* = 8.8 Hz, 1H), 7.44 (t, *J =* 8.0 Hz, 1H), 7.25 - 7.23 (m, 1H), 6.26 - 6.16 (m, 1H), 3.95 (s, 2H), 3.57 (s, 2H), 2.63 (d, *J =* 61.0 Hz, 4H), 2.46 (s, 3H), 1.87 (dd, *J=* 14.3, 6.7 Hz, 6H).

### Preparation process of example 1-27:

Compound (R)-(4-aminophenyl)(3,4-dimethylpiperazin-1-yl)methanone **11z₁** (1.3 g, 5.7 mmol, 87%) was prepared from **12a** (1 g, 7.3 mmol) and (R)-1,2-dimethylpiperazine **13z₁** (0.75 g, 6.6 mmol) by referring to the synthetic method of **11a.** C₁₃H₂₀N₃O [M+H]⁺: m/z =234.2.

Compound **I-27** (62 mg, 139 µmol, 34%) was prepared from 10a (150 mg, 416 µmol) and **11z₁** (116 mg, 499 µmol) by referring to the synthetic method of **I-1** in example 1. **¹H NMR** (400 MHz, DMSO-*d*₆, *ppm)* δ 10.38 (s, 1H), 9.46 (s, 1H), 7.97 - 7.72 (m, 4H), 7.46 (d, *J* = 8.3 Hz, 2H), 6.18 (p, *J* = 6.7 Hz, 1H), 4.44 - 4.00 (m, 1H), 2.96 - 2.51 (m, 4H), 2.20 (s, 3H), 2.14 - 1.97 (m, 2H), 1.77 (d, *J* = 6.7 Hz, 6H), 0.92 (d, *J* = 51.9 Hz, 3H).

### Preparation process of example 1-28:

Compound (S)-(4-aminophenyl)(3,4-dimethylpiperazin-1-yl)methanone **11z₂** (1.4 g, 5.9 mmol, 90%) was prepared from **12a** (1 g, 7.3 mmol) and (S)-1,2-dimethylpiperazine **13z₂** (0.75 g, 6.6 mmol) by referring to the synthetic method of **11a.** C₁₃H₂₀N₃O [M+H]⁺: m/z =234.2.

Compound **I-28** (76 mg, 172 µmol, 42%) was prepared from **10a** (150 mg, 416 µmol) and **11z₂** (116 mg, 499 µmol) by referring to the synthetic method of **I-1** in example 1. **¹H NMR** (400 MHz, DMSO-*d*₆, *ppm)* δ 10.38 (s, 1H), 9.46 (s, 1H), 7.91 (dd, J=13.0, 8.6 Hz, 3H), 7.80 (d, *J* = 8.8 Hz, 1H), 7.46 (d, *J* = 8.5 Hz, 2H), 6.18 (p, *J* = 6.7 Hz, 1H), 3.91 (d, *J* = 246.5 Hz, 1H), 3.31 (s, 2H), 2.71 (d, *J* = 28.9 Hz, 2H), 2.21 (s, 3H), 2.07 (d, *J =* 24.6 Hz, 2H), 1.77 (d, *J =* 6.6 Hz, 6H), 0.98 (s, 3H).

### Preparation process of example 1-29:

Compound (4-aminophenyl)(4-ethylpiperazin-1-yl)methanone **11z₃** (1.4 g, 5.9 mmol, 90%) was prepared from **12a** (1 g, 7.3 mmol) and 1-ethylpiperazine **13z₃** (0.75 g, 6.6 mmol) by referring to the synthetic method of **11a.** C₁₃H₂₀N₃O [M+H]⁺: m/z =234.2.

Compound **I-29** (98 mg, 221 µmol, 54%) was prepared from **10a** (150 mg, 416 µmol) and **11z₃** (116 mg, 499 µmol) by referring to the synthetic method of **I-1** in example 1. **¹H NMR** (400 MHz, Chloroform-d, *ppm)* δ 9.19 (s, 1H), 7.91 (d, *J =* 8.8 Hz, 1H), 7.84 - 7.77 (m, 2H), 7.62 - 7.53 (m, 2H), 7.53 - 7.46 (m, 2H), 6.21 (p, *J =* 6.7 Hz, 1H), 3.70 (d, *J=* 64.2 Hz, 4H), 2.48 (q, *J=* 7.2 Hz, 6H), 1.85 (d, *J=* 6.7 Hz, 6H), 1.12 (t, *J =* 7.2 Hz, 3H).

### Preparation process of example 1-30:

Compound (4-aminophenyl)(4-isopropylpiperazin-1-yl)methanone **11z₄** (1.5 g, 6.1 mmol, 92%) was prepared from **12a** (1 g, 7.3 mmol) and 1-isopropylpiperazine **13z₄** (0.85 g, 6.6 mmol) by referring to the synthetic method of **11a.** C₁₄H₂₂N₃O [M+H]⁺: m/z = 248.2.

Compound **I-30** (118 mg, 258 µmol, 63%) was prepared from **10a** (150 mg, 416 µmol) and **11z₄** (123 mg, 499 µmol) by referring to the synthetic method of **I-1** in example 1. **¹H NMR** (400 MHz, Chloroform-*d, ppm)* δ 9.19 (s, 1H), 7.91 (d, *J =* 8.8 Hz, 1H), 7.82 - 7.75 (m, 2H), 7.63 - 7.55 (m, 2H), 7.53 - 7.46 (m, 2H), 6.21 (p, *J= 6.7* Hz, 1H), 3.71 (d, *J* = 67.2 Hz, 4H), 2.78 (s, 1H), 2.58 (s, 4H), 1.85 (d, *J* = 6.7 Hz, 6H), 1.09 (d, *J =* 6.5 Hz, 6H).

### Preparation process of example 1-31:

Compound (4-aminophenyl)(4-(3-hydroxypropyl)piperazin-1-yl)methanone **11z₅** (1.5 g, 6.1 mmol, 92%) was prepared from **12a** (1 g, 7.3 mmol) and 3-(piperazin-1-yl)propan-1-ol **13z₅** (0.85 g, 6.6 mmol) by referring to the synthetic method of **11a.** C₁₄H₂₂N₃O [M+H]⁺: m/z = 264.2.

Compound **I-31** (114 mg, 241 µmol, 58%) was prepared from **10a** (150 mg, 416 µmol) and **11z₅** (131 mg, 499 µmol) by referring to the synthetic method of **I-1** in example 1. **¹H NMR** (400 MHz, Chloroform-*d, ppm*) δ 9.12 (s, 1H), 7.83 (d, *J =* 8.8 Hz, 1H), 7.64 - 7.57 (m, 2H), 7.54-7.48 (m, 2H), 7.45-7.38 (m, 2H), 6.14 (p, *J =* 6.7 Hz, 1H), 4.43(s,1H),4.26 (s, 2H), 3.85 (d, *J =* 5.3 Hz, 4H), 3.00 (s, 2H), 2.79 (s, 4H), 1.80 (d, *J= 6.7* Hz, 6H),1.65(m, 2H).

### Preparation process of example 1-32:

Compound (4-aminophenyl)(1,1-dioxothiomorpholino)methanone **11z₆** (1.6 g, 6.3 mmol, 96%) was prepared from **12a** (1 g, 7.3 mmol) and thiomorpholine 1,1-dioxide **13z₆** (0.89 g, 6.6 mmol) by referring to the synthetic method of **11a.** C₁₁H₁₅N₂O₃S [M+H]⁺: m/z = 255.1.

Compound **I-32** (139 mg, 300 µmol, 72%) was prepared from **10a** (150 mg, 416 µmol) and **11z₆** (127 mg, 499 µmol) by referring to the synthetic method of **I-1** in example 1. **¹H NMR** (400 MHz, Chloroform-*d, ppm)* δ 9.22 (s, 1H), 7.94 (d, *J=* 8.9 Hz, 1H), 7.87 (d, *J =* 8.6 Hz, 2H), 7.63 - 7.57 (m, 2H), 7.52 (d, *J =* 8.5 Hz, 2H), 6.23 - 6.17 (m, 1H), 4.17 (s, 4H), 3.10 (s, 4H), 1.87 (d, *J* = 6.7 Hz, 6H).

### Preparation process of example 1-33:

Compound **11z₇** (1.9 g, 6.1 mmol, 92%) was prepared from **12a** (1 g, 7.3 mmol) and tert-butyl 1,4-diazepane-1-carboxylate **13z₇** (1.3 g, 6.6 mmol) by referring to the synthetic method of **11a.** C₁₇H₂₆N₃O₃ [M+H]⁺: m/z = 320.2.

Compound **I-33** (134 mg, 312 µmol, two steps 76%) was prepared from **10a** (150 mg, 416 µmol) and tert-butyl 4-(4-aminobenzoyl)-1,4-diazepane-1-carboxylate **11z₇** (159 mg, 499 µmol) by referring to the synthetic method of **I-4** in example 1. **¹H NMR** (400 MHz, Chloroform-d, *ppm)* δ 9.19 (s, 1H), 7.90 (d, *J* = 8.8 Hz, 1H), 7.79 (d, *J* = 8.2 Hz, 2H), 7.59 (d, *J* = 8.3 Hz, 2H), 7.49 (d, *J* = 8.2 Hz, 2H), 6.21 (p, *J* = 6.7 Hz, 1H), 3.81 (s, 2H), 3.57 (s, 2H), 3.11 (s, 1H), 2.97 (s, 3H), 1.85 (d, *J* = 6.7 Hz, 8H).

### Preparation process of example 1-34:

Compound (4-aminophenyl)(4-methyl-1,4-diazepan-1-yl)methanone **11z₈** (1.5 g, 6.3 mmol, 95%) was prepared from **12a** (1 g, 7.3 mmol) and 1-methyl-1,4-diazepane **13z₈** (0.75 g, 6.6 mmol) by referring to the synthetic method of **11a.** C₁₃H₂₀N₃O [M+H]⁺: m/z = 234.2.

Compound **I-34** (102 mg, 229.6 µmol, 56%) was prepared from **10a** (150 mg, 416 µmol) and **11z₈** (159 mg, 499 µmol) by referring to the synthetic method of **I-1** in example 1.**¹H NMR** (400 MHz, Chloroform-*d, ppm)* δ 9.19 (s, 1H), 7.91 (d, *J=* 8.8 Hz, 1H), 7.79 (d, *J=* 8.3 Hz, 2H), 7.59 (d, *J=* 8.8 Hz, 1H), 7.52 (d, *J* = 20.5 Hz, 3H), 6.21 (p, *J =* 6.7 Hz, 1H), 3.73 (dd, *J=* 83.7, 18.7 Hz, 4H), 2.84 (s, 1H), 2.65 (d, *J* = 23.8 Hz, 3H), 2.43 (d, *J* = 28.9 Hz, 3H), 2.00 (d, *J* = 33.6 Hz, 2H), 1.85 (d, *J* = 6.7 Hz, 6H).

### Preparation process of example 1-35:

Compound (4-aminophenyl)(morpholino)methanone **11z₉** (1.5 g, 6.3 mmol, 95%) was prepared from **12a** (1 g, 7.3 mmol) and morpholine **13z₉** (0.57 g, 6.6 mmol) by referring to the synthetic method of **11a.** C₁₂H₁₇N₂O; [M+H]⁺: m/z = 221.1.

Compound **I-35** (109 mg, 262 µmol, 63%) was prepared from **10a** (150 mg, 416 µmol) and **11z₉** (110 mg, 499 µmol) by referring to the synthetic method of **I-1** in example 1. **¹H NMR** (400 MHz, Chloroform-*d, ppm)* δ 9.20 (s, 1H), 7.91 (d, *J=* 8.8 Hz, 1H), 7.81 (d, *J=* 8.2 Hz, 2H), 7.62 - 7.54 (m, 2H), 7.50 (d, *J=* 8.2 Hz, 2H), 6.21 (p, *J =* 6.7 Hz, 1H), 3.74 (s, 8H), 1.86 (d, *J =* 6.7 Hz, 6H).

### Preparation process of example 1-36:

Compound (4-aminophenyl)(4-hydroxypiperidin-1-yl)methanone **11z₁₀** (1.4 g, 6.1 mmol, 93%) was prepared from **12a** (1 g, 7.3 mmol) and piperidin-4-ol **13z₁₀** (0.67 g, 6.6 mmol) by referring to the synthetic method of **11a.** C₁₃H₂₀N₃O [M+H]⁺: m/z = 221.1.

Compound **I-36** (146 mg, 339 µmol, 68%) was prepared from **10a** (150 mg, 416 µmol) and **11z₁₀** (110 mg, 499 µmol) by referring to the synthetic method of **I-1** in example 1. **¹H NMR** (400 MHz, Chloroform-*d, ppm)* δ 9.19 (s, 1H), 7.90 (d, *J* = 8.8 Hz, 1H), 7.82 - 7.77 (m, 2H), 7.59 (d, *J* = 8.8 Hz, 2H), 7.50 - 7.46 (m, 2H), 6.21 (p, *J* = 6.8 Hz, 1H), 4.02 (s, 1H), 3.36 (s, 2H), 1.95 (s, 2H), 1.85 (d, *J* = 6.7 Hz, 6H).1.63 (m, 4H).

### Preparation process of example 1-37:

Compound (4-aminophenyl)(4,4-difluoropiperidin-1-yl)methanone **11z₁₁** (1.4 g, 6.1 mmol, 93%) was prepared from **12a** (1 g, 7.3 mmol) and 4,4-difluoropyridine **13z₁₁** (0.8 g, 6.6 mmol) by referring to the synthetic method of **11a.** C₁₂H₁₅FN₂O [M+H]⁺: m/z = 241.1.

Compound **I-37** (122 mg, 270 µmol, 65%) was prepared from **10a** (150 mg, 416 µmol) and **11z₁₁** (120 mg, 499 µmol) by referring to the synthetic method of **I-1** in example 1. **¹H NMR** (400 MHz, Chloroform-*d, ppm*) δ 9.20 (s, 1H), 7.92 (d, *J* = 8.8 Hz, 1H), 7.82 (d, *J* = 8.2 Hz, 2H), 7.63 - 7.54 (m, 2H), 7.54 - 7.47 (m, 2H), 6.21 (p, *J* = 6.7 Hz, 1H), 3.78 (s, 4H), 2.04 (s, 4H), 1.86 (d, *J* = 6.7 Hz, 6H).

### Preparation of intermediate 1-cyclopentyl-1H-[1,2,3]triazolo[4,5-H]quinazolin-8-yl trifluoromethanesulfonate (10b)

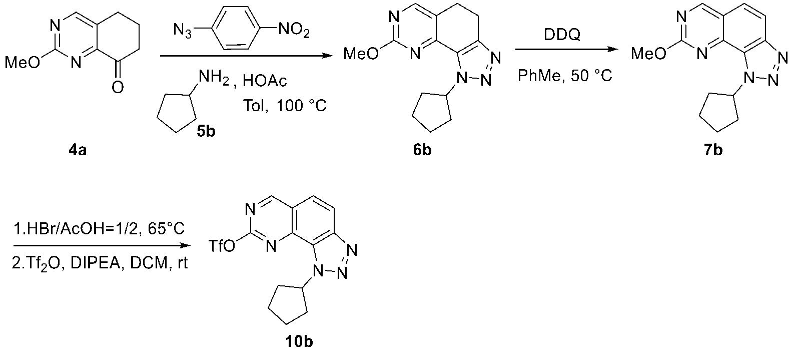

1) Compound **4a** (1.2 g, 6.74 mmol) was reacted to give 1-cyclopentyl-8-methoxy-4,5-dihydro-1H-[1,2,3]triazolo[4,5-H]quinazoline **6b** (1.06 g, 3.91 mmol, 58%) by referring to the synthetic method of 6a in example 1. **¹H NMR** (400 MHz, Chloroform-d, *ppm)* δ 8.46 (s, 1H), 4.68 (d, *J* = 7.4 Hz, 2H), 3.92 (s, 3H), 3.15 - 2.88 (m, 4H), 2.42 (dq, *J =* 13.2, 6.4 Hz, 2H), 2.32 (m, 1H), , 2.10 (td, *J =* 13.2, 6.4 Hz, 2H), 1.88 (d, *J* = 8.0 Hz, 4H).
2) **7b** (705 mg, 2.62 mmol, 71%) was prepared from **6b** (1.0 g, 3.69 mmol) by referring to the synthetic steps of **7a** in example 1. **LC-MS** (ESI), C₁₄H₁₆N₅O [M+H]⁺: m/z = 270.1.
3) **10b** (862 mg, 2.23 mmol, 85%) was prepared from **7b** (705 mg, 2.62 mmol) by referring to the synthetic steps of **10a** in example 1. **¹H NMR** (400 MHz, Chloroform-d, *ppm)* δ 9.39 (s, 1H), 8.21 (dd, *J* = 8.9, 0.8 Hz, 1H), 7.76 (dd, *J* = 8.9, 0.9 Hz, 1H), 6.14 (p, *J* = 6.7 Hz, 1H), 2.26 (tdd, *J* = 14.4, 7.5, 4.3 Hz, 4H), 2.05 - 1.92 (m, 2H), 1.85 - 1.71 (m, 2H).

**Table 3 Examples I-38 to I-42**

| **Example No.** | **Side chain structure** | **Example structure** | **Example name** | **LC-MS** (ESI) [M+H]⁺ |
|---|---|---|---|---|
| **I-38** | 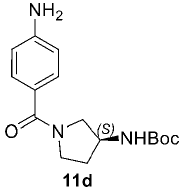 | 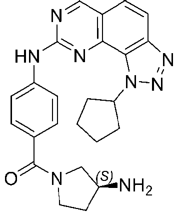 | (S)-(3-Aminopyrrolidin-1-yl)(4-((1-cyclopentyl-1H-[1,2,3]triazolo[4,5-H]quinazolin-8-yl)amino)phenyl)methanone | 443.2 |
| **I-39** | 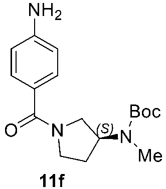 | 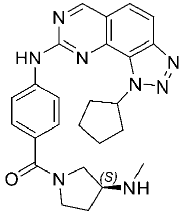 | (S)-(4-((1-cyclopentyl-1H-[1,2,3]triazolo[4,5-H]quinazolin-8-yl)amino)phenyl)(3-(methylamino)pyrrolidin-1-yl)methanone | 457.2 |
| **I-40** | 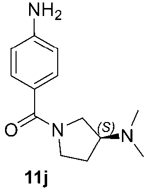 | 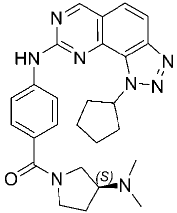 | (S)-(4-((1-cyclopentyl-1H-[1,2,3]triazolo[4,5-H]quinazolin-8-yl)amino)phenyl)(3-(dimethylamino)pyrrolidin-1-yl)methanone | 471.3 |
| **I-41** | 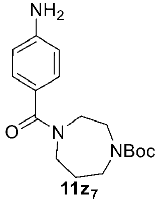 | 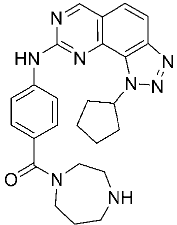 | (4-((1-Cyclopentyl-1H-[1,2,3]triazolo[4,5-H]quinazolin-8-yl)amino)phenyl)(1,4-diazepan-1-yl)methanone | 457.2 |
| **I-42** | 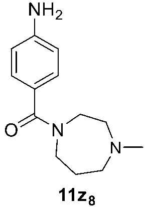 | 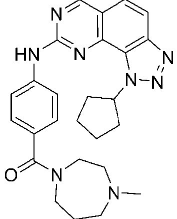 | (4-((1-Cyclopentyl-1H-[1,2,3]triazolo[4,5-H]quinazolin-8-yl)amino)phenyl)(4-methyl-1,4-diazepan-1-yl)methanone | 471.3 |

### Preparation of example I-38:

Compound **I-38** (108 mg, 244 µmol, two steps 63%) was prepared from **10b** (150 mg, 387 µmol) and tert-butyl (S)-(1-(4-aminobenzoyl)pyrrolidin-3-yl)carbamate **11d** (142 mg, 465 µmol) by referring to the synthetic method of **I-4** in example 1. **¹H NMR** (400 MHz, DMSO-*d*₆, *ppm)* δ 10.40 (s, 1H), 9.46 (s, 1H), 7.91 (dd, *J =* 13.7, 8.6 Hz, 3H), 7.80 (d, *J=* 8.8 Hz, 1H), 7.57 (t, *J =* 7.3 Hz, 2H), 6.33 (t, *J* = 6.8 Hz, 1H), 4.47 (s, 2H)3.66 (s, 3H), 3.52 (s, 2H), 2.36 (q, *J* = 7.2 Hz, 2H), 2.33 - 2.23 (m, 2H), 2.11 - 1.93 (m, 3H), 1.87 - 1.68 (m, 3H).

### Preparation of example I-39:

Compound **I-39** (102 mg, 224.6 µmol, two steps 58%) was prepared from **10b** (150 mg, 387 µmol) and tert-butyl (S)-(1-(4-aminobenzoyl)pyrrolidin-3-yl)(methyl)carbamate **11f** (148 mg, 465 µmol) by referring to the synthetic method of **I-4** in example 1. **¹H NMR** (400 MHz, Chloroform-d, *ppm)* δ 9.20 (s, 1H), 7.92 (d, *J =* 8.8 Hz, 1H), 7.82 (d, *J* = 8.2 Hz, 2H), 7.63 (dt, *J=* 19.7, 5.8 Hz, 4H), 6.39 (p, *J* = 6.8 Hz, 1H), 3.96 - 3.70 (m, 3H), 3.63 - 3.51 (m, 1H), 3.35 (d, *J =* 44.0 Hz, 1H), 2.58 - 2.36 (m, 7H), 2.27 - 2.05 (m, 3H), 1.90 (d, *J* = 13.6 Hz, 3H).

### Preparation of example I-40:

Compound **I-40** (75 mg, 159 µmol, 41%) was prepared from **10b** (150 mg, 387 µmol) and (S)-(4-aminophenyl)(3-(dimethylamino)pyrrolidin-1-yl)methanone **11j** (108 mg, 465 µmol) by referring to the synthetic method of **I-1** in example 1. **¹H NMR** (400 MHz, Chloroform-, *ppm d*) δ 9.19 (s, 1H), 7.90 (d, *J* = 8.8 Hz, 1H), 7.81 (d, *J* = 8.1 Hz, 2H), 7.60 (t, *J* = 9.0 Hz, 4H), 6.37 (t, *J* = 6.9 Hz, 1H), 3.69 (t, *J* = 67.8 Hz, 4H), 2.81 (d, *J =* 47.2 Hz, 1H), 2.46 - 2.36 (m, 6H), 2.28 (s, 2H), 2.11 (s, 2H), 1.91 (d, *J =* 18.9 Hz, 4H), 1.66 (s, 2H).

### Preparation of example I-41:

Compound **I-41** (104 mg, 252 µmmol, two steps 65%) was prepared from **10b** (150 mg, 387 µmol) and tert-butyl 4-(4-aminobenzoyl)-1,4-diazepane-1-carboxylate **11z₇** (148 mg, 465 µmol) by referring to the synthetic method of **I-4** in example 1. **¹H NMR** (400 MHz, DMSO-*d*₆, *ppm)* δ 10.38 (s, 1H), 9.46 (s, 1H), 7.95 - 7.87 (m, 3H), 7.80 (d, *J* = 8.8 Hz, 1H), 7.47 (d, *J* = 8.1 Hz, 2H), 6.33 (p, *J* = 6.8 Hz, 1H), 3.65 (d, *J* = 102.0 Hz, 4H), 3.23 - 3.06 (m, 4H), 2.41 - 2.32 (m, 2H), 2.32 - 2.22 (m, 2H), 2.03 - 1.87 (m, 4H), 1.87 - 1.74 (m, 2H).

### Preparation of example I-42:

Compound **I-42** (69 mg, 147 µmol, 38%) was prepared from **10b** (150 mg, 387 µmol) and (4-aminophenyl)(4-methyl-1,4-diazepan-1-yl)methanone **11z₈** (108 mg, 465 µmol) by referring to the synthetic method of **I-1** in example 1. **¹H NMR** (400 MHz, Chloroform-d, *ppm)* δ 9.19 (s, 1H), 7.91 (d, *J=* 8.8 Hz, 1H), 7.83 (d, *J* = 8.2 Hz, 2H), 7.65 (s, 1H), 7.59 (d, *J* = 8.8 Hz, 1H), 7.51 (d, *J* = 7.7 Hz, 2H), 6.35 (p, *J* = 6.9 Hz, 1H), 4.20 - 3.53 (m, 4H), 3.25 (s, 1H), 3.02 (s, 1H), 2.73 (s, 2H), 2.40 (dq, *J* = 15.2, 7.9 Hz, 5H), 2.11 (d, *J* = 8.4 Hz, 3H), 1.86 (q, *J =* 6.5, 6.0 Hz, 3H), 1.25 (s, 2H).

### Preparation of intermediate 4-(difluoromethyl)-1-isopropyl-8-methoxy-1H-[1,2,3]triazolo[4,5-H]quinazoline (10c)

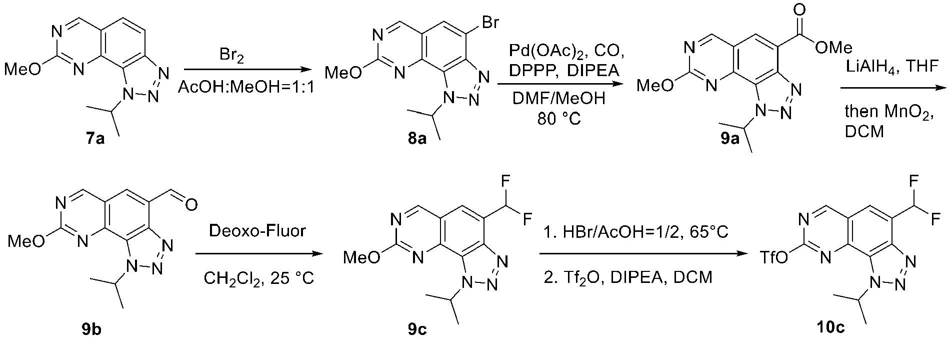

### 1): 4-Bromo-1-isopropyl-8-methoxy-1H-[1,2,3]triazolo[4,5-h]quinazoline (8a)

Br₂ (12.5 g, 80.0 mmol) was added to a solution of **7a** (3.9 g, 16.0 mmol) in AcOH/MeOH (1:1, 120 mL) at 0 °C, and the resulting mixture was reacted for 2 hours. Saturated aqueous solution of Na₂S₂O₃ (60 mL) was added to quench the reaction. The aqueous phase was extracted with CH₂Cl₂ (150 mL × 3). The organic phases were combined, washed with saturated aqueous solution of NaCl, dried over anhydrous sodium sulfate, vacuum filtered, and concentrated. The resulting residue was purified by flash chromatography on a silica gel column (petroleum ether/ethyl acetate = 6:1) to give a light yellow solid, which was the title compound **8a** (4.89 g, 15.2 mmol, 95%). **¹H NMR** (400 MHz, Chloroform-d, *ppm)* δ 9.25 (s, 1H), 7.89 (s, 1H), 6.13 (h, *J*= 6.7 Hz, 1H), 4.20 (s, 3H), 1.86 (d, *J* = 6.7 Hz, 6H).

### 2): Methyl 1-isopropyl-8-methoxy-1H-[1,2,3]triazolo[4,5-h]quinazolin-4-carboxylate (9a)

1,3-Bis(diphenylphosphino)propane (1.3 g, 2.28 mmol), palladium acetate (340 mg, 1.5 mmol), and N,N-diisopropylethylamine (10.5 mL, 76 mmol) were added to a solution of **8a** (4.9 g, 15.2 mmol) in N,N-dimethylformamide (38 mL) and methanol (38 mL) at room temperature. The reaction solution was purged with carbon monoxide gas twice. Then, the reaction solution was heated to 80 °C and reacted for 12 h under carbon monoxide. The resulting mixture was diluted with ethyl acetate (60 mL) and saturated aqueous solution of sodium bicarbonate (50 mL). The organic phase was separated, then washed with water (50 mL × 2) and saturated brine (60 mL × 1), respectively, dried over anhydrous sodium sulfate, filtered, and concentrated under reduced pressure. The resulting residue was purified by flash chromatography on a silica gel column (petroleum ether/ethyl acetate = 6:1) to give a white solid, which was the title compound **9a** (4.1 g, 13.3 mmol, 88%). **¹H NMR** (400 MHz, Chloroform-*d, ppm*) δ 9.41 (s, 1H), 8.50 (s, 1H), 6.19 (h, *J* = 6.8 Hz, 1H), 4.24 (s, 3H), 4.14 (s, 3H), 1.86 (d, *J* = 6.8 Hz, 6H).

### 3): 1-Isopropyl-8-methoxy-1H-[1,2,3]triazolo[4,5-h]quinazolin-4-carbaldehyde (9b)

Lithium aluminium hydride (2.0 g, 53.5 mmol) was added in batches to a solution of **9a** (4.1 g, 13.4 mmol) in tetrahydrofuran (133 mL) in an ice bath. The reaction solution was warmed to room temperature, stirred and reacted for another 2 hours. Then, the reaction flask was transferred to an ice-water bath. The reaction solution was diluted with ethyl acetate (40 mL), and saturated aqueous solution of potassium sodium tartrate (30 mL) was carefully added dropwise to quench the reaction. The reaction solution was warmed to room temperature again and stirred for 1 hour. The aqueous layer was separated and extracted with ethyl acetate (100 mL × 2). The combined organic phases were washed with saturated brine (100 mL × 1), dried (over anhydrous sodium sulfate), filtered, and concentrated under reduced pressure. The resulting residue was dissolved in dichloromethane (100 mL). Manganese dioxide (3 g) was added, and the resulting mixture was refluxed, stirred, and reacted overnight. The resulting mixture was concentrated under reduced pressure. The resulting residue was purified by flash chromatography on a silica gel column (petroleum ether/ethyl acetate = 12:1) to give a light yellow solid, which was the title compound **9b** (2.7 g, 9.8 mmol, 73%). **¹H NMR** (400 MHz, Chloroform-d, *ppm) δ* 10.92 (s, 1H), 9.47 (s, 1H), 8.34 (s, 1H), 6.16 (h, *J* = 6.8 Hz, 1H), 4.26 (s, 3H), 1.89 (d, *J* = 6.7 Hz, 6H).

### 4): 4-(Difluoromethyl)-1-isopropyl-8-methoxy-1H-[1,2,3]triazolo[4,5-h]quinazoline (9c)

Bis(2-methoxyethyl)aminosulfur trifluoride (4.3 g, 19.5 mmol) was added to a solution of **9b** (2.7 g, 9.8 mmol) in dichloromethane (100 mL) at room temperature. Then, the resulting mixture was stirred and reacted overnight. The reaction solution was directly concentrated under reduced pressure, and the resulting residue was purified by flash chromatography on a silica gel column (petroleum ether/ethyl acetate = 6:1) to give a light yellow solid, which was the title compound **9c** (2.4 g, 8.2 mmol, 84%). **¹H NMR** (400 MHz, Chloroform-d, *ppm)* δ 9.36 (s, 1H), 7.94 (t, *J* = 1.8 Hz, 1H), 7.52 (d, *J* = 55.0 Hz, 1H), 6.14 (p, *J* = 6.7 Hz, 1H), 4.22 (s, 3H) 1.86 (d, *J* = 6.7 Hz, 6H).

### 5) 10c (2.6 g, 6.2 mmol, 76%) was prepared from 9c (2.4 g, 8.2 mmol) by referring to the synthetic steps of 10a in example 1. ¹H NMR (400 MHz, Chloroform-d, ppm) δ 9.61 (s, 1H), 8.14 (t, J = 1.8 Hz, 1H), 7.52 (t, J = 54.6 Hz, 1H), 6.06 (hept, J = 6.7 Hz, 1H), 1.87 (d, J = 6.7 Hz, 6H).

**Table 4 Examples I-43 to I-47**

| **Example No.** | **Side chain structure** | **Example structure** | **Example name** | **LC-MS** (ESI) [M+H]⁺ |
|---|---|---|---|---|
| **I-43** | 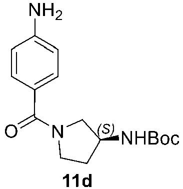 | 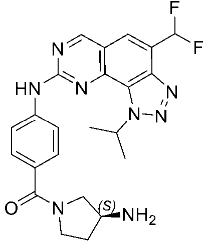 | (S)-(3-Aminopyrrolidin-1-yl)(4-(4-(difluoromethyl)-1-isopropyl-1H-[1,2,3]triazolo[4,5-H]quinazolin-8-yl)amino)phenyl)methanone | 447.2 |
| **I-44** | 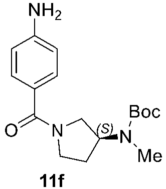 | 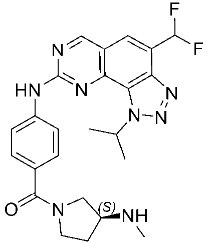 | (S)-(4-((4-(difluoromethyl)-1-isopropyl-1H-[1,2,3]triazolo[4,5-H]quinazolin-8-yl)amino)phenyl)(3-(methylamino)pyrrolidin-1-yl)methanone | 481.2 |
| **I-45** | 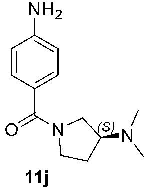 | 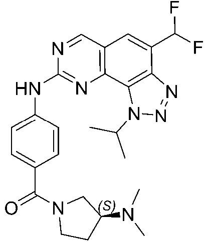 | (S)-(4-((4-(Difluoromethyl)-1-isopropyl-1H-[1,2,3]triazolo[4,5-H]quinazolin-8-yl)amino)phenyl)(3-(dimethylamino)pyrrolidin-1-yl)methanone | 495.2 |
| **I-46** | 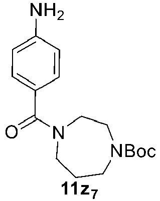 | 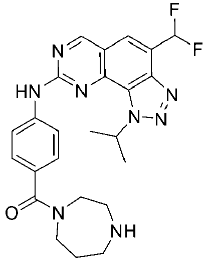 | (1,4-Diazepan-1-yl)(4-(4-(difluoromethyl)-1-isopropyl-1H-[1,2,3]triazolo[4,5-H]quinazolin-8-yl)amino)phenyl)methanone | 481.2 |
| **I-47** | 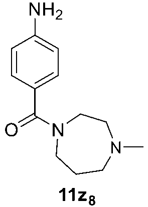 | 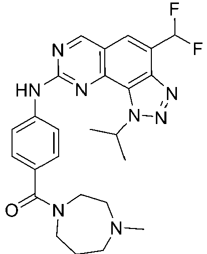 | (4-((4-(Difluoromethyl)-1-isopropyl-1H-[1,2,3]triazolo[4,5-H]quinazolin-8-yl)amino)phenyl)(4-methyl-1,4-diazepan-1-yl)methanone | 495.2 |

### Preparation of example I-43:

Compound **I-43** (67 mg, 151 µmol, two steps 62%) was prepared from **10c** (100 mg, 243 µmol) and tert-butyl (S)-(1-(4-aminobenzoyl)pyrrolidin-3-yl)carbamate **11d** (89 mg, 292 µmol) by referring to the synthetic method of **I-4** in example 1. **¹H NMR** (400 MHz, DMSO-*d*₆, *ppm) δ* 10.54 (s, 1H), 9.54 (s, 1H), 8.09 (t, *J* = 1.9 Hz, 1H), 7.96 - 7.86 (m, 2H), 7.72 - 7.49 (m, 3H), 6.20 (p, *J* = 6.5 Hz, 1H), 3.69 - 3.55 (m, 2H), 3.46 (dq, *J =* 13.5, 6.4, 5.6 Hz, 3H), 3.16 (d, *J* = 10.7 Hz, 1H), 2.04 - 1.86 (m, 2H), 1.78 (d, *J* = 6.7 Hz, 6H), 1.65 (d, *J* = 6.7 Hz, 1H).

### Preparation of example I-44:

Compound **I-44** (65 mg, 136 µmol, two steps 56%) was prepared from **10c** (100 mg, 243 µmol) and tert-butyl (S)-(1-(4-aminobenzoyl)pyrrolidin-3-yl)(methyl)carbamate **11f** (93 mg, 292 µmol) by referring to the synthetic method of **I-4** in example 1. **¹H NMR** (400 MHz, Chloroform-d, *ppm)* δ 9.26 (s, 1H), 7.88 (s, 1H), 7.80 (d, *J* = 8.3 Hz, 2H), 7.75 (s, 1H), 7.67 (d, *J=* 7.8 Hz, 2H), 7.53 (d, *J=* 55.2 Hz, 1H), 6.22 (p, *J= 6.7* Hz, 1H), 3.95 - 3.70 (m, 3H), 3.63 - 3.48 (m, 1H), 3.41 - 3.28 (m, 1H), 2.48 (d, *J=* 39.6 Hz, 3H), 2.26 - 2.02 (m, 1H), 1.87 (d, *J* = 6.7 Hz, 7H).

### Preparation of example I-45:

Compound **I-45** (50 mg, 105 µmol, 43%) was prepared from **10c** (100 mg, 243 µmol) and (S)-(4-aminophenyl)(3-(dimethylamino)pyrrolidin-1-yl)methanone **11j** (68 mg, 292 µmol) by referring to the synthetic method of **I-1** in example 1. **¹H NMR** (400 MHz, Chloroform-d, *ppm)* δ 9.25 (s, 1H), 7.87 (t, *J =* 1.8 Hz, 1H), 7.79 (d, *J* = 8.2 Hz, 2H), 7.69 - 7.61 (m, 3H), 7.51 (d, *J* = 55.1 Hz, 1H), 6.20 (q, *J* = 6.7 Hz, 1H), 4.04 - 3.81 (m, 1H), 3.77 - 3.41 (m, 3H), 2.78 (d, *J =* 41.8 Hz, 1H), 2.31 (d, *J* = 35.8 Hz, 6H), 2.16 - 1.89 (m, 2H), 1.87 - 1.79 (m, 6H).

### Preparation of example I-46:

Compound **I-46** (77 mg, 156 µmmol, two steps 64%) was prepared from **10c** (100 mg, 243 µmol) and tert-butyl 4-(4-aminobenzoyl)-1,4-diazepane-1-carboxylate **11z₇** (93 mg, 292 µmol) by referring to the synthetic method of **I-4** in example 1. **¹H NMR** (400 MHz, DMSO-*d*₆, *ppm) δ* 10.50 (s, 1H), 9.52 (s, 1H), 8.08 (d, *J =* 2.4 Hz, 1H), 7.89 (d, *J* = 8.2 Hz, 2H), 7.71 - 7.43 (m, 3H), 6.19 (p, *J* = 6.7 Hz, 1H), 3.61 (d, *J* = 17.1 Hz, 2H), 3.41 *(d, J=* 19.2 Hz, 3H), 2.83 (d, *J =* 44.4 Hz, 6H), 1.77 (d, *J* = 6.7 Hz, 6H).

### Preparation of example I-47:

Compound **I-47** (51 mg, 102 µmol, 42%) was prepared from **10c** (100 mg, 243 µmol) and (4-aminophenyl)(4-methyl-1,4-diazepan-1-yl)methanone **11z₈** (68 mg, 292 µmol) by referring to the synthetic method of **I-1** in example 1. **¹H NMR** (400 MHz, Chloroform-d, *ppm)* δ 9.25 (s, 1H), 7.87 (d, *J =* 2.3 Hz, 1H), 7.80 (d, *J* = 8.2 Hz, 2H), 7.65 (s, 1H), 7.60 - 7.30 (m, 3H), 6.20 (p, *J* = 6.7 Hz, 1H), 4.03 - 3.59 (m, 4H), 3.08 (s, 1H), 2.82 (d, *J =* 30.4 Hz, 2H), 2.62 (s, 3H), 2.45 (s, 1H), 2.13 (s, 1H), 1.85 (d, *J* = 6.7 Hz, 6H), 1.68 (s, 1H).

### Preparation of intermediate (S)-1-(1-((triisopropylsilyl)oxy)propan-2-yl)-1H-[1,2,3]triazolo[4,5-H]quinazolin-8-yl trifluoromethanesulfonate (10d)

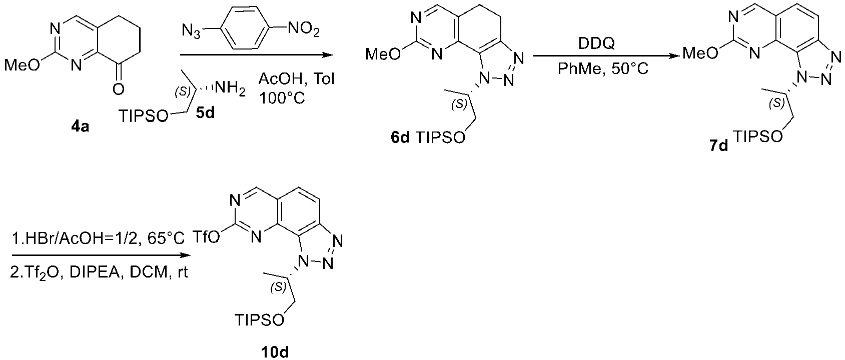

### 1): (S)-8-Methoxy-1-(1-((triisopropylsilyl)oxy)propan-2-yl)-4,5-dihydro-1H-[1,2,3]triazolo[4,5-H]quinazoline (6d)

**4a** (1.8 g, 10.0 mmol) was dissolved in toluene (50 mL). (S)-1-((triisopropylsilyl)oxy)propan-2-amine **5d** (6.5 g, 28.0 mmol), 1-azido-4-nitrobenzene (2.1 g, 13.0 mmol), and glacial acetic acid (171 µL, 3 mmol) were added successively at room temperature. The reaction solution was heated to 100 °C, stirred and reacted overnight. The reaction solution was cooled to room temperature and concentrated under reduced pressure. The resulting residue was purified by flash chromatography on a silica gel column (petroleum ether/ethyl acetate = 3:1) to give a light yellow solid, which was the title compound **6d** (2.8 g, 6.8 mmol, 68%). **LC-MS** (ESI) C₂₁H₃₄N₅O₂Si [M+H]⁺: m/z = 416.2.

### 2): (S)-8-Methoxy-1-(1-((triisopropylsilyl)oxy)propan-2-yl)-1H-[1,2,3]triazolo[4,5-h]quinazoline (7d)

2,3-Dichloro-5,6-dicyano-1,4-benzoquinone (3.1 g, 13.6 mmol) was added to a solution of **6d** (2.8 g, 6.8 mmol) in toluene (68 mL). The resulting mixture was heated to 50 °C and reacted for another 24 hours. The resulting mixture was concentrated under reduced pressure. The resulting residue was purified by flash chromatography on a silica gel column (petroleum ether/ethyl acetate = 20:1 to 6:1) to give a light yellow solid, which was the title compound **7d** (2.4 g, 5.8 mmol, 85%). **LC-MS** (ESI), C₂₁H₃₄N₅O₂Si [M+H]⁺: m/z = 416.2.

### 3: 10d (2.3 g, 4.3 mmol, 74%) was prepared from 7d (2.4 g, 5.8 mmol, 85%) by referring to the synthetic steps of 10a in example 1. ¹H NMR (400 MHz, Chloroform-d, ppm) δ 9.51 (d, J= 0.9 Hz, 1H), 8.28 (dd, J = 8.9, 1.2 Hz, 1H), 7.84 (dd, J = 9.0, 1.3 Hz, 1H), 6.27 - 6.10 (m, 1H), 4.33 (dd, J= 10.0, 7.9 Hz, 1H), 4.30 - 4.20 (m, 1H), 1.87 (dd, J = 6.9, 3.8 Hz, 3H), 0.92 - 0.81 (m, 3H), 0.79 (dd, J= 6.9, 2.1 Hz, 9H).

**Table 5 Examples I-48 to I-49**

| **Example No.** | **Side chain structure** | **Example structure** | **Example name** | **LC-MS** (ESI) [M+H]⁺ |
|---|---|---|---|---|
| **I-48** | 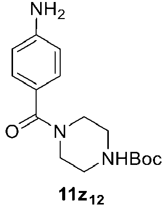 | 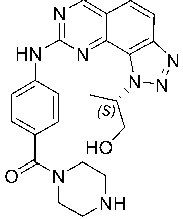 | (S)-(4-((1-(1-Hydroxypropan-2-yl)-1H-[1,2,3]triazolo[4,5-H]quinazolin-8-yl)amino)phenyl)(piperazin-1-yl)methanone | 433.2 |
| **I-49** | 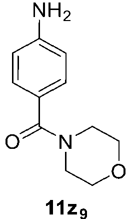 | 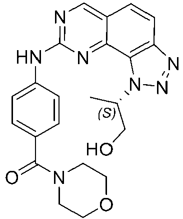 | (S)-(4-((1-(1-Hydroxypropan-2-yl)-1H-[1,2,3]triazolo[4,5-H]quinazolin-8-yl)amino)phenyl)(morpholino )methanone | 434.2 |

### Preparation of example I-48:

Compound tert-butyl 4-(4-aminobenzoyl)piperazine-1-carboxylate **11z₁₂** (1.4 g, 6.1 mmol, 93%) was prepared from **12a** (1 g, 7.3 mmol) and tert-butyl piperazine-1-carboxylate **13z₁₂** (1.2 g, 6.6 mmol) by referring to the synthetic method of **11a.** C₁₆H₂₄N₂O₃ [M+H]⁺: m/z = 306.2.

Compound **I-48** (43 mg, 99 µmol, two steps 53%) was prepared from **10d** (150 mg, 188 µmol) and **11z₁₂** (69 mg, 225 µmol) by referring to the synthetic method of **I-4** in example 1. **¹H NMR** (400 MHz, DMSO-*d*₆, *ppm) δ* 10.41 (s, 1H), 9.46 (s, 1H), 7.95 (d, *J =* 8.4 Hz, 2H), 7.90 (d, *J =* 8.8 Hz, 1H), 7.80 (d, *J =* 8.8 Hz, 1H), 7.50 (d, *J* = 8.4 Hz, 2H), 6.21 - 6.14 (m, 1H), 5.15 (t, *J* = 5.5 Hz, 1H), 4.11 (dt, *J* = 12.3, 5.8 Hz, 1H).

### Preparation of example I-49:

Compound **I-49** (54 mg, 124 µmol, 66%) was prepared from **10d** (150 mg, 188 µmol) and (4-aminophenyl)(morpholino)methanone **11z₉** (46 mg, 225 µmol) by referring to the synthetic method of **I-4a** in example 1. **¹H NMR** (600 MHz, DMSO-*d*₆, *ppm)* δ 10.37 (s, 1H), 9.45 (s, 1H), 7.99 - 7.76 (m, 4H), 7.47 (d, *J=* 8.2 Hz, 2H), 6.18 (q, *J =* 6.7 Hz, 1H), 5.11 (t, *J=* 5.5 Hz, 1H), 4.10 (dq, *J=* 13.0, 6.4 Hz, 1H), 3.99 (dt, *J* = 11.1, 4.9 Hz, 1H), 3.58 (d, *J* = 52.5 Hz, 9H), 1.71 (d, *J* = 6.9 Hz, 3H).

### Preparation of intermediate (R)-1-(1-((triisopropylsilyl)oxy)propan-2-yl)-1H-[1,2,3]triazolo[4,5-H]quinazolin-8-yl trifluoromethanesulfonate (10e)

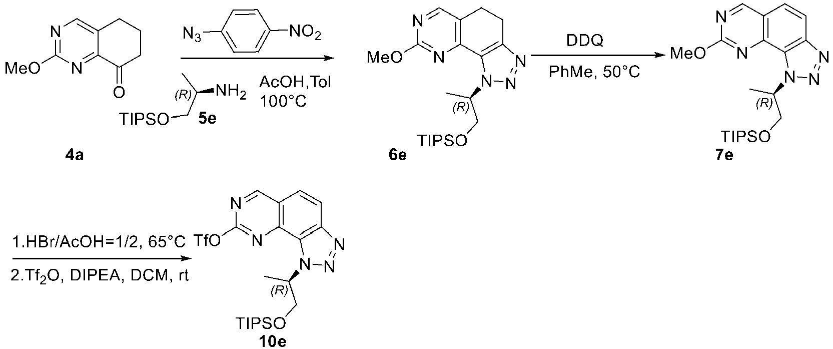

1) Compound **4a** (1.8 g, 10.0 mmol) was reacted to give **6e** (2.5 g, 6.2 mmol, 62%) by referring to the synthetic method of **6a** in example 1. **LC-MS** (ESI), C₂₁H₃₆N₅O₂Si [M+H]⁺: m/z = 418.3.
2) **6e** (2.5 g, 6.2 mmol) was reacted to give **7e** (2.3 g, 5.5 mmol, 88%) by referring to the synthetic method of **7a** in example 1. **LC-MS** (ESI), C₂₁H₃₄N₅O₂Si [M+H]⁺: m/z = 416.2.
3) **10e** (2.2 g, 4.1 mmol, 75%) was prepared from **7e** (2.3 g, 5.5 mmol) by referring to the synthetic steps of **10a** in example 1. **LC-MS** (ESI), C₂₁H₃₁F₃N₅O₄Si [M+H]⁺: m/z=534.2.

**Table 6 Examples I-50 to I-51**

| **Exampl e No.** | **Side chain structure** | **Example structure** | **Example name** | **LC-MS** (ESI) [M+H] + |
|---|---|---|---|---|
| **I-50** | 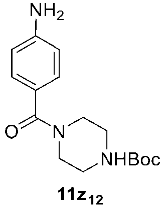 | 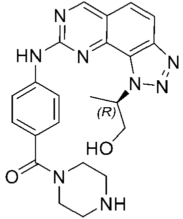 | (R)-(4-((1-(1-Hydroxypropan-2-yl)-1H-[1,2,3]triazolo[4,5-H]quinazolin-8-yl)amino)phenyl)(piperazin-1-yl)methanone | 433.2 |
| **I-51** | 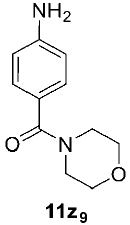 | 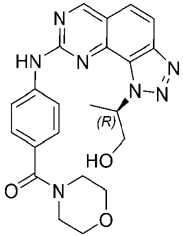 | (R)-(4-((1-(1-Hydroxypropan-2-yl)-1H-[1,2,3]triazolo[4,5-H]quinazolin-8-yl)amino)phenyl)(morpholino)methano ne | 434.2 |

### Preparation of example I-50:

Compound **I-50** (40 mg, 92 µmol, two steps 49%) was prepared from **10e** (150 mg, 188 µmol) and tert-butyl 4-(4-aminobenzoyl)piperazine-1-carboxylate **11z₁₂** (69 mg, 225 µmol) by referring to the synthetic method of **I-4** in example 1. **¹H NMR** (400 MHz, DMSO-*d*₆, *ppm)* δ 10.42 (s, 1H), 9.46 (s, 1H), 7.96 (d, *J* = 8.6 Hz, 2H), 7.90 (d, *J =* 8.8 Hz, 1H), 7.80 (d, *J =* 8.8 Hz, 1H), 7.52 (d, *J =* 8.4 Hz, 2H), 6.20 (p, *J =* 6.6 Hz, 1H), 5.20 - 5.14 (m, 1H), 4.16 - 4.07 (m, 1H), 3.98 (dt, *J =* 11.0, 5.4 Hz, 1H), 3.76 (s, 4H), 3.16 (t, *J =* 5.2 Hz, 4H), 1.72 (d, *J* = 6.8 Hz, 3H).

### Preparation of example I-51:

Compound **I-51** (47 mg, 109 µmol, 58%) was prepared from **10e** (150 mg, 188 µmol) and (4-aminophenyl)(morpholino)methanone **11z₉** (46 mg, 225 µmol) by referring to the synthetic method of **I-4a** in example 1. **¹H NMR** (400 MHz, DMSO-*d*₆, *ppm)* δ 10.38 (s, 1H), 9.45 (s, 1H), 7.98 - 7.91 (m, 2H), 7.89 (d, *J =* 8.8 Hz, 1H), 7.79 (d, *J =* 8.9 Hz, 1H), 7.50 - 7.44 (m, 2H), 6.21 - 6.14 (m, 1H), 5.13 (s, 1H), 4.11 (dd, *J* = 10.9, 7.5 Hz, 1H), 3.99 (dt, *J* = 10.9, 6.2 Hz, 1H), 3.71 - 3.44 (m, 8H), 1.71 (d, *J =* 6.8 Hz, 3H).

### Preparation of intermediate 1-(2-hydroxy-2-methylpropyl)-1H-[1,2,3]triazolo[4,5-H]quinazolin-8-yl trifluoromethanesulfonate (10f)

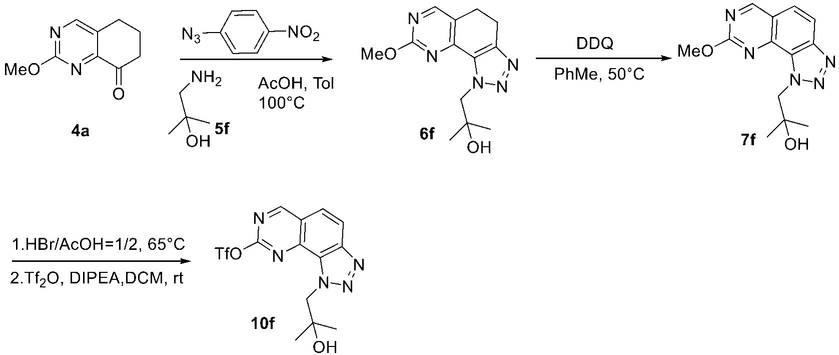

1) Compound **4a** (1.8 g, 10.0 mmol) was reacted to give **6f** (1.8 g, 6.5 mmol, 65%) by referring to the synthetic method of **6a** in example 1. **LC-MS** (ESI), C₁₃H₁₈N₅O₂ [M+H]⁺: m/z = 276.1.
2) **6f** (1.8 g, 6.5 mmol) was reacted to give **7f** (1.5 g, 5.5 mmol, 85%) by referring to the synthetic method of **7a** in example 1. **LC-MS** (ESI), C₁₃H₁₆N₅O₂ [M+H]⁺: m/z = 274.1.
3): **10f** (1.5 g, 3.9 mmol, 70%) was prepared from **7f** (1.5 g, 5.5 mmol) by referring to the synthetic steps of **10a** in example 1. **¹H NMR** (400 MHz, Chloroform-*d*, *ppm)* δ 9.53 (s, 1H), 8.30 (d, *J =* 8.9 Hz, 1H), 7.87 *(d, J=* 8.9 Hz, 1H), 5.28 (s, 2H), 2.77 (s, 1H), 1.38 (s, 6H).

**Table 7 Examples I-52 to I-53**

| **Exampl e No.** | **Side chain structure** | **Example structure** | **Example name** | **LC-MS** (ESI) [M+H] + |
|---|---|---|---|---|
| **I-52** | 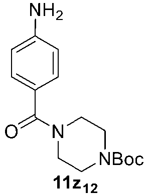 | 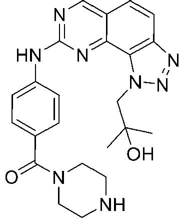 | (4-((1-(2-Hydroxy-2-methylpropyl)-1H-[1,2,3]triazolo[4,5-H]quinazolin-8-yl)amino)phenyl)(piperazin-1-yl)methanone | 447.2 |
| **I-53** | 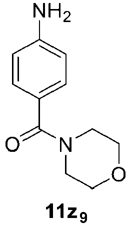 | 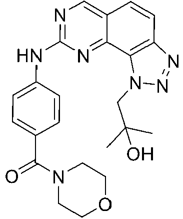 | (4-((1-(2-Hydroxy-2-methylpropyl)-1H-[1,2,3]triazolo[4,5-H]quinazolin-8-yl)amino)phenyl)(morpholino)methanon e | 448.2 |

### Preparation of example I-52:

Compound **I-52** (91 mg, 203 µmol, two steps 53%) was prepared from **10f** (150 mg, 384 µmol) and tert-butyl 4-(4-aminobenzoyl)piperazine-1-carboxylate **11z₁₂** (140 mg, 460 µmol) by referring to the synthetic method of **I-4** in example 1. **¹H NMR** (400 MHz, DMSO-*d*₆₀, *ppm)* δ 10.35 (d, *J* = 6.1 Hz, 1H), 9.45 (d,*J* = 1.8 Hz, 1H), 7.96 (dd, *J* = 8.3, 5.1 Hz, 2H), 7.90 (d, *J* = 8.8 Hz, 1H), 7.80 (d, *J* = 8.8 Hz, 1H), 7.44 (t, *J* = 8.3 Hz, 2H), 5.27 (s, 2H), 4.79 (d, *J=* 4.0 Hz, 1H), 3.65 - 3.38 (m, 4H), 3.17 (d, *J* = 4.5 Hz, 2H), 2.70 (s, 3H), 1.15 (s, 6H).

### Preparation of example I-53:

Compound **I-53** (111 mg, 249 µmol, 65%) was prepared from **10f** (150 mg, 384 µmol) and (4-aminophenyl)(morpholino)methanone **11z₉** (95 mg, 460 µmol) by referring to the synthetic method of **I-1** in example 1. **¹H NMR** (400 MHz, Chloroform-d, *ppm)* δ 9.22 (d, *J* = 0.9 Hz, 1H), 7.94 (dd, *J* = 8.8, 0.9 Hz, 1H), 7.77 - 7.68 (m, 2H), 7.63 (dd, *J* = 8.9, 0.9 Hz, 1H), 7.58 - 7.49 (m, 3H), 5.31 - 5.26 (m, 3H), 3.72 (s, 8H), 1.23 (s, 6H).

### Example I-54

### ((S)-3-Aminopyrrolidin-1-yl)(4-((1-((R)-3-hydroxy-3-methylbutan-2-yl)-1H-[1,2,3]triazolo[4,5-h]quinazolin-8-yl)amino)phenyl)methanone (I-54)

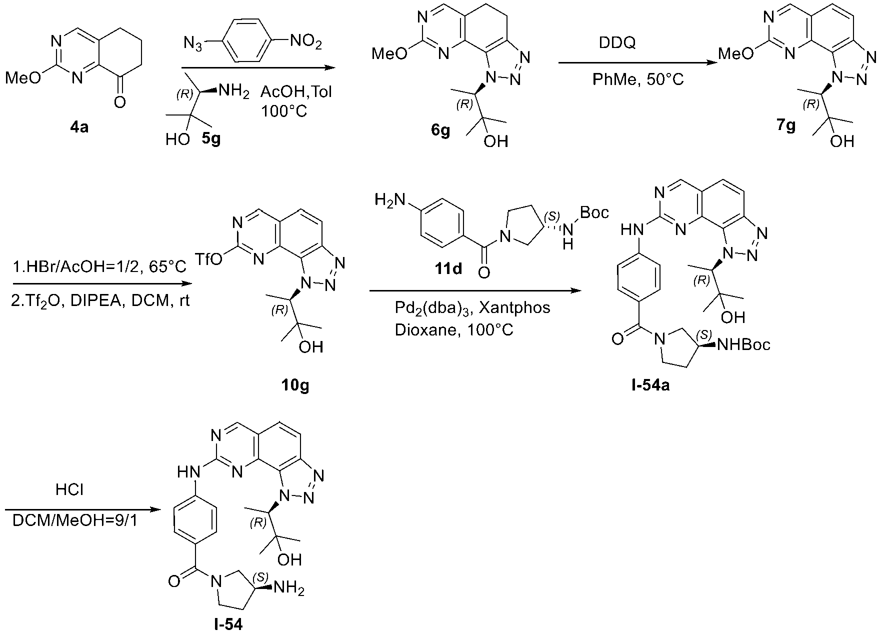

1): Compound **4a** (3.6 g, 20.0 mmol) was reacted to give **6g** (3.6 g, 12.4 mmol, 62%) by referring to the synthetic method of **6a. ¹H NMR** (400 MHz, Chloroform-d, *ppm)* δ 8.59 (s, 1H), 5.59 (q, *J* = 7.0 Hz, 1H), 3.89(s, 3H), 3.19 (q, *J* = 2.8, 1.7 Hz, 4H), 2.96 (s, 1H), 1.73 (d, *J* = 7.0 Hz, 3H), 1.32 (s, 3H), 1.17 (s, 3H).
2) **6g** (3.6 g, 12.4 mmol) was reacted to give **7g** (3.0 g, 10.3 mmol, 83%) by referring to the synthetic method of **7a. LC-MS** (ESI), C₁₄H₁₈N₅O₂ [M+H]⁺: m/z = 288.1.
3) **10g** (3.2 g, 7.8 mmol, 76%) was prepared from **7g** (3.0 g, 10.3 mmol) by referring to the synthetic steps of **10a** in example 1. **¹H NMR** (400 MHz, Chloroform-d, *ppm)* δ 9.54 (s, 1H), 8.30 (d, *J=* 8.9 Hz, 1H), 7.87 (d, *J* = 9.0 Hz, 1H), 6.09 (q, *J* = 7.0 Hz, 1H), 2.85 (s, 1H), 1.90 (d, *J* = 7.1 Hz, 3H), 1.35 (s, 3H), 1.27 (s, 3H).
4): tert-Butyl ((S)-1-(4-((1-((R)-3-hydroxy-3-methylbutan-2-yl)-1H-[1,2,3]triazolo[4,5-h]quinazolin-8-yl)amino)benzoyl)pyrrolidin-3-yl)carbamate **(I-54a)**

Tris(dibenzylideneacetone)dipalladium (18 mg, 10 mol%), 4,5-bis(diphenylphosphino-9,9-dimethylxanthene) (20 mg, 0.035 mmol), and potassium carbonate (57 mg, 0.42 mmol) were added to a solution of **10** g (150 mg, 0.35 mmol) and **11d** (158 mg, 0.52 mmol) in 1,4-dioxane (3 mL) at room temperature. After the addition, the reaction system was purged with argon twice. The resulting mixture was heated to 100 °C, sealed in a tube, and reacted for 4 hours. The reaction solution was cooled to room temperature, and then diluted with added ethyl acetate (20 mL) and semi-saturated aqueous solution of sodium bicarbonate (20 mL). The organic layer was separated, and the aqueous layer was extracted with ethyl acetate (30 mL) three times. The organic layers were combined, washed with saturated brine (30 mL), dried over anhydrous sodium sulfate, vacuum filtered, and concentrated. The resulting residue was purified by flash chromatography on a silica gel column (dichloromethane/methanol = 98:2) to give a light yellow solid, which was the title compound **I-54a** (150 mg, 0.27 mmol, 78%). **¹H NMR** (400 MHz, Chloroform-d, *ppm)* δ 9.21 (s, 1H), 7.93 (d, *J =* 8.9 Hz, 1H), 7.74 (d, *J* = 8.2 Hz, 2H), 7.62 (d, *J=* 9.0 Hz, 4H), 6.23 (q, *J* = 7.0 Hz, 1H), 4.65 (s, 1H), 4.26 (d, *J=* 52.7 Hz, 1H), 3.96 - 3.72 (m, 2H), 3.70 - 3.35 (m, 3H), 2.23 (d, *J=* 30.7 Hz, 1H), 1.92 (d, *J* = 8.8 Hz, 1H), 1.87 (d, *J* = 7.0 Hz, 3H), 1.61 (s, 9H), 1.36 (s, 3H), 1.19 (s, 3H).

5): Compound **I-54** (134 mg, 290.9 µmmol, 95%) was prepared from **I-54a** (150 mg, 0.35 mmol) by referring to the synthetic method of **I-4** in example 1. **¹H NMR** (400 MHz, DMSO-*d₆*, *ppm)* δ 10.37 (s, 1H), 9.45 (s, 1H), 7.96 (d, *J =* 8.4 Hz, 2H), 7.90 (d, *J* = 8.8 Hz, 1H), 7.80 (d, *J* = 8.9 Hz, 1H), 7.58 (t, *J* = 6.4 Hz, 2H), 6.35 (q, *J =* 7.0 Hz, 1H), 4.80 (s, 1H), 3.73 - 3.55 (m, 3H), 3.49 *(d, J=* 7.3 Hz, 2H), 3.29 - 3.17 (m, 2H), 2.00 (d, *J =* 6.8 Hz, 1H), 1.77 *(d, J=* 7.0 Hz, 3H), 1.73 - 1.65 (m, 1H), 1.26 (s, 3H), 1.10 (d, *J =* 2.5 Hz, 3H).

**Table 8 Examples I-55 to I-61**

| **Exampl e No.** | **Side chain structure** | **Example structure** | **Example name** | **LC-MS** (ESI) [M+H] + |
|---|---|---|---|---|
| **I-55** | 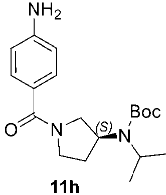 | 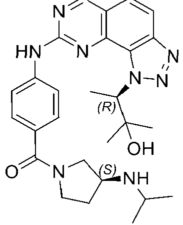 | (4-((1-((R)-3-hydroxy-3-methylbutan-2-yl)-1H-[1,2,3]triazolo[4,5-H]quinazolin-8-yl)amino)phenyl)((S)-3-(isopropylamino)pyrrolidin-1-yl)methanone | 503.3 |
| **I-56** | 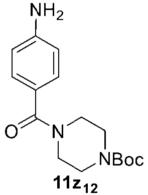 | 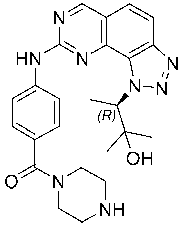 | (R)-(4-((1-(3-Hydroxy-3-methylbutan-2-yl)-1H-[1,2,3]triazolo[4,5-H]quinazolin-8-yl)amino)phenyl)(piperazin-1-yl)methanone | 461.2 |
| **I-57** | 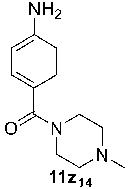 | 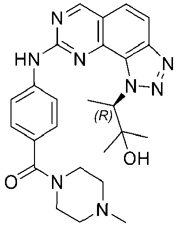 | (R)-(4-((1-(3-Hydroxy-3-methylbutan-2-yl)-1H-[1,2,3]triazolo[4,5-H]quinazolin-8-yl)amino)phenyl)(4-methylpiperazin-1-yl)methanone | 475.2 |
| **I-58** | 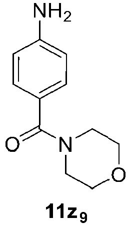 | 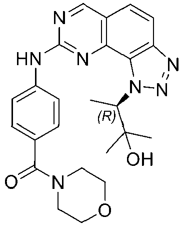 | (R)-(4-((1-(3-Hydroxy-3-methylbutan-2-yl)-1H-[1,2,3]triazolo[4,5-H]quinazolin-8-yl)amino)phenyl)(morpholino)methanone | 462.2 |
| **I-59** | 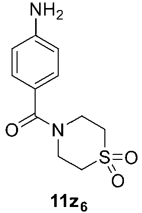 | 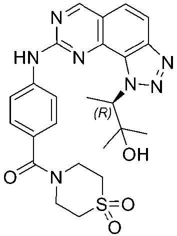 | (R)-(1,1-Dioxothiomorpholino)(4-((1-(3-hydroxy-3-methylbutan-2-yl)-1H-[1,2,3]triazolo[4,5-H]quinazolin-8-yl)amino)phenyl)methanone | 510.2 |
| **I-60** | 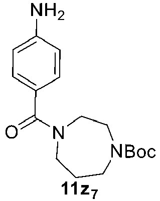 | 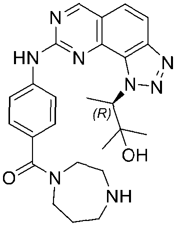 | (R)-(1,4-Diazepan-1-yl)(4-((1-(3-hydroxy-3-methylbutan-2-yl)-1H-[1,2,3]triazolo[4,5-H]quinazolin-8-yl)amino)phenyl)methanone | 475.2 |
| **I-61** | 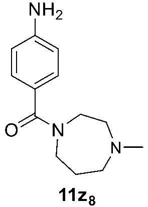 | 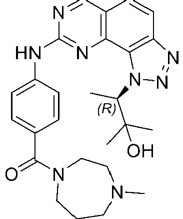 | (R)-(4-((1-(3-Hydroxy-3-methylbutan-2-yl)-1H-[1,2,3]triazolo[4,5-H]quinazolin-8-yl)amino)phenyl)(4-methyl-1,4-diazepan-1-yl)methanone | 489.3 |

### Preparation of example I-55:

Compound **I-55** (150 mg, 270 µmol, 73%) was prepared from **10g** (100 mg, 370 µmol) and tert-butyl (S)-(1-(4-aminobenzoyl)pyrrolidin-3-yl)(isopropyl)carbamate **11h** (154 mg, 445 µmol) by referring to the synthetic method of **I-54** in example 1. ¹**H NMR** (400 MHz, DMSO-*d₆*, *ppm)* δ 10.39 (s, 1H), 9.46 (s, 1H), 7.96 (d, *J* = 8.3 Hz, 2H), 7.90 (d, *J* = 8.8 Hz, 1H), 7.80 (d, *J* = 8.8 Hz, 1H), 7.58 (d, *J* = 8.2 Hz, 2H), 6.35 (q, *J* = 7.0 Hz, 1H), 4.80 (s, 1H), 3.71 (s, 1H), 3.57 (d, *J* = 43.6 Hz, 4H), 3.17 (d, *J* = 3.7 Hz, 1H), 2.10 (s, 1H), 1.77 (d, *J =* 7.1 Hz, 3H), 1.24 (d, *J* = 8.4 Hz, 6H), 1.11 (s, 6H), 1.01 (s, 2H).

### Preparation of example I-56:

Compound **I-56** (105 mg, 230 µmol, two steps, 62%) was prepared from **10g** (100 mg, 370 µmol) and tert-butyl 4-(4-aminobenzoyl)piperazine-1-carboxylate **11z₁₂** (136 mg, 445 µmol) by referring to the synthetic method of **I-54** in example 1. **¹H NMR**(400 MHz, DMSO-*d*₆, *ppm)* δ 10.37 (s, 1H), 9.45 (s, 1H), 7.95 (d, *J =* 8.5 Hz, 2H), 7.90 (d, *J =* 8.7 Hz, 1H), 7.79 (d, *J=* 8.8 Hz, 1H), 7.44 (d, *J=* 8.5 Hz, 2H), 6.32 (t, *J* = 7.1 Hz, 1H), 4.78 (s, 1H), 3.62 - 3.41 (m, 4H), 2.81 (s, 4H), 1.76 (d, *J=* 7.0 Hz, 3H), 1.23 (s, 3H), 1.10 (d, *J* = 6.6 Hz, 3H).

### Preparation of example I-57:

Compound (4-aminophenyl)(4-methylpiperazin-1-yl)methanone **11z₁₄** (1.34 g, 6.1 mmol, 93%) was prepared from **12a** (1 g, 7.3 mmol) and **13z** (0.66 g, 6.6 mmol) by referring to the synthetic method of **11a.** C₁₂H₁₈N₃O [M+H]⁺: m/z = 220.1.

Compound **I-57** (97 mg, 167 µmol, 45%) was prepared from **10g** (155 mg, 384 µmol) and **11z₁₄** (1 mg, 445 µmol) by referring to the synthetic method of **I-54a** in example 1. **¹H NMR** (400 MHz, Chloroform-*d, ppm)* δ 9.21 (s, 1H), 7.93 (d, *J =* 8.9 Hz, 1H), 7.73 (d, *J* = 8.2 Hz, 2H), 7.62 (d, *J* = 8.8 Hz, 1H), 7.58 (s, 1H), 7.50 (d, *J* = 8.2 Hz, 2H), 6.21 (q, *J* = 7.0 Hz, 1H), 3.58 (d, *J* = 51.9 Hz, 5H), 2.46 (s, 4H), 2.35 (s, 3H), 1.86 (d, *J* = 7.0 Hz, 3H), 1.37 (s, 3H), 1.07 (s, 3H).

### Preparation of example I-58:

Compound **I-58** (123 mg, 267 µmol, 72%) was prepared from **10g** (155 mg, 384 µmol) and (4-aminophenyl)(morpholino)methanone **11z₉** (92 mg, 445 µmol) by referring to the synthetic method of **I-54a** in example 1. **¹H NMR** (400 MHz, Chloroform-d, *ppm)* δ 9.21 (s, 1H), 7.93 (d, *J* = 8.8 Hz, 1H), 7.78 - 7.72 (m, 2H), 7.62 (d, *J* = 8.9 Hz, 1H), 7.58 (s, 1H), 7.53 - 7.48 (m, 2H), 6.21 (q, *J* = 7.0 Hz, 1H), 3.73 (s, 8H), 3.50 (s, 1H), 1.86 *(d, J=* 7.0 Hz, 3H), 1.37 (s, 3H), 1.09 (s, 3H).

### Preparation of example I-59:

Compound **I-59** (130 mg, 256 µmol, 69%) was prepared from **10g** (155 mg, 384 µmol) and (4-aminophenyl)(1,1-dioxothiomorpholino)methanone **11z₆** (113 mg, 445 µmol) by referring to the synthetic method of **I-54a** in example 1. **¹H NMR** (400 MHz, DMSO-*d*₆, *ppm)* δ 10.39 (s, 1H), 9.46 (s, 1H), 7.96 (d, *J* = 8.2 Hz, 2H), 7.90 (d, *J=* 8.7 Hz, 1H), 7.79 (d, *J=* 8.8 Hz, 1H), 7.54 (d, *J* = 8.2 Hz, 2H), 6.33 (q, *J=* 7.0 Hz, 1H), 4.77 (s, 1H), 3.91 (s, 4H), 3.27 (t, *J* = 5.3 Hz, 4H), 1.78 (d, *J* = 7.0 Hz, 3H), 1.24 (s, 3H), 1.08 (s, 3H).

### Preparation of example I-60:

Compound **I-60** (107 mg, 226 µmol, two steps 61%) was prepared from **10g** (100 mg, 370 µmol) and tert-butyl 4-(4-aminobenzoyl)-1,4-diazepane-1-carboxylate **11z₇** (255 mg, 445 µmol) by referring to the synthetic method of **I-54** in example 1. **¹H NMR** (400 MHz, DMSO-*d*₆, *ppm)* δ 10.37 (s, 1H), 9.46 (s, 1H), 7.99 - 7.93 (m, 2H), 7.90 (d, *J* = 8.8 Hz, 1H), 7.80 (d, *J* = 8.8 Hz, 1H), 7.47 (d, *J* = 8.2 Hz, 2H), 6.35 (q, *J* = 7.1 Hz, 1H), 4.78 (s, 1H), 3.64 (d, *J* = 93.8 Hz, 4H), 3.18 (s, 1H), 3.08 (s, 4H), 1.90 (s, 2H), 1.77 (d, *J* = 7.1 Hz, 3H), 1.24 (s, 3H), 1.10 (d, *J* = 3.8 Hz, 3H).

### Preparation of example I-61:

Compound **I-61** (96 mg, 196.3 µmol, 53%) was prepared from **10g** (155 mg, 384 µmol) and (4-aminophenyl)(4-methyl-1,4-diazepan-1-yl)methanone **11z₈** (104 mg, 446 µmol) by referring to the synthetic method of **I-54a** in example 1. **¹H NMR** (400 MHz, Chloroform-d, *ppm) δ* 9.21 (s, 1H), 7.93 (d, *J=* 8.8 Hz, 1H), 7.73 (d, *J=* 8.3 Hz, 2H), 7.62 (d, *J* = 8.8 Hz, 1H), 7.58 (s, 1H), 7.51 (d, *J =* 7.7 Hz, 2H), 6.23 - 6.18 (m, 1H), 3.83 (d, *J =* 22.9 Hz, 2H), 3.58 (t, *J* = 26.0 Hz, 3H), 2.78 (d, *J* = 75.9 Hz, 4H), 2.03 (s, 2H), 1.86 (d, *J* = 7.0 Hz, 3H), 1.37 (s, 3H), 1.08 (s, 3H).

### Preparation of the intermediate 1-((1R,2R)-2-hydroxy-2-methylcyclopentyl)-1H-[1,2,3]triazolo[4,5-h]quinazolin-8-yl trifluoromethanesulfonate (10h)

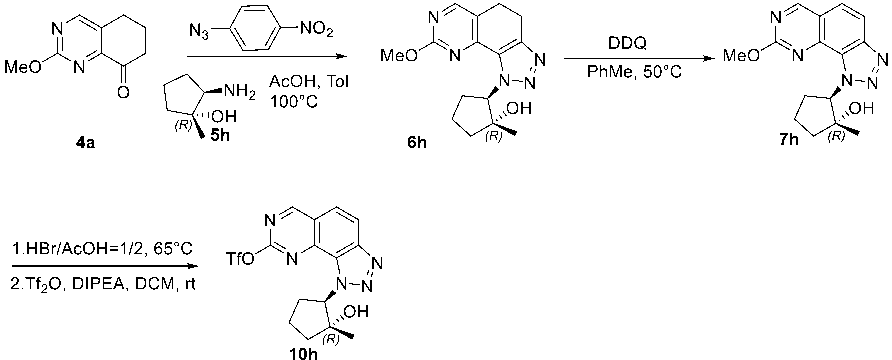

1) Compound **4a** (1.78 g, 10.0 mmol) was reacted to give **6h** (2.26 g, 7.5 mmol, 75%) by referring to the synthetic method of **6a** in example 1. **¹H NMR** (400 MHz, Chloroform-d, *ppm)* δ 8.58 (s, 1H), 5.67 (dd, *J =* 8.4, 6.9 Hz, 1H), 3.92 (s, 3H), 3.18 (d, *J =* 3.3 Hz, 4H), 2.70 (ddd, *J* = 13.9, 9.5, 7.0 Hz, 1H), 2.57 - 2.43 (m, 2H), 2.08 - 1.91 (m, 4H), 0.95 (s, 3H).
2) **6h** (2.26 g, 7.5 mmol) was reacted to give **7h** (1.9 g, 6.5 mmol, 87%) by referring to the synthetic method of **7a** in example 1. **LC-MS,** (ESI) C₁₅H₁₈N₅O₂ [M+H]⁺: m/z = 300.1.
3) **10h** (2.1 g, 5.1 mmol, 78%) was prepared from **7h** (1.9 g, 6.5 mmol) by referring to the synthetic steps of **10a** in example 1. **¹H NMR** (400 MHz, Chloroform-d, *ppm)* δ 9.55 (s, 1H), 8.30 (d, *J =* 8.9 Hz, 1H), 7.88 (d, *J* = 8.9 Hz, 1H), 6.19 (dd, *J =* 8.4, 6.6 Hz, 1H), 2.89 (dtd, *J* = 15.8, 9.0, 8.3, 4.2 Hz, 1H), 2.68 (dtd, *J* = 13.7, 8.5, 4.6 Hz, 1H), 2.57 (s, 1H), 2.24 - 1.94 (m, 4H), 0.90 (s, 3H).

**Table 9 Examples I-62 to I-71**

| **Exampl e No.** | **Side chain structure** | **Example structure** | **Example name** | **LC-MS**(ESI) [M+H] + |
|---|---|---|---|---|
| **I-62** | 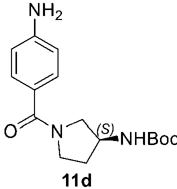 | 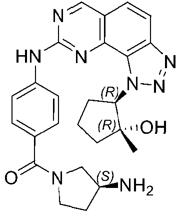 | ((S)-3-Aminopyrrolidin-1-yl)(4-((1-((1R,2R)-2-hydroxy-2-methylcyclopentyl)-1H-[1,2,3]triazolo[4,5-H]quinazolin-8-yl)amino)phenyl)methanone | 473.2 |
| **I-63** | 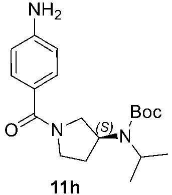 | 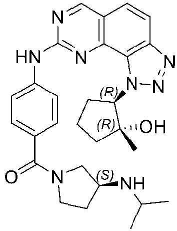 | (4-((1-((1R,2R)-2-Hydroxy-2-methylcyclopentyl)-1H-[1,2,3]triazolo[4,5-H]quinazolin-8-yl)amino)phenyl)((S)-3-(isopropylamino)pyrrolidin-1-yl)methanone | 515.3 |
| **I-64** | 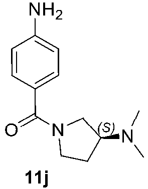 | 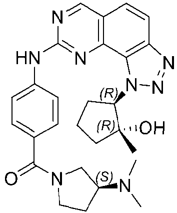 | ((S)-3-(Dimethylamino)pyrrolidin-1-yl)(4-((1-((1R,2R)-2-hydroxy-2-methylcyclopentyl)-1H-[1,2,3]triazolo[4,5-H]quinazolin-8-yl)amino)phenyl)methanone | 501.3 |
| **I-65** | 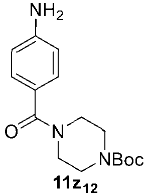 | 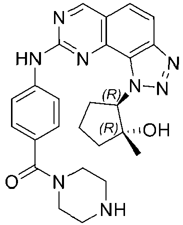 | (4-((1-((1R,2R)-2-Hydroxy-2-methylcyclopentyl)-1H-[1,2,3]triazolo[4,5-H]quinazolin-8-ylamino)phenyl)(piperazin-1-yl)methanone | 473.2 |
| **I-66** | 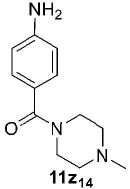 | 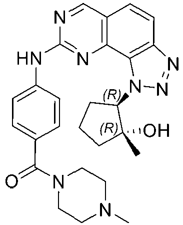 | (4-((1-((1R,2R)-2-Hydroxy-2-methylcyclopentyl)-1H-[1,2,3]triazolo[4,5-H]quinazolin-8-yl)amino)phenyl)(4-methylpiperazin-1-yl)methanone | 487.2 |
| **I-67** | 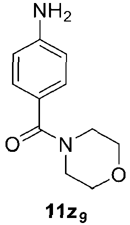 | 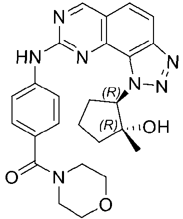 | (4-((1-((1R,2R)-2-Hydroxy-2-methylcyclopentyl)-1H-[1,2,3]triazolo[4,5-H]quinazolin-8-yl)amino)phenyl)(morpholino)methano ne | 474.2 |
| **I-68** | 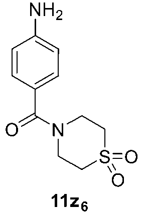 | 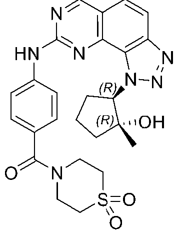 | (1,1-Dioxothiomorpholino)(4-((1-((1R,2R)-2-hydroxy-2-methylcyclopentyl)-1H-[1,2,3]triazolo[4,5-H]quinazolin-8-yl)amino)phenyl)methanone | 522.2 |
| **I-69** | 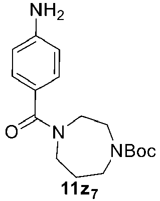 | 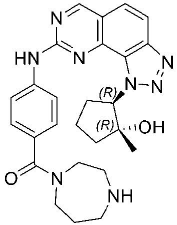 | (1,4-diazepan-1-yl)(4-((1-((1R,2R)-2-hydroxy-2-methylcyclopentyl)-1H-[1,2,3]triazolo[4, 5-H]quinazolin-8-yl)amino)phenyl)methanone | 487.2 |
| **I-70** | 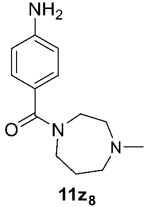 | 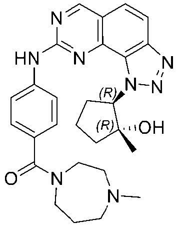 | (4-((1-((1,2R)-2-hydroxy-2-methylcyclopentyl)-1H-[1,2,3]triazolo[4, 5-H]quinazolin-8-yl)amino)phenyl)(4-methyl-1, 4-diazepan-1-yl)methanone | 501.3 |
| **I-71** | 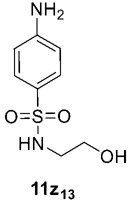 | 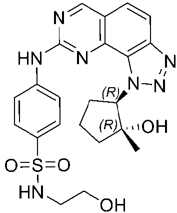 | 4-((1-(((1R,2R)-2-hydroxy-2-methylcyclopentyl)-1H-[1,2,3]triazolo[4,5-H]quinazolin-8-yl)amino)-N-(2-hydroxyethyl)benzenesulfonamide | 484.2 |

### Preparation of example I-62:

Compound **I-62** (110 mg, 234 µmol, two steps 65%) was prepared from **10h** (150 mg, 360 µmol) and tert-butyl (S)-(1-(4-aminobenzoyl)pyrrolidin-3-yl)carbamate **11d** (132 mg, 432 µmol) by referring to the synthetic method of **I-54** in example 1. **¹H NMR**(400 MHz, Chloroform-d, *ppm)* δ 9.21 (s, 1H), 7.91 (d, *J* = 8.8 Hz, 1H), 7.82 - 7.71 (m, 2H), 7.64 (q, *J* = 8.9 Hz, 4H), 6.25 (s, 1H), 3.94 - 3.26 (m, 6H), 2.85 (s, 1H), 2.63 (s, 1H), 2.26 - 2.01 (m, 4H), 1.91 (d, *J =* 4.6 Hz, 1H), 1.76 (s, 2H), 0.87 (s, 3H).

### Preparation of example I-63:

Compound **I-63** (104 mg, 201 µmol, 56%) was prepared from **10h** (150 mg, 360 µmol) and tert-butyl (S)-(1-(4-aminobenzoyl)pyrrolidin-3-yl)(isopropyl)carbamate **11h** (150 mg, 432 µmol) by referring to the synthetic method of **I-54** in example 1. **¹H NMR**(400 MHz, Chloroform-d, *ppm)* δ 9.20 (s, 1H), 7.90 (d, *J* = 8.8 Hz, 1H), 7.82 (s, 1H), 7.74 (s, 2H), 7.61 (dd, *J* = 12.1, 8.5 Hz, 3H), 6.27 (d, *J* = 7.7 Hz, 1H), 3.98 - 3.30 (m, 5H), 2.90 (d, *J* = 50.0 Hz, 2H), 2.64 (s, 1H), 2.28 - 2.05 (m, 5H), 1.91 (d, *J* = 9.6 Hz, 3H), 1.79 (s, 2H), 1.13 (s, 2H), 1.06 (s, 2H), 0.89 (s, 3H).

### Preparation of example I-64:

Compound **I-64** (95 mg, 191 µmol, 53%) was prepared from **10h** (150 mg, 360 µmol) and (S)-(4-aminophenyl)(3-(dimethylamino)pyrrolidin-1-yl)methanone **11j** (101 mg, 432 µmol) by referring to the synthetic method of **I-54a** in example 1. **¹H NMR** (400 MHz, Chloroform-d, *ppm)* δ 9.21 (s, 1H), 7.91 (d, *J* = 8.8 Hz, 1H), 7.77 (dd, *J* = 16.5, 8.4 Hz, 3H), 7.67 - 7.52 (m, 3H), 6.26 (dd, *J* = 19.2, 7.8 Hz, 1H), 3.88 (dd, *J* = 47.4, 9.6 Hz, 1H), 3.75 - 3.41 (m, 3H), 3.00 - 2.48 (m, 4H), 2.32 (s, 3H), 2.26 (s, 3H), 2.23 - 2.05 (m, 4H), 1.96 - 1.82 (m, 2H), 0.89 - 0.87 (m, 3H).

### Preparation of example I-65:

Compound **I-65** (112 mg, 237 µmol, two steps 66%) was prepared from **10h** (150 mg, 360 µmol) and tert-butyl 4-(4-aminobenzoyl)piperazine-1-carboxylate **11z₁₂** (132 mg, 432 µmol) by referring to the synthetic method of **I-54** in example 1. **¹HNMR** (400 MHz, Chloroform-d, *ppm)* δ 9.22 (s, 1H), 7.91 (d, *J* = 8.8 Hz, 1H), 7.75 (d, *J* = 8.3 Hz, 2H), 7.61 (d, *J* = 8.4 Hz, 2H), 7.51 (d, *J* = 8.2 Hz, 2H), 6.22 (t, *J* = 7.6 Hz, 1H), 3.56 (d, *J* = 57.7 Hz, 4H), 3.27 - 2.72 (m, 5H), 2.61 (dt, *J* = 9.0, 4.1 Hz, 1H), 2.10 (ddd, *J* = 18.2, 9.9, 5.8 Hz, 3H), 1.97 - 1.82 (m, 1H), 1.49 (s, 1H), 0.86 (s, 3H).

### Preparation of example I-66:

Compound **I-66** (84 mg, 173 µmol, 48%) was prepared from **10h** (150 mg, 360 µmol) and (4-aminophenyl)(4-methylpiperazin-1-yl)methanone **11z₁₄** (95 mg, 432 µmol) by referring to the synthetic method of **I-54a** in example 1. **¹H NMR** (400 MHz, Chloroform-d, *ppm*) δ 9.21 (s, 1H), 7.91 (d, *J* = 8.8 Hz, 1H), 7.80 - 7.69 (m, 3H), 7.61 (d, *J* = 8.8 Hz, 1H), 7.51 (d, *J* = 8.2 Hz, 2H), 6.22 (t, *J* = 7.6 Hz, 1H), 3.70 (d, *J* = 44.9 Hz, 4H), 2.89 (dq, *J* = 15.3, 7.8 Hz, 1H), 2.65 - 2.42 (m, 4H), 2.35 (s, 3H), 2.23 - 1.88 (m, 6H), 0.86 (s, 3H).

### Preparation of example I-67:

Compound **I-67** (141 mg, 299 µmol, 83%) was prepared from **10h** (150 mg, 360 µmol) and (4-aminophenyl)(morpholino)methanone **11z₉** (89 mg, 432 µmol) by referring to the synthetic method of **I-54a** in example 1. **¹H NMR** (400 MHz, Chloroform-*d*, *ppm*) δ 9.22 (s, 1H), 7.92 (d, *J* = 8.8 Hz, 1H), 7.77 (d, *J* = 8.1 Hz, 2H), 7.62 (d, *J* = 9.7 Hz, 2H), 7.52 (d, *J* = 8.1 Hz, 2H), 6.22 (t, *J* = 7.6 Hz, 1H), 3.73 (s, 8H), 3.07 (s, 1H), 2.89 (dd, *J* = 14.0, 7.4 Hz, 1H), 2.61 (d, *J* = 6.7 Hz, 1H), 2.10 (dq, *J* = 19.9, 13.6, 9.3 Hz, 3H), 1.88 (d, *J* = 10.6 Hz, 1H), 0.86 (s, 3H).

### Preparation of example I-68:

Compound **I-68** (110 mg, 212 µmol, 59%) was prepared from **10h** (150 mg, 360 µmol) and (4-aminophenyl)(1,1-dioxothiomorpholino)methanone **11z₆** (110 mg, 432 µmol) by referring to the synthetic method of **I-54a** in example 1. **¹H NMR** (400 MHz, Chloroform-*d*, *ppm*) δ 9.24 (s, 1H), 7.94 (d, *J* = 8.8 Hz, 1H), 7.84 (d, *J* = 8.3 Hz, 2H), 7.69 (s, 1H), 7.63 (d, *J* = 8.9 Hz, 1H), 7.55 (d, *J* = 8.4 Hz, 2H), 6.25 (t, *J* = 7.3 Hz, 1H), 4.16 (s, 4H), 3.10 (s, 4H), 2.98 - 2.74 (m, 2H), 2.74 - 2.53 (m, 1H), 2.30 - 1.97 (m, 3H), 1.97 - 1.82 (m, 1H). 0.88 (s, 3H).

### Preparation of example I-69:

Compound **I-69** (126 mg, 259 µmol, two steps, 72%) was prepared from **10h** (150 mg, 360 µmol) and tert-butyl 4-(4-aminobenzoyl)-1,4-diazepane-1-carboxylate **11z₇** (138 mg, 432 µmol) by referring to the synthetic method of **I-54** in example 1. **¹H NMR**(400 MHz, Chloroform-d, *ppm)* δ 9.21 (s, 1H), 7.91 (d, *J* = 8.8 Hz, 1H), 7.75 (d, *J* = 8.1 Hz, 2H), 7.61 (d, *J* = 8.4 Hz, 2H), 7.51 (d, *J* = 8.2 Hz, 2H), 6.24 (t, *J* = 7.5 Hz, 1H), 3.69 (d, *J* = 89.5 Hz, 4H), 2.98 (t, *J* = 51.0 Hz, 6H), 2.62 (d, *J* = 11.9 Hz, 1H), 2.17 - 1.98 (m, 3H), 1.98 - 1.84 (m, 2H), 1.79 (s, 1H), 0.87 (s, 3H).

### Preparation of example I-70:

Compound **I-70** (104 mg, 209 µmol, 58%) was prepared from **10h** (150 mg, 360 µmol) and (4-aminophenyl)(4-methyl-1,4-diazepan-1-yl)methanone **11z₈** (101 mg, 432 µmol) by referring to the synthetic method of **I-54a** in example 1. **¹H NMR** (400 MHz, Chloroform-*d*, *ppm*) δ 9.21 (s, 1H), 7.90 (d, *J* = 8.8 Hz, 1H), 7.75 (d, *J* = 7.8 Hz, 3H), 7.70 - 7.45 (m, 3H), 6.24 (t, *J* = 7.5 Hz, 1H), 3.81 (d, *J* = 13.8 Hz, 2H), 3.62 (d, *J* = 26.5 Hz, 2H), 2.84 (t, *J* = 8.0 Hz, 2H), 2.68 (s, 1H), 2.65 - 2.56 (m, 2H), 2.41 (d, *J* = 23.7 Hz, 3H), 2.17 - 2.01 (m, 4H), 1.91 (d, *J* = 7.2 Hz, 4H), 0.87 (s, 3H).

### Preparation of example I-71:

Compound 4-amino-N-(2-hydroxyethyl)benzenesulfonamide **11z₁₃** (0.91 g, 0.42 mmol, 80%) was prepared from 4-aminobenzenesulfonic acid **12b** (1 g, 0.58 mmol) and morpholine 2-aminoethan-1-ol **13z₁₃** (0.32 g, 0.53 mmol) by referring to the synthetic method **of 11a.** C₈H₁₃N₂O₃S [M+H]⁺: m/z = 217.1.

Compound **I-71** (135 mg, 281 µmol, 78%) was prepared from **10h** (150 mg, 360 µmol) and 4-amino-N-(2-hydroxyethyl)benzenesulfonamide **11z₁₃** (93 mg, 432 µmol) by referring to the synthetic method of **I-54a** in example 1. **¹H NMR** (400 MHz, DMSO-*d*₆, *ppm*) δ 10.58 (s, 1H), 9.50 (s, 1H), 8.19 (d, *J* = 8.6 Hz, 2H), 7.94 (d, *J* = 8.8 Hz, 1H), 7.86 - 7.78 (m, 3H), 7.44 (t, *J* = 6.0 Hz, 1H), 6.41 (dd, *J* = 8.2, 3.8 Hz, 1H), 5.16 (s, 1H), 4.67 (s, 1H), 3.39 (t, *J* = 6.5 Hz, 2H), 2.81 (q, *J* = 6.3 Hz, 2H), 2.67 - 2.61 (m, 1H), 2.41 - 2.31 (m, 1H), 2.15 - 1.85 (m, 5H), 0.85 (s, 3H).

### Biological example 1

### Assay of kinase activity

CDK/cyclin catalyzes the phosphorylation of a substrate, which concomitantly consumes ATP and produces ADP. The inhibition rate (%) in the presence of a small molecule inhibitor was assessed by measuring the amount of ADP generated in the reaction. The assay compounds were thoroughly mixed by vortexing and then diluted in DMSO to 100× the assay concentration. 40 nL of a compound was transferred to a 384-well assay plate using a nanoliter pipetting system (ECHO^{®} 655 SYSTEM). A 2× kinase solution was prepared using a 1× kinase reaction buffer (50 mM Hepes, 10 mM MgCl₂, 0.01% Brij35, 2 mM DTT). 2 µL of the kinase solution was transferred to the 384-well assay plate, and the plate was centrifuged at 1000 rpm for 60 seconds in a microplate centrifuge. The plate was incubated at 25 °C in a biochemical incubator for 10 minutes. A 2x mixture of substrate and ATP was prepared in the kinase reaction buffer. Then, 2 µL of the mixture of substrate and ATP was added to the 384-well assay plate. The plate was centrifuged at 1000 rpm for 60 seconds in a microplate centrifuge, and incubated at 25 °C in a biochemical incubator for 60 minutes. 4 µL of ADP-Glo Reagent (Cat. No. V9103, manufacturer: Promega) incubated at 25 °C in a biochemical incubator was added to each well of the 384-well assay plate. The plate was centrifuged at 1000 rpm for 60 seconds in a microplate centrifuge, and incubated at 25 °C in a biochemical incubator for 40 minutes. 8 µL of Detection Reagent incubated at 25 °C in a biochemical incubator was added to each well of the 384-well assay plate. The plate was centrifuged at 1000 rpm for 60 seconds in a microplate centrifuge, and incubated at 25 °C in a biochemical incubator for 40 minutes. Finally, luminescence signals were read using a multifunctional microplate reader (model: PHERAstar FSX, manufacturer: BMG). IC50 values were calculated by nonlinear fitting of the concentration-response curves using GraphPad Prism software.

The compounds of the present disclosure were tested in the kinase activity assay. It was found that they had good inhibitory activities against CDK2/cyclin E, CDK4/cyclin D1, CDK6/cyclin D3 and CDK9/cyclin T1. The results of the kinase inhibitory activities of the most representative compounds of the present disclosure are shown in the table below.

| **Example No.** | **CDK2/E1 (nM)** | **CDK4/D1 (nM)** | **CDK6/D3 (nM)** | **CDK9/T1 (nM)** |
|---|---|---|---|---|
| **I-1** | 0.2 | 2 | 13 | 9 |
| **I-2** | 0.3 | 2 | 12 | 8 |
| **I-3** | 0.4 | 1 | 4 | 6 |
| **I-4** | 0.08 | 0.5 | 8 | 5 |
| **I-5** | 0.07 | 0.8 | 10 | 7 |
| **I-6** | 0.06 | 0.5 | 3 | 2 |
| **I-7** | 0.1 | 0.8 | 3 | 3 |
| **I-8** | 0.1 | 0.8 | 19 | 3 |
| **I-9** | 0.2 | 1 | 15 | 2 |
| **I-10** | 0.1 | 0.6 | 4 | 2 |
| **I-11** | 0.08 | 0.7 | 6 | 2 |
| **I-12** | 1 | 3 | 18 | / |
| **I-13** | 0.8 | 4 | 29 | / |
| **I-14** | 2 | 2 | 39 | / |
| **I-15** | 1 | 2 | 94 | 44 |
| **I-16** | 4 | 12 | 230 | 78 |
| **I-17** | 0.8 | 2 | 190 | 60 |
| **I-18** | 2 | 5 | 35 | 25 |
| **I-19** | 1 | 2 | 12 | 8 |
| **I-20** | 1 | 1 | 16 | 3 |
| **I-21** | 0.5 | 1 | 14 | 2 |
| **I-22** | 2 | 4 | 23 | 4 |
| **I-23** | 0.4 | 3 | 35 | 24 |
| **I-24** | 0.1 | 1 | 26 | 8 |
| **I-25** | 0.2 | 1 | 29 | 16 |
| **I-26** | 1 | 4 | 19 | 16 |
| **I-27** | 2 | 5 | 14 | 16 |
| **I-28** | 2 | 2 | 18 | 14 |
| **I-29** | 1 | 2 | 12 | 6 |
| **I-30** | 1 | 2 | 94 | 44 |
| **I-31** | 7 | 8 | 89 | 380 |
| **I-32** | 3 | 15 | 230 | 315 |
| **I-33** | 0.2 | 1 | 26 | 45 |
| **I-34** | 0.09 | 0.7 | 19 | 2 |
| **I-35** | 0.7 | 2 | 12 | 6 |
| **I-36** | 0.8 | 4 | 16 | 11 |
| **I-37** | 0.8 | 3 | 11 | 10 |
| **I-38** | 0.05 | 0.5 | 3 | 4 |
| **I-39** | 0.07 | 0.7 | 4 | 2 |
| **I-40** | 0.06 | 0.7 | 3 | 2 |
| **I-41** | 0.08 | 1 | 5 | 2 |
| **I-42** | 0.06 | 1 | 4 | 2 |
| **I-43** | 1 | 2 | 8 | 130 |
| **I-44** | 0.7 | 2 | 16 | 140 |
| **I-45** | 0.8 | 3 | 36 | 144 |
| **I-46** | 0.7 | 3 | 24 | 134 |
| **I-47** | 0.7 | 2 | 20 | 148 |
| **I-48** | 3 | 7 | 205 | 344 |
| **I-49** | 3 | 21. | 401 | 390 |
| **I-50** | 2 | 4 | 85 | 100 |
| **I-51** | 2 | 7 | 149 | 122 |
| **I-52** | 4 | 10 | 127 | 419 |
| **I-53** | 8 | 49 | 803 | 995 |
| **I-54** | 1 | 2 | 16 | 8 |
| **I-55** | 0.7 | 2 | 18 | 6 |
| **I-56** | 0.8 | 1 | 4 | 15 |
| **I-57** | 0.9 | 1 | 4 | 23 |
| **I-58** | 1 | 2 | 9 | 19 |
| **I-59** | 1 | 2 | 15 | 46 |
| **I-60** | 0.8 | 1 | 3 | 19 |
| **I-61** | 0.9 | 1 | 2 | 16 |
| **I-62** | 0.08 | 0.5 | 4 | 3 |
| **I-63** | 0.08 | 0.6 | 3 | 3 |
| **I-64** | 0.1 | 0.6 | 4 | 3 |
| **I-65** | 0.6 | 0.6 | 3 | 3 |
| **I-66** | 0.8 | 1 | 15 | 6 |
| **I-67** | 1 | 2 | 21 | 46 |
| **I-68** | 1 | 2 | 34 | 112 |
| **I-69** | 0.05 | 0.5 | 3 | 2 |
| **I-70** | 0.06 | 0.7 | 2 | 2 |
| **I-71** | 0.9 | 1 | 6 | 9 |
| WO2021/023104 **Example I-39** | 0.7 | 4 | 14 | 12 |
| WO2021/023104 **Example I-40** | 0.7 | 2 | 12 | 5 |
| **PF06873600 (Ebvaciclib)** | 0.3 | 7 | 33 | 452 |
| **CYC-065 (Fadraciclib)** | 3 | 228 | >1000 | 76 |

### Biological example 2

### Assay of cell proliferation

Human ovarian cancer OVCAR3 cells were inoculated at 2000 cells/well in a 96-well plate and cultured in RPMI 1640 medium containing 20% FBS at 37 °C and 5% CO₂ overnight in an incubator. The next day, the compounds were subjected to a three-fold gradient dilution in DMSO from a peak concentration of 2 mM to give nine concentrations. Then, the compounds were diluted 3:200 in growth medium, and finally diluted 1:3 in the cell culture supernatant to give a culture supernatant containing 0.5% DMSO, with the compounds treatment concentrations ranging from 10 µM to 0.1 nM. The cells and the compounds were incubated at 37 °C and 5% CO₂ for 6 days. Then, cell proliferation assay was performed using the CellTiter-Glo (manufacturer: Promega, Cat. No. G9242), and luminescence signals were read using a multifunctional microplate reader (model: Synergy H1, manufacturer: BiotTek). IC50 values were calculated by fitting of the concentration-response curves with a four-parameter analysis method using GraphPad Prism software.

The cell proliferation assays of human colon cancer HCT-116 cells, human breast cancer MCF-7 cells, human breast cancer HCC1806 cells, human acute myeloid leukemia MV-4-11 cells, and human gastric cancer MKN1 cells were performed according to the same method, with different inoculation densities set according to different cell growth characteristics.

The IC50 (nM) values of the cell proliferation inhibitory activities of the most representative compounds of the present disclosure are shown in the table below.

| **Example No.** | **HCC1806** | **OVCAR3** | **MV-4-11** | **MNK1** | **HCT-116** | **MCF7** |
|---|---|---|---|---|---|---|
| **I-1** | 223 | 119 | 17 | 45 | / | / |
| **I-2** | 175 | 94 | 15 | 46 | / | / |
| **I-3** | 302 | 203 | / | 196 | 94 | / |
| **I-4** | 24 | 12 | 3 | 8 | 25 | 18 |
| **I-5** | 53 | 12 | 3 | 11 | / | / |
| **I-6** | 73 | 26 | 2 | 7 | / | / |
| **I-7** | 20 | 14 | 5 | 16 | 11 | 8 |
| **I-8** | 16 | 7 | 2 | 4 | / | / |
| **I-9** | 53 | 26 | 3 | 11 | / | / |
| **I-10** | 22 | 16 | 4 | 15 | 11 | 11 |
| **I-11** | 122 | 148 | 4 | 29 | / | / |
| **I-12** | 84 | 26 | 6 | 30 | / | / |
| **I-13** | 330 | 156 | 4 | 252 | / | / |
| **I-14** | 424 | 102 | 11 | 66 | / | / |
| **I-15** | 177 | 264 | / | / | / | / |
| **I-16** | 374 | 90 | 9 | 110 | / | / |
| **I-17** | 319 | 93 | 12 | 46 | / | / |
| **I-18** | 247 | 214 | 23 | 129 | / | / |
| **I-19** | 379 | 49 | 10 | 85 | 21 | 42 |
| **I-20** | 109 | 39 | 6 | 23 | / | / |
| **I-21** | 112 | 35 | 4 | 31 | / | / |
| **I-22** | 115 | 27 | 5 | 56 | / | / |
| **I-23** | 112 | 73 | 4 | 57 | / | / |
| **I-24** | 44 | 18 | 3 | 6 | / | / |
| **I-25** | 85 | 33 | 4 | 13 | / | / |
| **I-26** | 436 | 45 | / | 41 | / | / |
| **I-27** | 186 | 69 | 6 | 32 | / | / |
| **I-28** | 174 | 59 | 9 | 42 | / | / |
| **I-29** | 207 | 40 | / | 15 | / | / |
| **I-30** | 151 | 19 | / | 20 | / | / |
| **I-31** | 273 | 193 | / | 67 | / | / |
| **I-32** | 167 | 80 | 9 | 44 | / | / |
| **I-33** | 19 | 5 | 2 | 3 | / | / |
| **I-34** | 6 | 7 | 2 | 5 | / | / |
| **I-35** | 233 | 224 | 16 | 21 | 95 | 162 |
| **I-36** | 403 | 140 | 15 | 28 | 80 | 167 |
| **I-37** | 832 | 372 | 20 | 58 | 136 | 239 |
| **I-38** | 13 | 8 | / | 1 | / | / |
| **I-39** | 7 | 5 | / | 1 | / | / |
| **I-40** | 16 | 10 | / | 5 | / | / |
| **I-41** | 15 | 11 | / | 4 | / | / |
| **I-42** | 7 | 6 | / | 3 | / | / |
| **I-43** | 631 | 676 | / | 444 | / | / |
| **I-44** | 404 | 321 | / | 127 | / | / |
| **I-45** | 476 | 359 | / | 241 | / | / |
| **I-46** | 317 | 275 | / | 170 | / | / |
| **I-47** | 169 | 146 | / | 111 | / | / |
| **I-48** | 5502 | / | / | / | / | / |
| **I-49** | 2159 | 1371 | / | / | / | / |
| **I-50** | 1739 | 1311 | / | 548 | / | / |
| **I-51** | 441 | 301 | 51 | 100 | / | / |
| **I-52** | 5091 | / | / | / | / | / |
| **I-53** | 3166 | 1723 | / | / | / | / |
| **I-54** | 268 | 192 | 7 | 49 | / | / |
| **I-55** | 80 | 31 | 5 | 15 | / | / |
| **I-56** | 365 | 275 | 11 | 81 | / | / |
| **I-57** | 465 | 32 | 11 | 17 | / | / |
| **I-58** | 656 | 313 | 19 | 90 | / | / |
| **I-59** | 543 | 389 | 18 | 127 | / | / |
| **I-60** | 98 | 88 | 12 | 41 | / | / |
| **I-61** | 8 | 11 | 4 | 8 | / | / |
| **I-62** | 54 | 28 | 5 | 8 | / | / |
| **I-63** | 15 | 6 | 1 | 1 | / | / |
| **I-64** | 12 | 7 | 2 | 3 | / | / |
| **I-65** | 111 | 35 | 5 | 8 | / | / |
| **I-66** | 93 | 17 | 2 | 8 | / | / |
| **I-67** | 137 | 45 | 5 | 13 | / | / |
| **I-68** | 121 | 181 | 8 | 62 | / | / |
| **I-69** | 20 | 15 | 3 | 6 | / | / |
| **I-70** | 3 | 2 | 1 | 1 | / | / |
| **I-71** | 18 | 5 | 2 | 3 | / | / |
| WO2021/023104 **Example I-39** | 396 | 168 | 14 | 9 | 46 | 26 |
| WO2021/023104 **Example I-40** | 298 | 166 | 10 | 10 | 75 | 28 |
| **PF06873600 (Ebvaciclib)** | 481 | 118 | 100 | 77 | 69 | 24 |
| **CYC-065 (Fadraciclib)** | 623 | 349 | 171 | 209 | / | / |

### Biological example 3

### Western blotting for detecting the phosphorylated Rb at Ser780 and Ser807/811

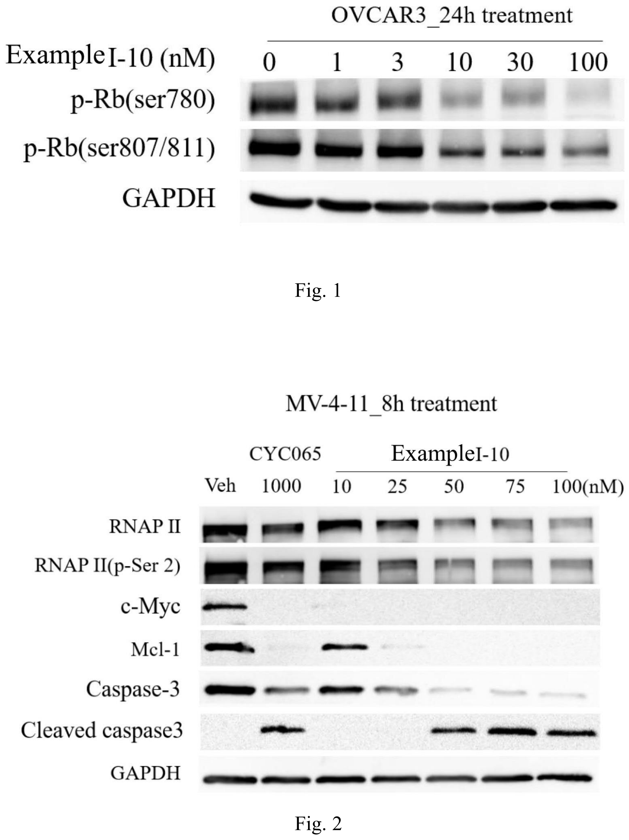

OVCAR3 cells were inoculated at 500,000 cells/well in a 6-well plate and cultured in RPMI 1640 medium containing 20% FBS at 37 °C and 5% CO₂ overnight in an incubator. The next day, the compound of example I-10 was diluted in DMSO to 200-fold the concentration of each treatment, then diluted in growth medium to 5-fold the concentration of the treatment, and finally diluted 1:5 in the cell culture supernatant. The cells were treated for 24 hours. The cells were lysed on ice using a RIPA lysis buffer containing protease inhibitors and phosphatase inhibitors. The lysis supernatant was obtained by centrifugation. The protein concentration in the lysis supernatant was determined using the BCA protein quantification method. Then, the protein bands were separated by SDS-PAGE gel electrophoresis and electrotransferred to a PVDF membrane. The PVDF membrane was blocked with 5% skimmed milk powder, and then incubated with antibodies against phospho-Ser780-Rb (manufacturer: CST, Cat. No. 8180S), phospho-Ser807/811-Rb (manufacturer: CST, Cat. No. 8516S), and GADPH (manufacturer: Abclonal, Cat. No. AC002) at 4 °C overnight. The membrane was washed to remove the unbound antibodies, and then incubated with a species-appropriate horseradish peroxidase-conjugated secondary antibody at room temperature for one hour. The membrane was washed, then incubated with a chemiluminescent substrate, and developed. The results are shown in Fig. 1.

### Western blotting for detecting the CDK9 downstream molecular pathways and apoptosis molecular pathways

MV-4-11 cells were inoculated at 2×10⁶ cells/well in a 6-well plate and cultured in RPMI 1640 medium containing 10% FBS at 37 °C and 5% CO₂ overnight in an incubator. The next day, the compound of example I-10 was diluted in DMSO to 200-fold the concentration of each treatment, then diluted in growth medium to 5-fold the concentration of the treatment, and finally diluted 1:5 in the cell culture supernatant. The cells were treated for 8 hours (CYC065 was used as a control compound). The cells were lysed on ice using a RIPA lysis buffer containing protease inhibitors and phosphatase inhibitors. The lysis supernatant was obtained by centrifugation. The protein concentration in the lysis supernatant was determined using the BCA protein quantification method. Then, the protein bands were separated by SDS-PAGE gel electrophoresis and electrotransferred to a PVDF membrane. The PVDF membrane was blocked with 5% skimmed milk powder, and then incubated with antibodies against RNAP II (manufacturer: Merck, Cat. No.: 05-623), phospho-Ser2 RNAP II (manufacturer: Merck, Cat. No.: 3864963), c-Myc (manufacturer: CST, Cat. No.: 18583S), Mcl-1 (manufacturer: CST, Cat. No.: 39224S), Caspase 3 (manufacturer: CST, Cat. No.: 9662), Cleaved Caspase 3 (manufacturer: CST, Cat. No.: 9664), and GADPH (manufacturer: Abclonal, Cat. No.: AC002) at 4 °C overnight. The membrane was washed to remove the unbound antibodies, and then incubated with a species-appropriate horseradish peroxidase-conjugated secondary antibody at room temperature for one hour. The membrane was washed, then incubated with a chemiluminescent substrate, and developed. The results are shown in Fig. 2.

### Biological example 4

### Tumor models

### OVCAR-3 tumor model

Human ovarian cancer OVCAR-3 cells were cultured in RPMI 1640 medium containing 20% FBS at 37 °C and 5% CO₂ in an incubator. Matrigel was mixed with serum-free RPMI 1640 medium at a ratio of 1:1, and then the mixture was used to resuspend the OVCAR-3 cancer cells at 2 × 10⁷ cells/0.2 ml. Then, the cells were inoculated subcutaneously into the right rib area of female NOD-SCID mice. When the volume of tumors reached approximately 100-150 mm³, the mice were randomly divided into groups for treatment, with six animals per group. The compound of example I-10 was prepared in a 20% aqueous solution of hydroxypropyl-gamma-cyclodextrin containing an equivalent amount of hydrochloric acid, and administered by oral gavage (p.o.) at dosages of 5, 10, and 15 (mg/kg) twice daily, 8 hours apart, for a total of 22 days. The PF06873600 treatment group was used as a positive control, and the untreated group was given solvent by oral gavage. The tumor volume was measured twice a week using a vernier caliper by measuring the long and short diameters of a tumor. The formula for calculating a tumor volume was: volume (mm³) = (long diameter (mm) × short diameter (mm) × short diameter (mm)/2. The mice were weighed daily.

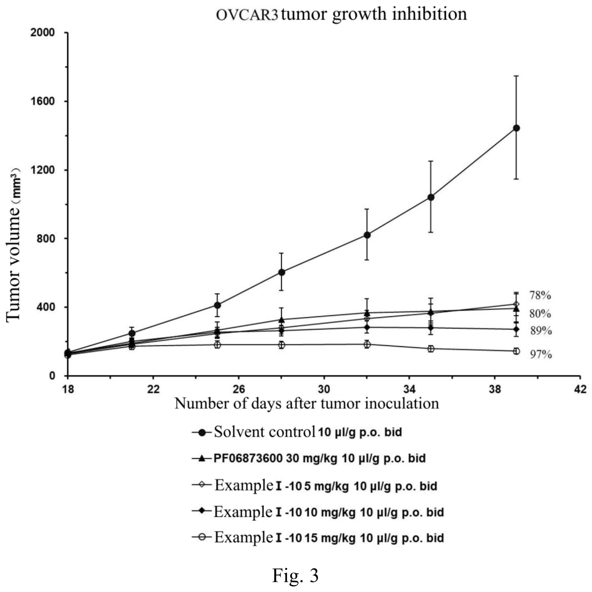

The therapeutic effect of a drug was evaluated using the tumor growth inhibition (TGI%). The calculation formula was: TGI = (1-(TV_{treated/last} - TV_{treated/day0})/(TV_{vehicle/last} - TV_{vehicle/day0})) × 100%, wherein, TV represents tumor volume, and the subscripts represent group and time, respectively. Fig. 3 shows the growth inhibitory effect of example I-10 on the OVCAR-3 tumor model, showing a dose-dependent relationship.

### MKN1 tumor model

Human gastric cancer MKN1 cells were cultured in RPMI 1640 medium containing 10% FBS at 37 °C and 5% CO₂ in an incubator. Matrigel was mixed with serum-free RPMI 1640 medium at a ratio of 1:1, and then the mixture was used to resuspend the MKN1 cancer cells at 1 × 10⁷ / 0.1 ml. Then, the cells were inoculated subcutaneously into the right rib area of female BALB/c nude mice. When the volume of tumors reached approximately 100-150 mm³, the mice were randomly divided into groups for treatment, with six animals per group. The compound was prepared in a 20% aqueous solution of hydroxypropyl-gamma-cyclodextrin containing an equivalent amount of hydrochloric acid, and administered by oral gavage (p.o.) at dosages of 10, 20, and 30 (mg/kg) once daily, for a total of 22 days. The untreated group was given solvent by oral gavage. The tumor volume was measured twice a week using a vernier caliper by measuring the long and short diameters of a tumor. The formula for calculating a tumor volume was: volume (mm³) = (long diameter (mm) × short diameter (mm) × short diameter (mm)/2. The mice were weighed daily.

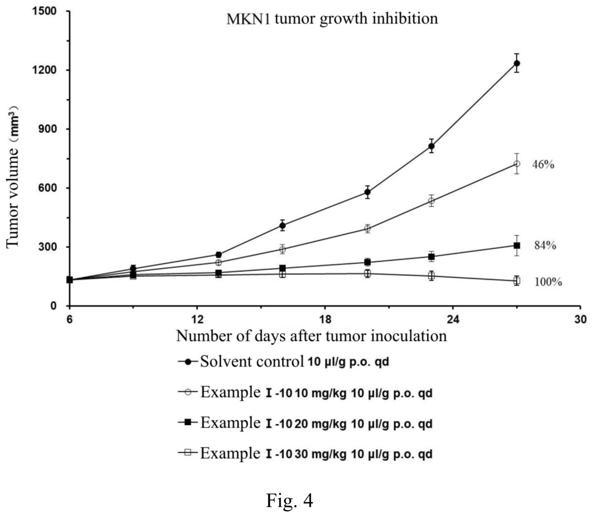

The therapeutic effect of a drug was evaluated using the tumor growth inhibition (TGI%). The calculation formula was: TGI = (1-(TV_{treated/last} - TV_{treated/day0})/(TV_{vehicle/last} - TV_{vehicle/day0})) × 100%, wherein, TV represents tumor volume, and the subscripts represent group and time, respectively. Fig. 4 shows the growth inhibitory effect of example I-10 on the MKN1 tumor model, showing a dose-dependent relationship.

## Claims

1. A compound of formula (I), or a pharmaceutically acceptable salt, an enantiomer, a diastereomer, a racemate, a solvate, a hydrate, a polymorph, a prodrug, or an isotopic variant thereof, or a mixture thereof: 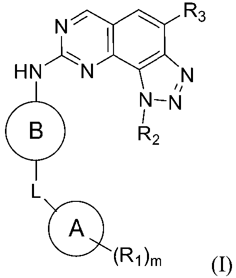 wherein
L is selected from -C(O)- and -S(O)₂-, alternatively -C(O)-;
ring A is 5- to 10-membered heterocyclyl;
ring B is C₆₋₁₀ aryl;
R₁ is selected from H, D, halogen, -C₀₋₆ alkylene-CN, -C₀₋₆ alkylene-NR₁ₐR_{1b}, -C₀₋₆ alkylene-OR₁ₐ, C₁₋₆ alkyl, C₁₋₆ haloalkyl, C₂₋₆ alkenyl and C₂₋₆ alkynyl;
R₁ₐ is selected from H, D, C₁₋₆ alkyl and C₁₋₆ haloalkyl;
R_{1b} is selected from H, D, C₁₋₆ alkyl and C₁₋₆ haloalkyl;
m is selected from 0, 1, 2, 3, 4 and 5;
R₂ is selected from C₁₋₆ alkyl, C₁₋₆ haloalkyl, C₅₋₁₀ cycloalkyl, 5- to 10-membered heterocyclyl, -C₀₋₆ alkylene-CN, -C₀₋₆ alkylene-NH₂ and -C₁₋₆ alkylene-OH, and the R₂ is optionally substituted with 1, 2, 3, 4 or 5 R₂ₐ;
R₂ₐ is selected from H, D, halogen, ORₐ, CN, NR_{b}R_{c}, C₁₋₆ alkyl and C₁₋₆ haloalkyl;
R₃ is selected from H, D, halogen, C₁₋₆ alkyl and C₁₋₆ haloalkyl;
Rₐ, R_{b} and R_{c} are selected from H, C₁₋₆ alkyl and C₁₋₆ haloalkyl;
each of the above groups may be optionally substituted with one or more deuterium atoms, up to fully deuterated;
with the proviso that, when R₂ is isopropyl and R₃ is H, ring A is not 6-membered heterocyclyl.

2. The compound according to claim 1, or a pharmaceutically acceptable salt, an enantiomer, a diastereomer, a racemate, a solvate, a hydrate, a polymorph, a prodrug, or an isotopic variant thereof, or a mixture thereof, wherein 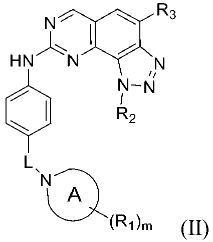 wherein
L is selected from -C(O)- and -S(O)₂-, alternatively -C(O)-;
ring A is 5- to 10-membered heterocyclyl;
R₁ is selected from H, D, halogen, OH, CN, NR₁ₐR_{1b}, -C₁₋₆ alkylene-OH, C₁₋₆ alkyl and C₁₋₆ haloalkyl;
R₁ₐ is selected from H, D and C₁₋₆ alkyl;
R_{1b} is selected from H, D and C₁₋₆ alkyl;
m is selected from 0, 1, 2, 3, 4 and 5;
R₂ is selected from C₁₋₆ alkyl, C₁₋₆ haloalkyl, C₅₋₁₀ cycloalkyl and -C₁₋₆ alkylene-OH, and the R₂ is optionally substituted with 1, 2, 3, 4 or 5 R₂ₐ;
R₂ₐ is selected from H, D, OH and C₁₋₆ alkyl;
R₃ is selected from H, D, halogen and C₁₋₆ haloalkyl, alternatively H or CHF₂, yet alternatively H;
each of the above groups may be optionally substituted with one or more deuterium atoms, up to fully deuterated.

3. The compound according to claim 1 or 2, or a pharmaceutically acceptable salt, an enantiomer, a diastereomer, a racemate, a solvate, a hydrate, a polymorph, a prodrug, or an isotopic variant thereof, or a mixture thereof, wherein
R₂ is selected from C₁₋₄ alkyl, C₅₋₈ cycloalkyl and -C₁₋₄ alkylene-OH, and the R₂ is optionally substituted with 1, 2 or 3 R₂ₐ;
R₂ₐ is selected from H, D, OH and C₁₋₆ alkyl, alternatively H, D, OH or CH₃;
alternatively,
R₂ is selected from 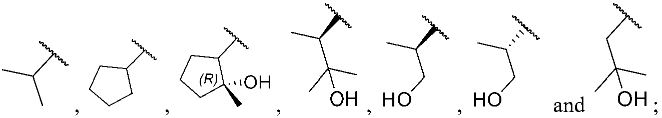
yet alternatively, R₂ is

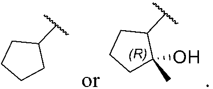

4. The compound according to any one of claims 1 to 3, or a pharmaceutically acceptable salt, an enantiomer, a diastereomer, a racemate, a solvate, a hydrate, a polymorph, a prodrug, or an isotopic variant thereof, or a mixture thereof, wherein
ring A is 5- to 8-membered heterocyclyl;
R₁ is selected from H, D, halogen, OH, CN, NR₁ₐR_{1b}, -C₁₋₄ alkylene-OH and C₁₋₄ alkyl;
R₁ₐ is selected from H, D and C₁₋₄ alkyl, alternatively, R₁ₐ is selected from H, CH₃ and isopropyl;
R_{1b} is selected from H, D and C₁₋₄ alkyl, alternatively, R_{1b} is selected from H and CH₃;
m is selected from 0, 1, 2 and 3;
alternatively, 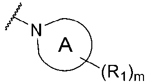 is selected from 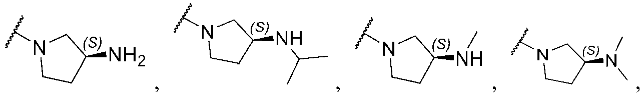 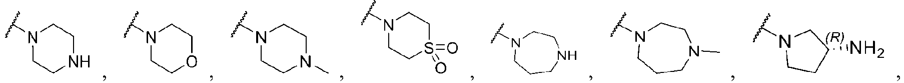 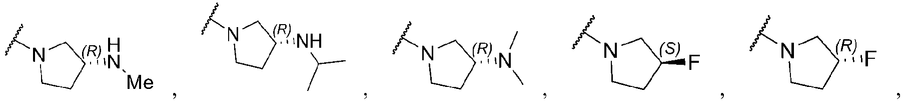 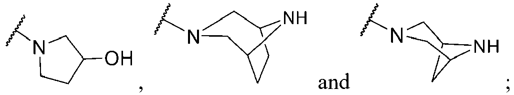
yet alternatively,

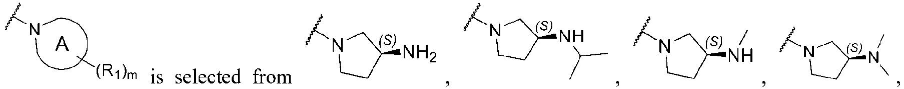

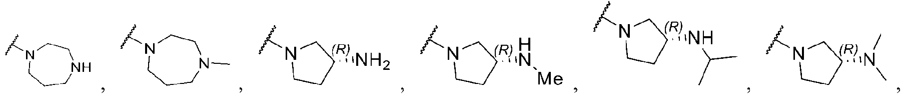

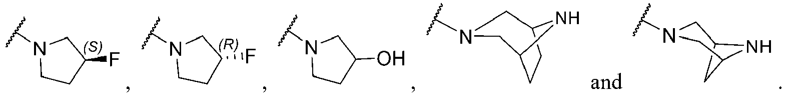

5. The compound according to any one of claims 1 to 4, or a pharmaceutically acceptable salt, an enantiomer, a diastereomer, a racemate, a solvate, a hydrate, a polymorph, a prodrug, or an isotopic variant thereof, or a mixture thereof, wherein the compound is a compound of formula (III): 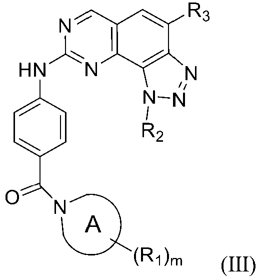 wherein
ring A is 5- to 8-membered heterocyclyl;
R₁ is selected from H, D, halogen, OH, CN, NR₁ₐR_{1b}, -C₁₋₄ alkylene-OH and C₁₋₄ alkyl;
R₁ₐ is selected from H, D and C₁₋₄ alkyl, alternatively, R₁ₐ is selected from H, CH₃ and isopropyl;
R_{1b} is selected from H, D and C₁₋₄ alkyl, alternatively, R_{1b} is selected from H and CH₃;
m is selected from 0, 1, 2 and 3;
R₂ is selected from C₁₋₄ alkyl, C₅₋₈ cycloalkyl and -C₁₋₄ alkylene-OH, and the R₂ is optionally substituted with 1, 2 or 3 R₂ₐ;
R₂ₐ is selected from H, D, OH and C₁₋₆ alkyl, alternatively H, OH or CH₃;
R₃ is selected from H, D and C₁₋₆ haloalkyl, alternatively H or CHF₂, yet alternatively H;
each of the above groups may be optionally substituted with one or more deuterium atoms, up to fully deuterated.

6. The compound according to claim 5, or a pharmaceutically acceptable salt, an enantiomer, a diastereomer, a racemate, a solvate, a hydrate, a polymorph, a prodrug, or an isotopic variant thereof, or a mixture thereof, wherein: 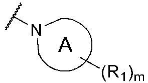 is selected from 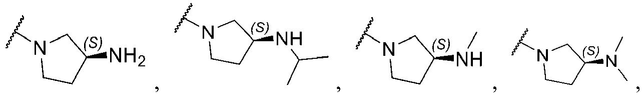 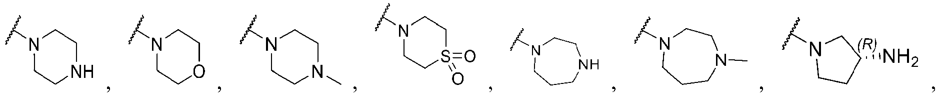 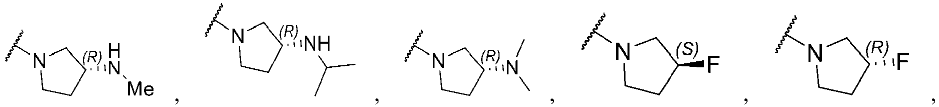 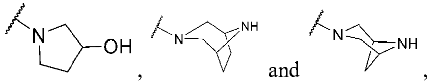 alternatively, 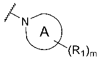 is selected from 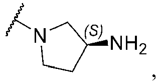 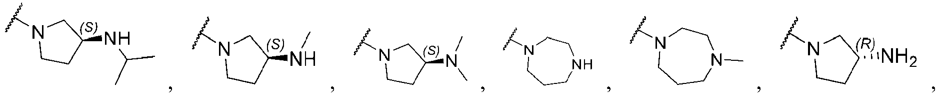 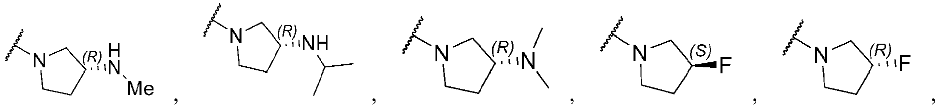 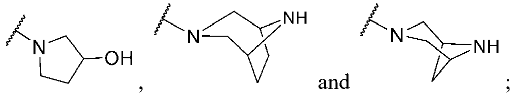
R₂ is selected from isopropyl, cyclopentyl and -C₁₋₂ alkylene-OH, and the R₂ is optionally substituted with 1, 2 or 3 R₂ₐ;
R₂ₐ is selected from H, D, OH and C₁₋₄ alkyl, alternatively selected from H, OH and CH₃;
alternatively, R₂ is selected from 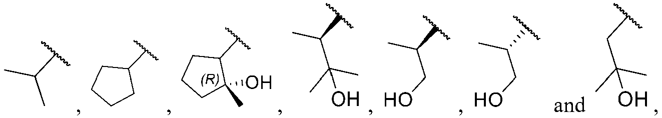
alternatively 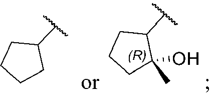
R₃ is selected from H, D and C₁₋₄ haloalkyl, alternatively H or CHF₂, yet alternatively H;
each of the above groups may be optionally substituted with one or more deuterium atoms, up to fully deuterated.

7. The compound according to any one of claims 1 to 6, or a pharmaceutically acceptable salt, an enantiomer, a diastereomer, a racemate, a solvate, a hydrate, a polymorph, a prodrug, or an isotopic variant thereof, or a mixture thereof, wherein the compound has the following structure: 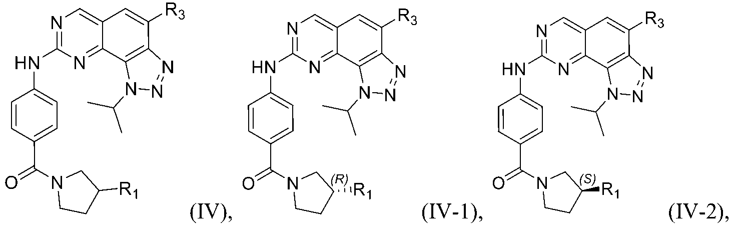 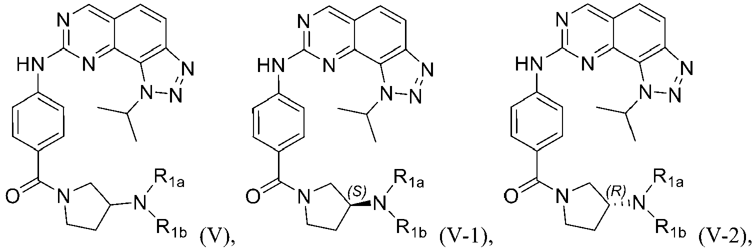 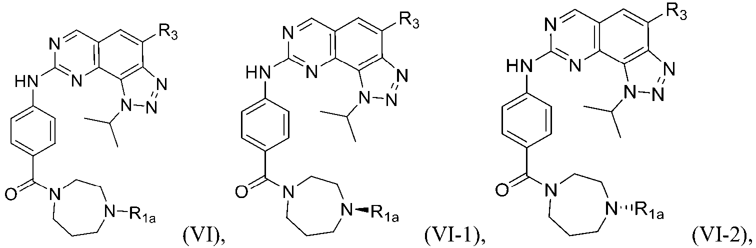 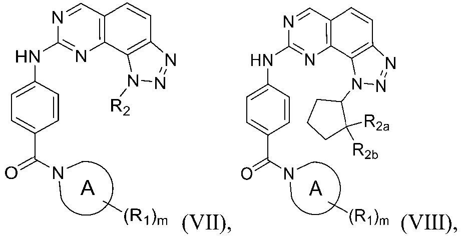 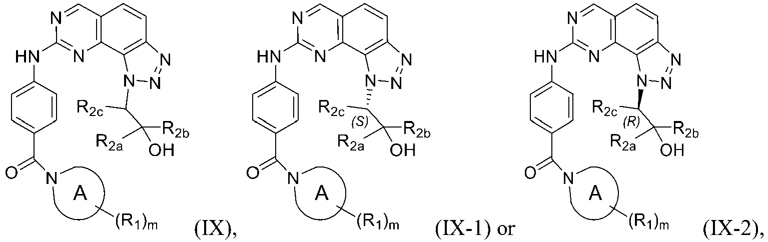 wherein
R_{2b} is selected from H, D, OH, CN, NH₂, C₁₋₆ alkyl and C₁₋₆ haloalkyl;
R_{2c} is selected from H, D, C₁₋₆ alkyl and C₁₋₆ haloalkyl;
the remaining variables are as defined in claims 1 to 6.

8. The compound according to claim 7, or a pharmaceutically acceptable salt, an enantiomer, a diastereomer, a racemate, a solvate, a hydrate, a polymorph, a prodrug, or an isotopic variant thereof, or a mixture thereof, wherein the compound is a compound of formula (IV), (IV-1) or (IV-2): 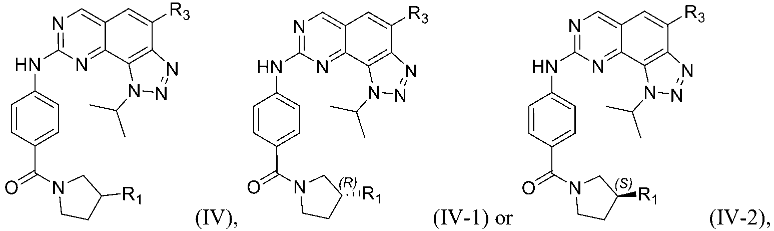 wherein
R₁ is selected from halogen, OH, CN and NR₁ₐR_{1b};
R₁ₐ is selected from H, D, C₁₋₆ alkyl and C₁₋₆ haloalkyl;
R_{1b} is selected from H, D, C₁₋₆ alkyl and C₁₋₆ haloalkyl;
R₃ is selected from H, D, C₁₋₆ alkyl and C₁₋₆ haloalkyl, alternatively H;
each of the above groups may be optionally substituted with one or more deuterium atoms, up to fully deuterated.

9. The compound according to claim 8, or a pharmaceutically acceptable salt, an enantiomer, a diastereomer, a racemate, a solvate, a hydrate, a polymorph, a prodrug, or an isotopic variant thereof, or a mixture thereof, wherein:
R₁ is selected from halogen, OH, CN and NR₁ₐR_{1b};
R₁ₐ is selected from H, D and C₁₋₄ alkyl;
R_{1b} is selected from H, D and C₁₋₄ alkyl;
R₃ is selected from H, D and C₁₋₄ haloalkyl, alternatively H;
each of the above groups may be optionally substituted with one or more deuterium atoms, up to fully deuterated.

10. The compound according to claim 8 or 9, or a pharmaceutically acceptable salt, an enantiomer, a diastereomer, a racemate, a solvate, a hydrate, a polymorph, a prodrug, or an isotopic variant thereof, or a mixture thereof, wherein:
R₁ is selected from F, OH and NR₁ₐR_{1b};
R₁ₐ is selected from H, CH₃ and isopropyl;
R_{1b} is selected from H and CH₃;
R₃ is selected from H and CHF₂, alternatively H.

11. The compound according to claim 7, or a pharmaceutically acceptable salt, an enantiomer, a diastereomer, a racemate, a solvate, a hydrate, a polymorph, a prodrug, or an isotopic variant thereof, or a mixture thereof, wherein the compound is a compound of formula (V), (V-1) or (V-2): 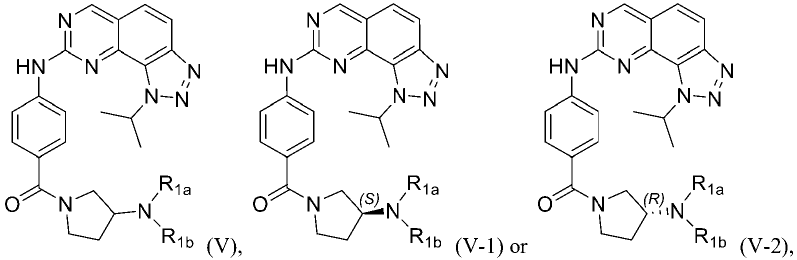 wherein
R₁ₐ is selected from H, D, C₁₋₆ alkyl and C₁₋₆ haloalkyl;
R_{1b} is selected from H, D, C₁₋₆ alkyl and C₁₋₆ haloalkyl;
alternatively,
R₁ₐ is selected from H, D and C₁₋₄ alkyl;
R_{1b} is selected from H, D and C₁₋₄ alkyl;
yet alternatively,
R₁ₐ is selected from H, CH₃ and isopropyl;
R_{1b} is selected from H and CH₃;
each of the above groups may be optionally substituted with one or more deuterium atoms, up to fully deuterated.

12. The compound according to claim 7, or a pharmaceutically acceptable salt, an enantiomer, a diastereomer, a racemate, a solvate, a hydrate, a polymorph, a prodrug, or an isotopic variant thereof, or a mixture thereof, wherein the compound is a compound of formula (VI), (VI-1) or (VI-2): 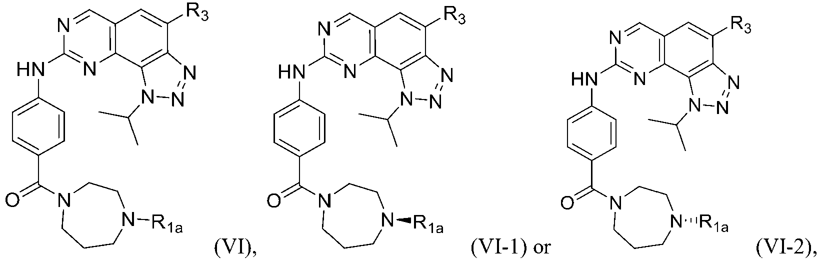 wherein
R₁ₐ is selected from H, D, C₁₋₆ alkyl and C₁₋₆ haloalkyl;
R₃ is selected from H, D, C₁₋₆ alkyl and C₁₋₆ haloalkyl, alternatively H;
alternatively,
R₁ₐ is selected from H, D and C₁₋₄ alkyl, alternatively H or methyl;
R₃ is selected from H, D and C₁₋₄ haloalkyl, alternatively H or CHF₂, yet alternatively H;
each of the above groups may be optionally substituted with one or more deuterium atoms, up to fully deuterated.

13. The compound according to claim 7, or a pharmaceutically acceptable salt, an enantiomer, a diastereomer, a racemate, a solvate, a hydrate, a polymorph, a prodrug, or an isotopic variant thereof, or a mixture thereof, wherein the compound is a compound of formula (VII): 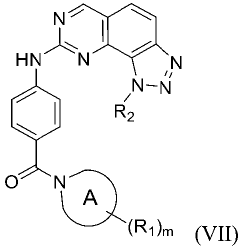 wherein
ring A is 5- to 10-membered heterocyclyl;
R₁ is selected from H, D, OH, CN, NH₂, C₁₋₆ alkyl, C₁₋₆ haloalkyl and NR₁ₐR_{1b};
R₁ₐ is selected from H, D, C₁₋₆ alkyl and C₁₋₆ haloalkyl;
R_{1b} is selected from H, D, C₁₋₆ alkyl and C₁₋₆ haloalkyl;
m is selected from 0, 1, 2, 3, 4 and 5;
R₂ is C₅₋₁₀ cycloalkyl, alternatively cyclopentyl, and the R₂ is optionally substituted with n R₂ₐ;
R₂ₐ is selected from H, D, OH, CN, NH₂, C₁₋₆ alkyl and C₁₋₆ haloalkyl;
n is selected from 0, 1, 2, 3, 4 and 5;
alternatively,
ring A is selected from 5-membered heterocyclyl and 7- to 10-membered heterocyclyl;
each of the above groups may be optionally substituted with one or more deuterium atoms, up to fully deuterated.

14. The compound according to claim 13, or a pharmaceutically acceptable salt, an enantiomer, a diastereomer, a racemate, a solvate, a hydrate, a polymorph, a prodrug, or an isotopic variant thereof, or a mixture thereof, wherein
ring A is 5- to 8-membered heterocyclyl;
R₁ is selected from H, D, C₁₋₄ alkyl and NR₁ₐR_{1b};
R₁ₐ is selected from H, D and C₁₋₄ alkyl, alternatively H, CH₃ or isopropyl;
R_{1b} is selected from H, D and C₁₋₄ alkyl, alternatively H or CH₃;
m is selected from 0, 1, 2 and 3;
R₂ is C₅₋₈ cycloalkyl, alternatively cyclopentyl, and the R₂ is optionally substituted with n R₂ₐ;
R₂ₐ is selected from H, OH and C₁₋₄ alkyl;
n is selected from 0, 1, 2 and 3;
alternatively,
ring A is selected from 5-membered heterocyclyl and 7-membered heterocyclyl;
each of the above groups may be optionally substituted with one or more deuterium atoms, up to fully deuterated.

15. The compound according to claim 13 or 14, or a pharmaceutically acceptable salt, an enantiomer, a diastereomer, a racemate, a solvate, a hydrate, a polymorph, a prodrug, or an isotopic variant thereof, or a mixture thereof, wherein 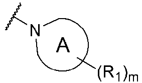 is selected from 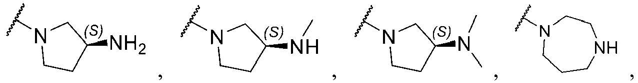 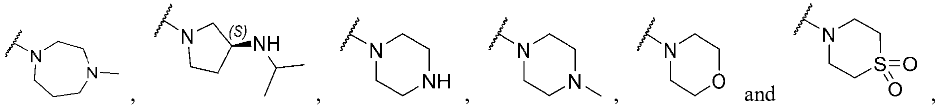 alternatively 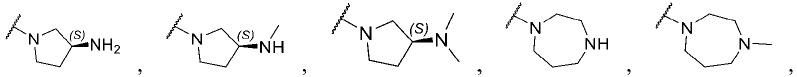 or 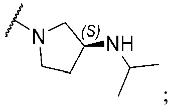
R₂ is cyclopentyl, and the R₂ is optionally substituted with n R₂ₐ;
R₂ₐ is selected from H, OH and CH₃;
n is selected from 0, 1 and 2;
alternatively, R₂ is

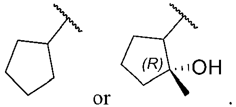

16. The compound according to any one of claims 13 to 15, or a pharmaceutically acceptable salt, an enantiomer, a diastereomer, a racemate, a solvate, a hydrate, a polymorph, a prodrug, or an isotopic variant thereof, or a mixture thereof, wherein the compound is a compound of formula (VIII): 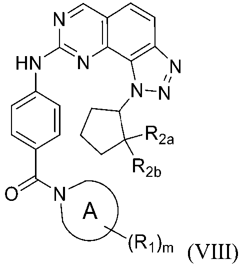 wherein
R_{2b} is selected from H, D, OH, CN, NH₂, C₁₋₆ alkyl and C₁₋₆ haloalkyl;
alternatively,
R_{2b} is selected from H, D, OH and C₁₋₄ alkyl;
yet alternatively,
R_{2b} is selected from H, D and CH₃;
the remaining variables are as defined in claims 13 to 15.

17. The compound according to claim 7, or a pharmaceutically acceptable salt, an enantiomer, a diastereomer, a racemate, a solvate, a hydrate, a polymorph, a prodrug, or an isotopic variant thereof, or a mixture thereof, wherein the compound is a compound of formula (IX), (IX-1) or (IX-2): 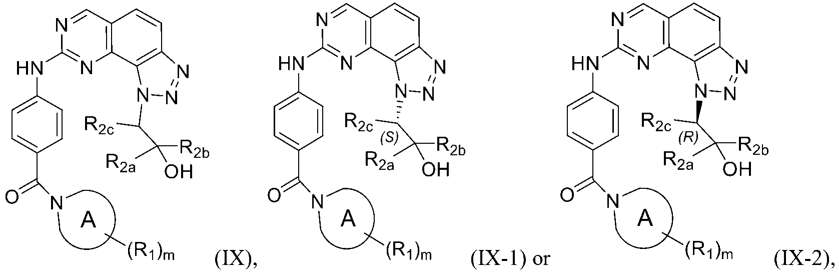 wherein
ring A is 5- to 10-membered heterocyclyl;
R₁ is selected from H, D, OH, CN, C₁₋₆ alkyl, C₁₋₆ haloalkyl and NR₁ₐR_{1b};
R₁ₐ is selected from H, D, C₁₋₆ alkyl and C₁₋₆ haloalkyl;
R_{1b} is selected from H, D, C₁₋₆ alkyl and C₁₋₆ haloalkyl;
m is selected from 0, 1, 2, 3, 4 and 5;
R₂ₐ is selected from H, D, C₁₋₆ alkyl and C₁₋₆ haloalkyl;
R_{2b} is selected from H, D, C₁₋₆ alkyl and C₁₋₆ haloalkyl;
R_{2c} is selected from H, D, C₁₋₆ alkyl and C₁₋₆ haloalkyl;
alternatively,
ring A is selected from 5-membered heterocyclyl and 7-membered heterocyclyl;
each of the above groups may be optionally substituted with one or more deuterium atoms, up to fully deuterated.

18. The compound according to claim 17, or a pharmaceutically acceptable salt, an enantiomer, a diastereomer, a racemate, a solvate, a hydrate, a polymorph, a prodrug, or an isotopic variant thereof, or a mixture thereof, wherein:
ring A is 5- to 8-membered heterocyclyl;
R₁ is selected from H, D, C₁₋₄ alkyl and NR₁ₐR_{1b};
R₁ₐ is selected from H, D and C₁₋₄ alkyl, alternatively selected from H, CH₃ and isopropyl;
R_{1b} is selected from H, D and C₁₋₄ alkyl, alternatively H or CH₃;
m is selected from 0, 1, 2 and 3;
R₂ₐ is selected from H, D and C₁₋₄ alkyl, alternatively H or CH₃;
R_{2b} is selected from H, D and C₁₋₄ alkyl, alternatively H or CH₃;
R_{2c} is selected from H, D and C₁₋₄ alkyl, alternatively H or CH₃;
alternatively,
ring A is selected from 5-membered heterocyclyl and 7-membered heterocyclyl;
each of the above groups may be optionally substituted with one or more deuterium atoms, up to fully deuterated.

19. The compound according to claim 17 or 18, or a pharmaceutically acceptable salt, an enantiomer, a diastereomer, a racemate, a solvate, a hydrate, a polymorph, a prodrug, or an isotopic variant thereof, or a mixture thereof, wherein 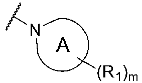 is selected from 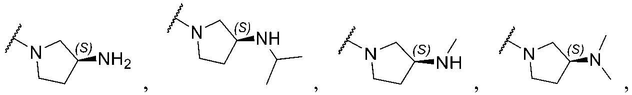 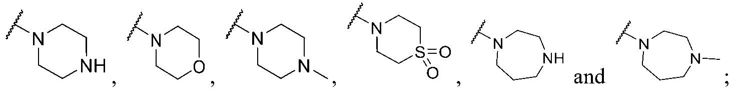
R₂ₐ is selected from H and CH₃;
R_{2b} is selected from H and CH₃;
R_{2c} is selected from H and CH₃;
each of the above groups may be optionally substituted with one or more deuterium atoms, up to fully deuterated.

20. A compound, or a pharmaceutically acceptable salt, an enantiomer, a diastereomer, a racemate, a solvate, a hydrate, a polymorph, a prodrug, or an isotopic variant thereof, wherein the compound is selected from:

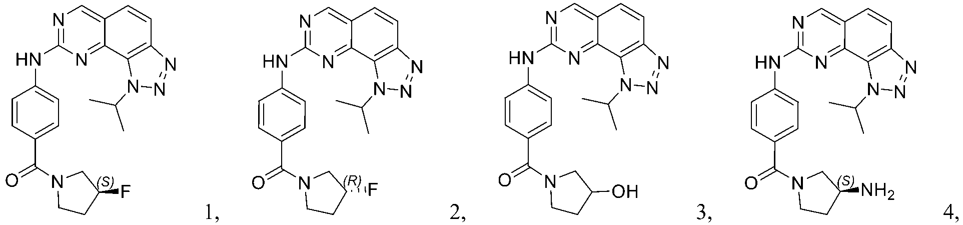

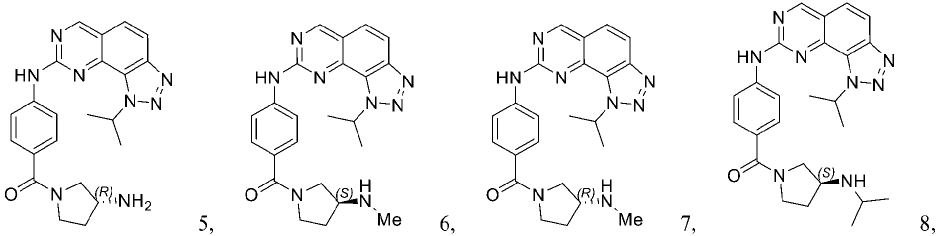

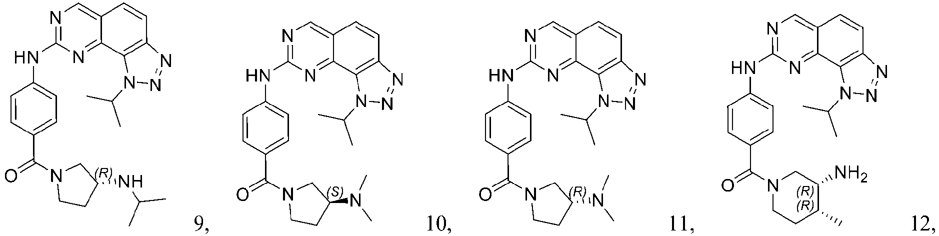

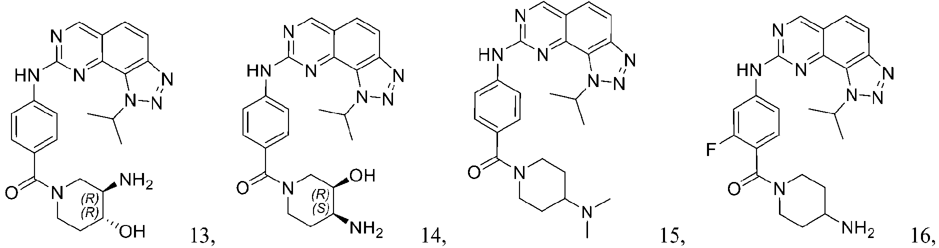

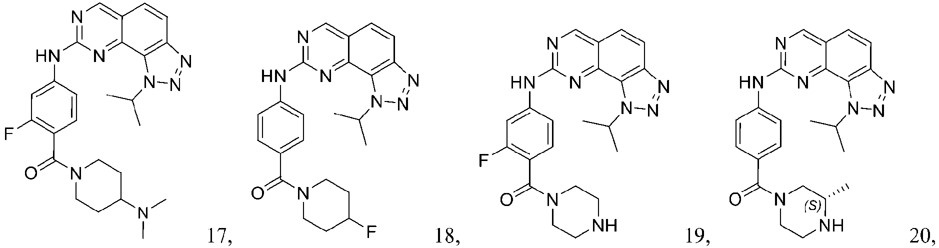

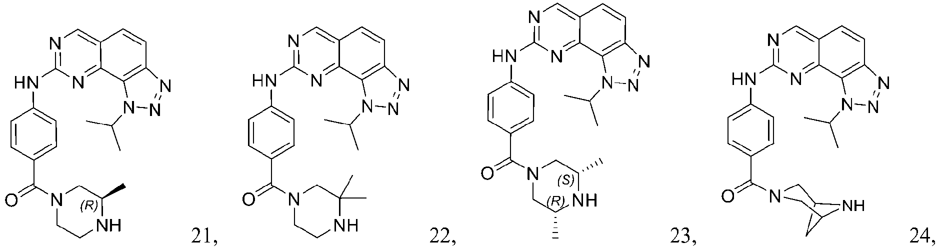

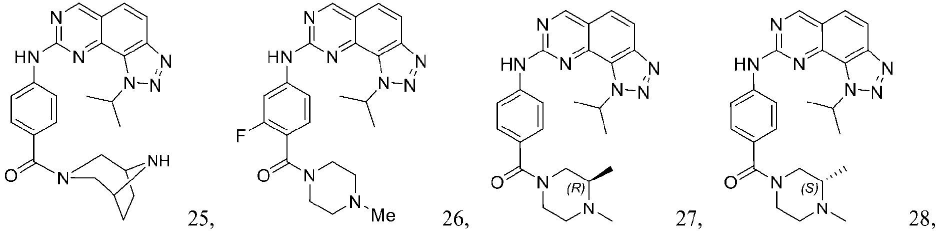

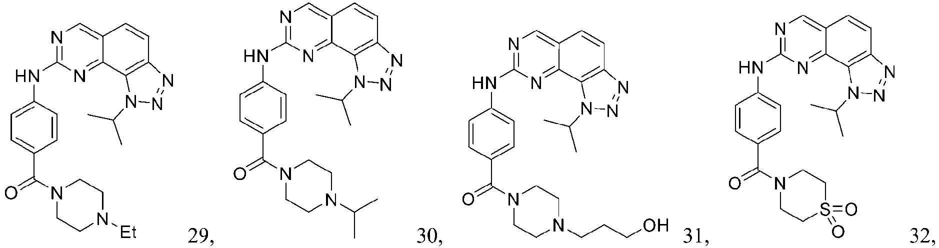

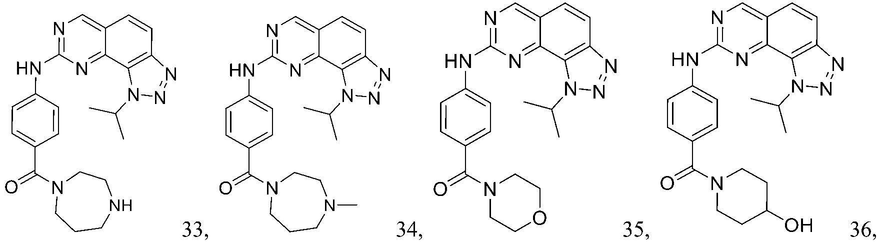

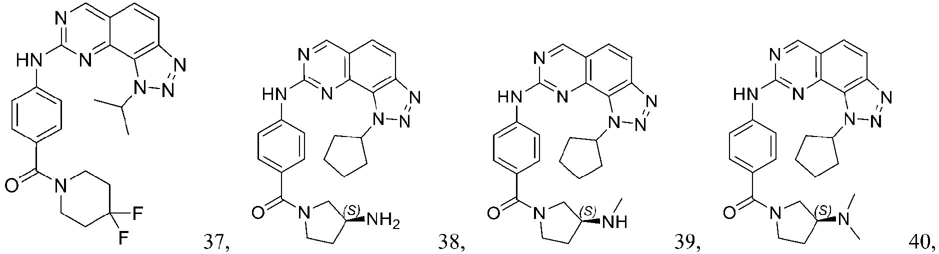

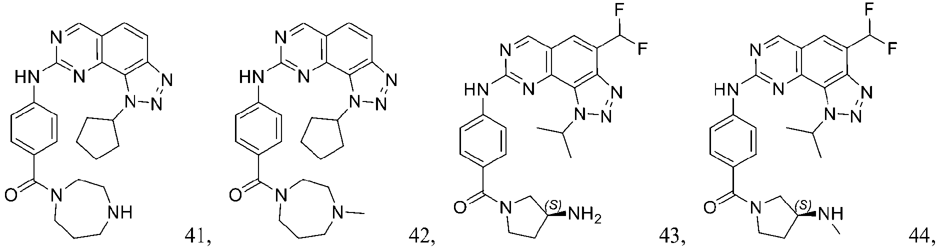

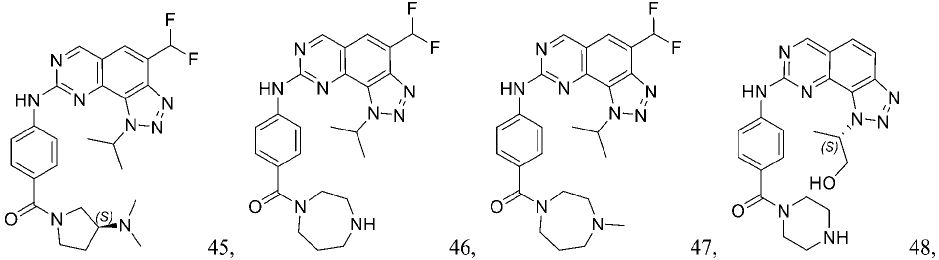

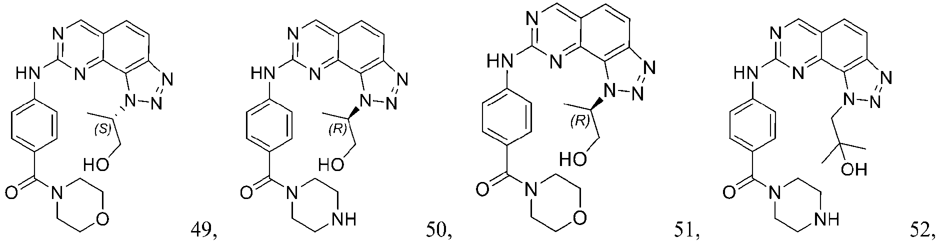

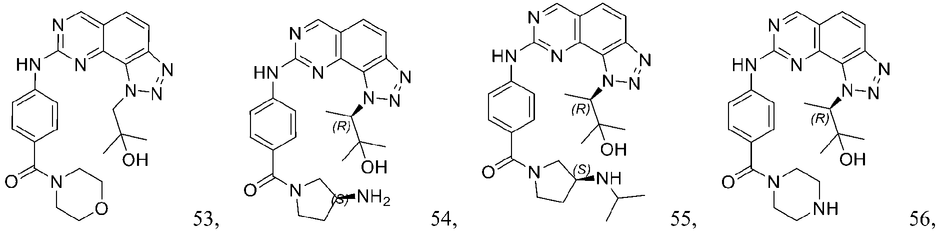

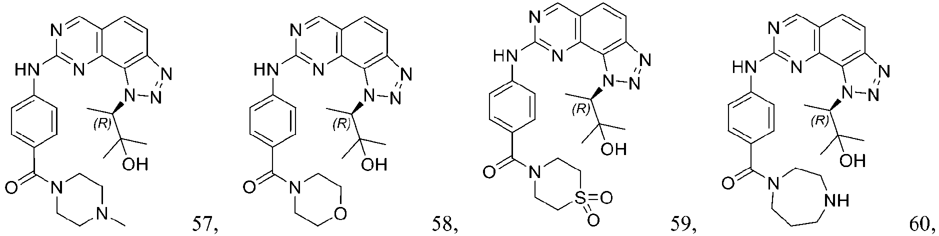

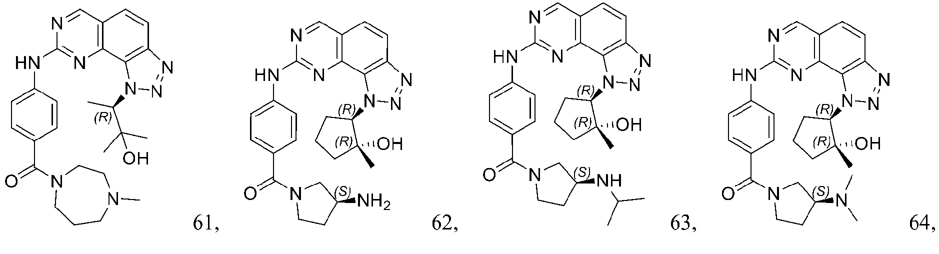

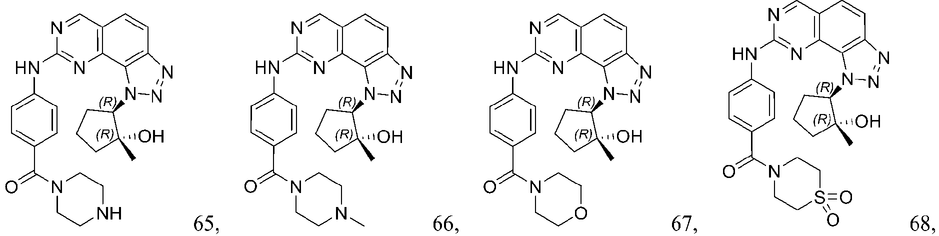

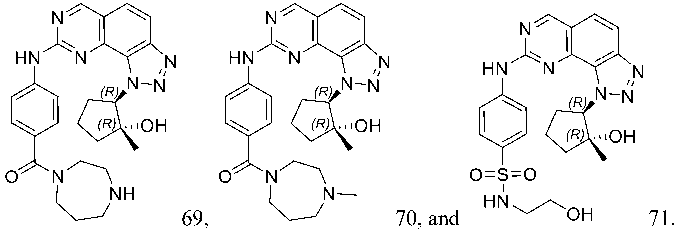

21. A pharmaceutical composition, comprising the compound according to any one of claims 1 to 20, or a pharmaceutically acceptable salt, an enantiomer, a diastereomer, a racemate, a solvate, a hydrate, a polymorph, a prodrug, or an isotopic variant thereof, and pharmaceutically acceptable excipient(s).

22. The pharmaceutical composition according to claim 21, which further comprises other therapeutic agent(s).

23. A kit, comprising
a first container, comprising the compound according to any one of claims 1 to 20, or a pharmaceutically acceptable salt, an enantiomer, a diastereomer, a racemate, a solvate, a hydrate, a polymorph, a prodrug, or an isotopic variant thereof; and optionally, a second container, comprising other therapeutic agent(s); and optionally, a third container, comprising pharmaceutically excipient(s) for diluting or suspending the compound and/or other therapeutic agent(s).

24. Use of the compound according to any one of claims 1 to 20, or a pharmaceutically acceptable salt, an enantiomer, a diastereomer, a racemate, a solvate, a hydrate, a polymorph, a prodrug or an isotopic variant thereof, in the manufacture of a medicament for the treatment and/or prevention of a CDK-mediated disease.

25. A method of treating and/or preventing a CDK-mediated disease in a subject, which comprises administering to the subject the compound according to any one of claims 1 to 20, or a pharmaceutically acceptable salt, an enantiomer, a diastereomer, a racemate, a solvate, a hydrate, a polymorph, a prodrug or an isotopic variant thereof, or the pharmaceutical composition according to claim 21 or 22.

26. The compound according to any one of claims 1 to 20, or a pharmaceutically acceptable salt, an enantiomer, a diastereomer, a racemate, a solvate, a hydrate, a polymorph, a prodrug or an isotopic variant thereof, or the pharmaceutical composition according to claim 21 or 22, for use in the treatment and/or prevention of a CDK-mediated disease.

27. The use according to claim 24 or the method according to claim 25 or the compound or composition for use according to claim 26, wherein the CDK-mediated disease includes cell proliferative diseases, includes but is not limited to cell proliferative diseases such as solid tumors such as sarcomas and carcinomas (e.g., fibrosarcoma, myxosarcoma, liposarcoma, chondrosarcoma, osteosarcoma, chordoma, angiosarcoma, endothelial sarcoma, lymphangiosarcoma, lymphangioendothelioma, synovialoma, mesothelioma, ewing sarcoma, leiomyosarcoma, rhabdomyosarcoma, colon cancer, pancreatic cancer, breast cancer, ovarian cancer, prostate cancer, squamous cell carcinoma, basal cell carcinoma, adenocarcinoma, hidradenoma, sebaceous carcinoma, papillary carcinoma, papillary adenocarcinoma, cystadenocarcinoma, medullary carcinoma, bronchial carcinoma, renal cell carcinoma, liver cancer, cholangiocarcinoma, choriocarcinoma, seminoma, embryonal cancer, embryonal carcinosarcoma, cervical cancer, uterine cancer, testicular cancer, lung cancer, small cell lung cancer, bladder cancer, epithelial cancer, glioma, astrocytoma, medulloblastoma, craniopharyngioma, ependymoma, pineal tumor, hemangioblastoma, acoustic neuroma, oligodendroglioma, schwannoma, meningioma, melanoma, neuroblastoma and retinoblastoma).
